(19) Europäisches Patentamt
European Patent Office
Office européen des brevets

(11) EP 4 628 483 A1

(12) **EUROPEAN PATENT APPLICATION**
published in accordance with Art. 153(4) EPC

(43) Date of publication:
**08.10.2025 Bulletin 2025/41**

(21) Application number: **23896784.8**

(22) Date of filing: **28.11.2023**

(51) International Patent Classification (IPC):
***C07D 237/32*** (2006.01) ***C07D 403/04*** (2006.01)
***C07D 403/14*** (2006.01) ***A61K 31/502*** (2006.01)
***A61P 35/00*** (2006.01)

(52) Cooperative Patent Classification (CPC):
**A61K 31/502; A61P 35/00; C07D 237/32; C07D 403/04; C07D 403/14**

(86) International application number:
**PCT/CN2023/134733**

(87) International publication number:
**WO 2024/114635 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2022 CN 202211514520**
**26.05.2023 CN 202310612126**
**16.11.2023 CN 202311533426**
**20.11.2023 CN 202311545467**

(71) Applicant: **Chia Tai Tianqing Pharmaceutical Group Co., Ltd.**
**Lianyungang, Jiangsu 222062 (CN)**

(72) Inventors:
- **LIU, Fei**
  **Lianyungang, Jiangsu 222062 (CN)**
- **XU, Hongjiang**
  **Lianyungang, Jiangsu 222062 (CN)**
- **GU, Jiajia**
  **Lianyungang, Jiangsu 222062 (CN)**
- **SHI, Wei**
  **Lianyungang, Jiangsu 222062 (CN)**
- **GAO, Peng**
  **Lianyungang, Jiangsu 222062 (CN)**

(74) Representative: **Potter Clarkson**
**Chapel Quarter**
**Mount Street**
**Nottingham NG1 6HQ (GB)**

# (54) DIHYDROPHTHALAZINE-CONTAINING COMPOUND

(57) The present disclosure belongs to the field of medicinal chemistry; and relates to a dihydrophthalazine-containing compound, a preparation method therefor, a pharmaceutical composition containing same, and the use thereof in the treatment of relevant diseases (e.g. cancer).

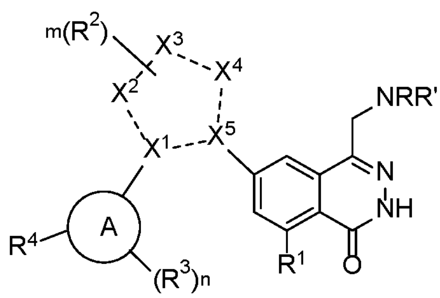

I

EP 4 628 483 A1

## Description

### CROSS-REFERENCE TO RELATED APPLICATION

The present application claims priority and benefits of the Chinese Patent Application No. 202211514520.5 filed with the China National Intellectual Property Administration on November 29, 2022, the Chinese Patent Application No. 202310612126.3 filed with the China National Intellectual Property Administration on May 26, 2023, the Chinese Patent Application No. 202311533426.9 filed with the China National Intellectual Property Administration on November 16, 2023, and the Chinese Patent Application No. 202311545467.X filed with the China National Intellectual Property Administration on November 20, 2023, the disclosures of which are incorporated herein by reference in their entireties.

### TECHNICAL FIELD

The present disclosure relates to a dihydrophthalazine-containing compound, a preparation method therefor, a pharmaceutical composition containing the compound, and use thereof for treating related diseases (e.g., cancer).

### BACKGROUND

Protein arginine N-methyltransferase (PRMT5) is a type II arginine methyltransferase that can catalyze the transfer of a methyl group from S-adenosyl-L-methionine (SAM) to an omega-nitrogen of the guanidino functional group of a protein L-arginine residue (omega monomethylation) and transfer a second methyl group to another omega-nitrogen, producing symmetrical dimethylarginine (sDMA). PRMT5 forms a complex with MEP50 (methylosome protein 50), which is required for substrate recognition and orientation, as well as for the catalytic activity of PRMT5 as a methyltransferase against histone 2A and histone 4. Homozygous deletions of p16/CDKN2a are common in cancer, and these mutations often involve the co-deletion of adjacent genes, including the gene encoding methylthioadenonine phosphorylase (MTAP). It is estimated that approximately 15% of human cancers exhibit homozygous deletions of the MTAP gene. Cells lacking MTAP activity have elevated levels of MTAP substrate methylthioadenosine (MTA), which is a potent inhibitor of PRMT5. Inhibition of PRMT5 activity results in decreased methylation activity and increased sensitivity of cell proliferation to PRMT5 depletion or loss of activity. Thus, loss of MTAP activity decreases the methylation activity of PRMT5, making cells selectively dependent on PRMT5 activity. Thus, in MTAP-deficient cancers, the synergistic inhibition of PRMT5 activity by MTA will provide therapeutic benefits for a variety of cancers. The compounds of the present disclosure provide such therapeutic benefits as MTA-synergistic PRMT5 inhibitors, which negatively regulate the activity of MTA-bound PRMT5 in cells, particularly MTAP-deficient cells, or are used for treating various forms of MTAP-related cancers. There is a need to develop a novel MTA-synergistic PRMT5 inhibitor that is able to inhibit PRMT5 activity at elevated MTA concentrations, particularly in MTAP-deficient cells.

### SUMMARY

The present disclosure relates to a compound of formula I, a stereoisomer thereof, or a pharmaceutically acceptable salt thereof,

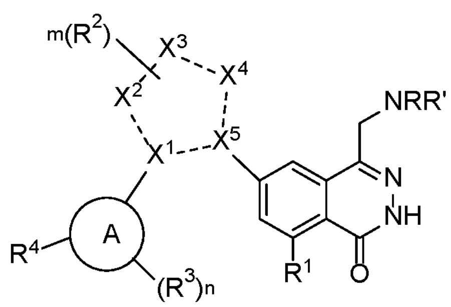

I

wherein

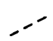

represents a single bond or a double bond;

R and R' are each independently selected from the group consisting of H, $C_{1-10}$ alkyl, and $C_{3-10}$ cycloalkyl;
$R^1$ is selected from the group consisting of hydrogen, halogen, OH, $C_{1-10}$ alkyl, halogenated $C_{1-10}$ alkyl, $C_{1-10}$ alkoxy, $C_{3-10}$ cycloalkyl, 3- to 10-membered heterocycloalkyl, 3- to 10-membered heterocycloalkenyl, $C_{2-10}$ alkenyl, $C_{2-10}$ alkynyl, $-NHR^a$, and $-NR^aR^b$;
$R^a$ and $R^b$ are each independently selected from the group consisting of H, $C_{1-10}$ alkyl, $C_{3-10}$ cycloalkyl, $-COC_{1-10}$ alkyl, and $-S(O)_2C_{1-10}$ alkyl;
$X^1$, $X^2$, $X^3$, $X^4$, and $X^5$ are each independently selected from the group consisting of S, C, CH, $CH_2$, N, and NH;
$R^2$ are each independently selected from the group consisting of =O, OH, $NH_2$, CN, halogen, $C_{1-10}$ alkyl, $C_{1-10}$ alkoxy, $C_{3-10}$ cycloalkyl, and 3- to 10-membered heterocycloalkyl, the $C_{1-10}$ alkyl, $C_{1-10}$ alkoxy, $C_{3-10}$ cycloalkyl, or 3- to 10-membered heterocycloalkyl being optionally substituted with one or more groups selected from the group consisting of OH, $NH_2$, CN, and halogen;
m is selected from the group consisting of 0, 1, 2, 3, 4, and 5;
ring A is selected from the group consisting of $C_{6-10}$ aryl, 5- to 12-membered heteroaryl, 5- to 15-membered heterocyclyl, and benzo $C_{5-7}$ cycloalkenyl;
$R^3$ are each independently selected from the group consisting of OH, $NH_2$, CN, halogen, and the following groups optionally substituted with one or more $R^5$: $C_{1-12}$ alkyl, $C_{1-12}$ alkoxy, $C_{1-12}$ alkyl S-, $C_{1-12}$ alkyl NH-, $(C_{1-12}$ alkyl$)_2$ N-, $C_{1-12}$ alkyl NHC(O)-, $(C_{1-12}$ alkyl$)_2$NC(O)-, $C_{1-12}$ alkyl C(O)NH-, $C_{1-12}$ alkyl OC(O)-, $C_{1-12}$ alkyl C(O)O-, $C_{2-12}$ alkenyl, $C_{2-12}$ alkynyl, $C_{3-12}$ cycloalkyl, 3- to 12-membered heterocycloalkyl, $C_{6-12}$ aryl, or 5- to 12-membered heteroaryl;
$R^5$ is selected from the group consisting of OH, $NH_2$, CN, halogen, and the following groups optionally substituted with one or more $R^d$: $C_{1-10}$ alkyl, $C_{1-10}$ alkoxy, $C_{1-10}$ alkyl S-, $C_{1-10}$ alkyl NH-, $(C_{1-10}$ alkyl$)_2$ N-, $C_{2-10}$ alkenyl, $C_{3-10}$ cycloalkyl, 3- to 10-membered heterocycloalkyl, $C_{6-10}$ aryl, or 5- to 10-membered heteroaryl;
$R^d$ is selected from the group consisting of OH, $NH_2$, CN, COOH, CHO, halogen, $C_{1-10}$ alkyl, $C_{1-10}$ alkoxy, $C_{1-10}$ alkyl S-, $C_{1-10}$ alkyl NH-, and $(C_{1-10}$ alkyl$)_2$ N-, the $C_{1-10}$ alkyl, $C_{1-10}$ alkoxy, $C_{1-10}$ alkyl S-, $C_{1-10}$ alkyl NH-, or $(C_{1-10}$ alkyl$)_2$ N- being optionally substituted with one or more groups selected from the group consisting of OH, $NH_2$, CN, and halogen;
n is selected from the group consisting of 0, 1, 2, 3, 4, and 5;
$R^4$ is selected from being absent, or is selected from -X-cycB;
X is selected from the group consisting of NH, N($C_{1-10}$ alkyl), O, and S;
cycB is selected from the group consisting of $C_{3-10}$ cycloalkyl, 3- to 10-membered heterocycloalkyl, $C_{6-10}$ aryl, and 5- to 10-membered heteroaryl, the $C_{3-10}$ cycloalkyl, 3- to 10-membered heterocycloalkyl, $C_{6-10}$ aryl, or 5- to 10-membered heteroaryl being optionally substituted with one or more $R^c$;
$R^c$ are each independently selected from the group consisting of OH, $NH_2$, CN, halogen, $C_{1-10}$ alkyl, $C_{1-10}$ alkoxy, $C_{2-10}$ alkenyl, and $C_{2-10}$ alkynyl, the $C_{1-10}$ alkyl, $C_{1-10}$ alkoxy, $C_{2-10}$ alkenyl, or $C_{2-10}$ alkynyl being optionally substituted with one or more groups selected from the group consisting of OH, $NH_2$, CN, and halogen;
and the following compounds are excluded:

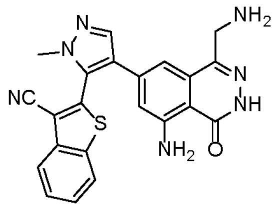 and 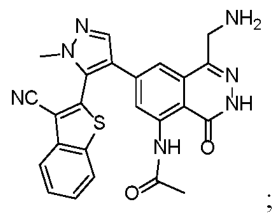 ;

optionally, the proviso is that:

when $R^1$ is selected from the group consisting of hydrogen, halogen, OH, $C_{1-10}$ alkyl, halogenated $C_{1-10}$ alkyl, $C_{1-10}$ alkoxy, $C_{3-10}$ cycloalkyl, 3- to 10-membered heterocycloalkyl, and 3- to 10-membered heterocycloalkenyl, n is selected from the group consisting of 1, 2, 3, 4, and 5, one of $R^3$ is selected from the group consisting of $C_{2-12}$ alkenyl and $C_{2-12}$ alkynyl, the $C_{2-12}$ alkenyl or $C_{2-12}$ alkynyl being optionally substituted with one or more $R^5$, and the others of $R^3$ (if present, i.e., when n is selected from the group consisting of 2, 3, 4, and 5) are each independently selected from the group consisting of OH, $NH_2$, CN, halogen, and the following groups optionally substituted with one or more $R^5$: $C_{1-12}$ alkyl, $C_{1-12}$ alkoxy, $C_{1-12}$ alkyl S-, $C_{1-12}$ alkyl NH-, $(C_{1-12}$ alkyl$)_2$ N-, $C_{1-12}$ alkyl NHC(O)-, $(C_{1-12}$ alkyl$)_2$ NC(O)-, $C_{1-12}$ alkyl C(O)NH-, $C_{1-12}$ alkyl OC(O)-, $C_{1-12}$ alkyl C(O)O-, $C_{2-12}$ alkenyl, $C_{2-12}$ alkynyl, $C_{3-12}$ cycloalkyl, 3- to 12-membered heterocycloalkyl, $C_{6-12}$ aryl, or 5- to 12-membered heteroaryl;
the substituents or groups described above may be optionally substituted with one or more substituents.

In some embodiments, the proviso is that:

when $R^1$ is selected from the group consisting of hydrogen, halogen, OH, $C_{1-10}$ alkyl, halogenated $C_{1-10}$ alkyl, $C_{1-10}$ alkoxy, $C_{3-10}$ cycloalkyl, 3- to 10-membered heterocycloalkyl, and 3- to 10-membered heterocycloalkenyl, n is selected from the group consisting of 1, 2, 3, 4, and 5, one of $R^3$ is selected from the group consisting of $C_{2-10}$ alkenyl and $C_{2-10}$ alkynyl, the $C_{2-10}$ alkenyl or $C_{2-10}$ alkynyl being optionally substituted with one or more groups selected from OH, $NH_2$, CN, and halogen, and the others of $R^3$ (if present, i.e., when n is selected from the group consisting of 2, 3, 4, and 5) are each independently selected from the group consisting of OH, $NH_2$, CN, halogen, $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, and 5- to 10-membered heteroaryl, the $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, or 5- to 10-membered heteroaryl being optionally substituted with one or more groups selected from the group consisting of OH, $NH_2$, CN, and halogen.

In some embodiments, $R^3$ are each independently selected from the group consisting of OH, $NH_2$, CN, halogen, $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, and 5- to 10-membered heteroaryl, the $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, or 5- to 10-membered heteroaryl being optionally substituted with one or more groups selected from the group consisting of OH, $NH_2$, CN, and halogen.

In some embodiments, ring A is selected from the group consisting of $C_{6-10}$ aryl and 5- to 15-membered heterocyclyl.

In some embodiments, R and R' are each independently selected from the group consisting of H, $C_{1-6}$ alkyl, and $C_{3-8}$ cycloalkyl.

In some embodiments, R and R' are each independently selected from the group consisting of H, $C_{1-3}$ alkyl, and $C_{3-6}$ cycloalkyl.

In some embodiments, R and R' are each independently selected from the group consisting of H and $C_{1-3}$ alkyl. In some embodiments, R and R' are selected from H.

In some embodiments, $R^1$ is selected from the group consisting of hydrogen, halogen, OH, $C_{1-6}$ alkyl, one or more halogenated $C_{1-6}$ alkyl, $C_{1-6}$ alkoxy, $C_{3-6}$ cycloalkyl, 3- to 6-membered heterocycloalkyl, 3- to 6-membered heterocycloalkenyl, $C_{2-6}$ alkenyl, $C_{2-6}$ alkynyl, $-NHR^a$, and $-NR^aR^b$.

In some embodiments, $R^1$ is selected from the group consisting of hydrogen, halogen, OH, $C_{1-6}$ alkyl, $C_{2-6}$ alkenyl, $C_{2-6}$ alkynyl, $-NHR^a$, and $-NR^aR^b$.

In some embodiments, $R^1$ is selected from the group consisting of hydrogen, halogen, $NH_2$, $C_{2-6}$ alkenyl, and $C_{2-6}$ alkynyl.

In some embodiments, $R^1$ is selected from the group consisting of hydrogen, F, Cl, Br, $NH_2$, $C_{2-4}$ alkenyl, and $C_{2-4}$ alkynyl.

In some embodiments, $R^1$ is selected from the group consisting of hydrogen, Cl, $NH_2$, ethenyl, ethynyl, and propynyl. In some embodiments, $R^1$ is selected from the group consisting of hydrogen, Cl, $NH_2$, ethenyl, ethynyl, and 1-propynyl.

In some other embodiments, $R^1$ is selected from the group consisting of hydrogen, $NH_2$, $C_{2-6}$ alkenyl, and $C_{2-6}$ alkynyl.

In some other embodiments, $R^1$ is selected from the group consisting of hydrogen, $NH_2$, $C_{2-4}$ alkenyl, and $C_{2-4}$ alkynyl. In some other embodiments, $R^1$ is selected from the group consisting of hydrogen, $NH_2$, $C_{2-3}$ alkenyl, and $C_{2-3}$ alkynyl.

In some other embodiments, $R^1$ is selected from the group consisting of hydrogen, $NH_2$, ethenyl, and ethynyl.

In some specific embodiments, $R^1$ is selected from the group consisting of hydrogen, halogen, OH, $C_{1-6}$ alkyl, one or more halogenated $C_{1-6}$ alkyl, $C_{1-6}$ alkoxy, $C_{3-6}$ cycloalkyl, 3- to 6-membered heterocycloalkyl, and 3- to 6-membered heterocycloalkenyl. In some specific embodiments, $R^1$ is selected from the group consisting of hydrogen, halogen, OH, and $C_{1-6}$ alkyl. In some specific embodiments, $R^1$ is selected from hydrogen.

In some specific embodiments, $R^1$ is selected from the group consisting of $C_{2-6}$ alkenyl, $C_{2-6}$ alkynyl, $-NHR^a$, and $-NR^aR^b$. In some specific embodiments, $R^1$ is selected from the group consisting of $NH_2$, $C_{2-6}$ alkenyl, and $C_{2-6}$ alkynyl. In some specific embodiments, $R^1$ is selected from the group consisting of $NH_2$, $C_{2-4}$ alkenyl, and $C_{2-4}$ alkynyl. In some specific embodiments, $R^1$ is selected from the group consisting of $NH_2$, $C_{2-3}$ alkenyl, and $C_{2-3}$ alkynyl.

In some embodiments, $R^1$ is selected from the group consisting of $NH_2$, ethenyl, and ethynyl.

In some specific embodiments, $R^1$ is selected from the group consisting of $C_{2-6}$ alkenyl and $C_{2-6}$ alkynyl. In some specific embodiments, $R^1$ is selected from the group consisting of $C_{2-4}$ alkenyl and $C_{2-4}$ alkynyl. In some specific embodiments, $R^1$ is selected from the group consisting of $C_{2-3}$ alkenyl and $C_{2-3}$ alkynyl. In some embodiments, $R^1$ is selected from the group consisting of ethenyl and ethynyl. In some embodiments, $R^1$ is selected from the group consisting of ethenyl, ethynyl, and 1-propynyl. In some specific embodiments, $R^1$ is selected from the group consisting of $-NHR^a$ and $-NR^aR^b$. In some specific embodiments, $R^1$ is selected from $NH_2$. In some specific embodiments, $R^1$ is hydrogen.

In some embodiments, $R^a$ and $R^b$ are each independently selected from the group consisting of H, $C_{1-6}$ alkyl, $C_{3-6}$

cycloalkyl, $-COC_{1-6}$ alkyl, and $-S(O)_2C_{1-6}$ alkyl.

In some embodiments, $R^a$ and $R^b$ are each independently selected from the group consisting of H, $C_{1-4}$ alkyl, $C_{3-6}$ cycloalkyl, $-COC_{1-4}$ alkyl, and $-S(O)_2C_{1-4}$ alkyl.

In some embodiments, $R^a$ and $R^b$ are each independently selected from the group consisting of H, $C_{1-3}$ alkyl, $C_{3-5}$ cycloalkyl, $-COC_{1-3}$ alkyl, and $-S(O)_2C_{1-3}$ alkyl.

In some embodiments, $R^a$ and $R^b$ are each independently selected from the group consisting of H and $C_{1-3}$ alkyl. In some embodiments, $R^a$ and $R^b$ are selected from H.

In some embodiments, $X^1$ is selected from the group consisting of C and N. In some embodiments, $X^2$ is selected from the group consisting of CH and NH. In some embodiments, $X^3$ is selected from the group consisting of CH, $CH_2$, and N. In some embodiments, $X^4$ is selected from the group consisting of CH, N, and NH. In some embodiments, $X^5$ is selected from the group consisting of C and N.

In some embodiments, $X^1$ is selected from the group consisting of C and N, $X^2$ is selected from the group consisting of CH and NH, $X^3$ is selected from the group consisting of CH, $CH_2$, and N, $X^4$ is selected from the group consisting of CH, N, and NH, and $X^5$ is selected from the group consisting of C and N.

In some embodiments, $X^1$ and $X^5$ are each independently selected from the group consisting of C and N, and $X^2$, $X^3$, and $X^4$ are each independently selected from the group consisting of CH, $CH_2$, N, and NH.

In some embodiments, one or two of $X^1$, $X^2$, $X^3$, $X^4$, and $X^5$ are selected from the group consisting of N and NH; the others are selected from the group consisting of C, CH, and $CH_2$. In some embodiments, one of $X^1$, $X^2$, $X^3$, $X^4$, and $X^5$ is selected from the group consisting of N and NH; the others are selected from the group consisting of C, CH, and $CH_2$. In some embodiments, two of $X^1$, $X^2$, $X^3$, $X^4$, and $X^5$ are selected from the group consisting of N and NH; the others are selected from the group consisting of C, CH, and $CH_2$.

In some embodiments, the structural moiety

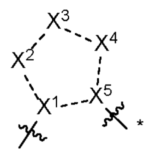

is selected from the group consisting of pyrrolyl, imidazolyl, dihydroimidazolyl, and pyrazolyl. In some embodiments, the structural moiety

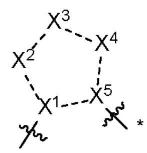

is selected from pyrazolyl.

In some embodiments, the structural moiety

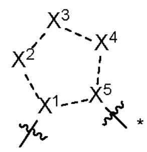

is selected from the group consisting of

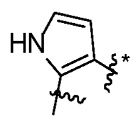

,

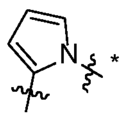, 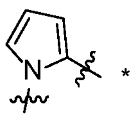, 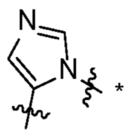, 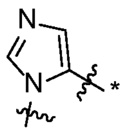, 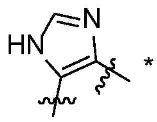, 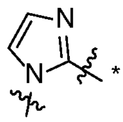, 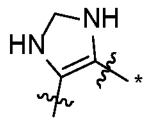,

and

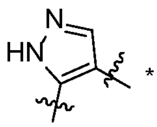,

wherein * indicates a corresponding site connected to

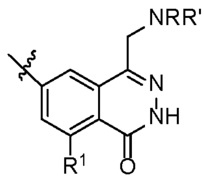.

In some specific embodiments, the structural moiety

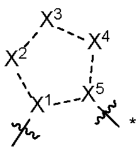

is selected from the group consisting of

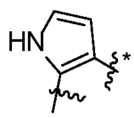,

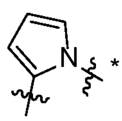, and 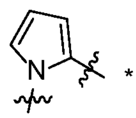,

wherein * indicates a corresponding site connected to

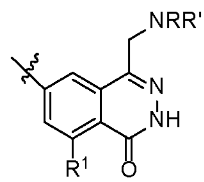.

In some specific embodiments, the structural moiety

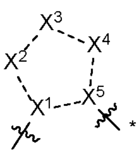

is selected from the group consisting of

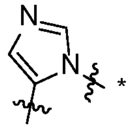,

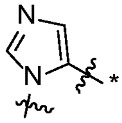, 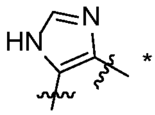, 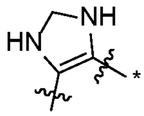, and 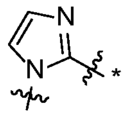,

wherein * indicates a corresponding site connected

to 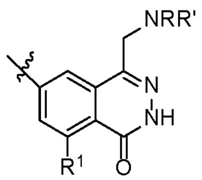.

In some specific embodiments, the structural moiety

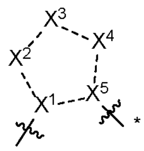

is selected from

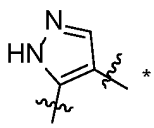,

wherein * indicates a corresponding site connected to

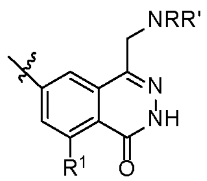.

In some embodiments, $R^2$ is a group connected to $X^2$, $X^3$, and $X^4$. In some embodiments, $R^2$ is a group connected to $X^2$. In some embodiments, $R^2$ is a group connected to $X^2$ and/or $X^3$. In some embodiments, $R^2$ is a group connected to $X^2$ and/or $X^4$.

In some embodiments, $R^2$ are each independently selected from the group consisting of =O, OH, $NH_2$, CN, halogen, $C_{1-6}$ alkyl, $C_{1-6}$ alkoxy, $C_{3-6}$ cycloalkyl, and 3- to 6-membered heterocycloalkyl, the $C_{1-6}$ alkyl, $C_{1-6}$ alkoxy, $C_{3-6}$ cycloalkyl, or 3- to 6-membered heterocycloalkyl being optionally substituted with one or more groups selected from the group consisting of OH, $NH_2$, CN, and halogen.

In some embodiments, $R^2$ are each independently selected from the group consisting of =O, OH, $NH_2$, CN, halogen, $C_{1-6}$ alkyl, and $C_{1-6}$ alkoxy, the $C_{1-6}$ alkyl or $C_{1-6}$ alkoxy being optionally substituted with one or more groups selected from the group consisting of OH, $NH_2$, CN, and halogen.

In some embodiments, $R^2$ are each independently selected from the group consisting of =O, OH, $NH_2$, CN,

halogen, and $C_{1-6}$ alkyl, the $C_{1-6}$ alkyl being optionally substituted with one or more groups selected from the group consisting of OH, $NH_2$, CN, and halogen.

In some embodiments, $R^2$ are each independently selected from the group consisting of =O and $C_{1-6}$ alkyl.

In some embodiments, $R^2$ are each independently selected from the group consisting of =O and $C_{1-3}$ alkyl.

In some embodiments, $R^2$ are each independently selected from the group consisting of =O and methyl.

In some embodiments, $R^2$ is methyl.

In some embodiments, m is selected from the group consisting of 1, 2, 3, and 4. In some embodiments, m is selected from the group consisting of 1, 2, and 3. In some embodiments, m is selected from the group consisting of 1 and 2. In some embodiments, m is 1.

In some embodiments, m is 1, and $R^2$ is connected to $X^2$. In some embodiments, m is 2, and two $R^2$ are connected to $X^2$ and $X^3$, respectively. In some embodiments, m is 2, and two $R^2$ are connected to $X^2$ and $X^4$, respectively. In some embodiments, $R^2$ is connected to $X^2$, and $X^2$ is selected from the group consisting of C and N. In some embodiments, $R^2$ is connected to $X^3$, and $X^3$ is C. In some embodiments, $R^2$ is connected to $X^4$, and $X^4$ is C.

In some embodiments, m is 1, $R^2$ is connected to $X^2$, and $R^2$ is methyl.

In some embodiments, the structural moiety

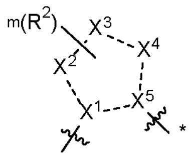

is selected from the group consisting of

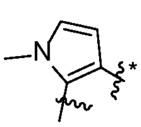, 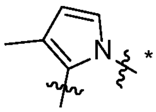, 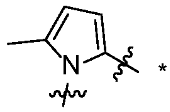, 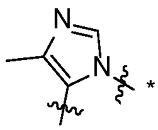, 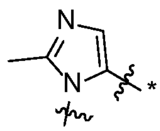, 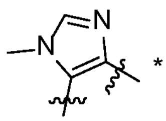,

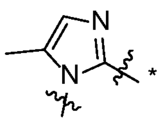, 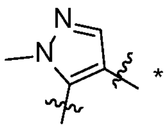, 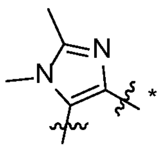, 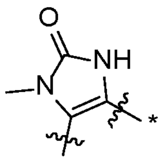, 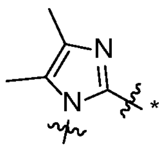, and 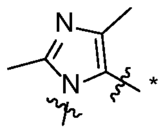,

wherein * indicates a corresponding site connected to

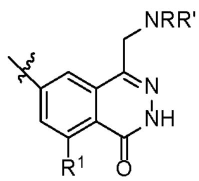.

In some embodiments, the structural moiety

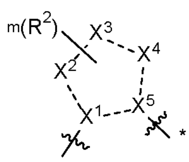

is selected from

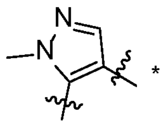

,

wherein * indicates a corresponding site connected to

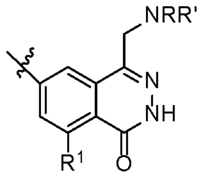

.

In some embodiments, ring A is selected from the group consisting of $C_{6-10}$ aryl and 5- to 15-membered heterocyclyl.

In some embodiments, ring A is selected from the group consisting of $C_{6-10}$ aryl and 5- to 12-membered heterocyclyl.

In some embodiments, ring A is selected from the group consisting of $C_{6-10}$ aryl, 5- to 10-membered heteroaryl, benzo 5- to 7-membered heterocycloalkenyl, and benzo $C_{5-7}$ cycloalkenyl.

In some embodiments, ring A is selected from the group consisting of $C_{6-10}$ aryl, 5- to 6-membered heteroaryl, benzo 5- to 6-membered heterocycloalkenyl, and benzo $C_{5-6}$ cycloalkenyl.

In some embodiments, ring A is selected from the group consisting of $C_{6-10}$ aryl and 5- to 10-membered heteroaryl. In some embodiments, ring A is selected from the group consisting of $C_{6-10}$ aryl and 5- to 6-membered heteroaryl. In some embodiments, ring A is selected from $C_{6-10}$ aryl. In some embodiments, ring A is selected from the group consisting of phenyl and $C_{10}$ aryl.

In some embodiments, ring A is selected from the group consisting of phenyl and naphthyl.

In some embodiments, the structural moiety

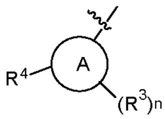

is selected from the group consisting of

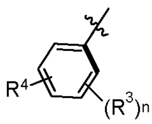

and

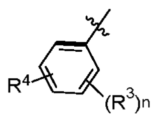

;

in some embodiments, the structural moiety

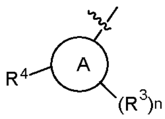

is selected from the group consisting of

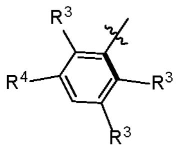

and

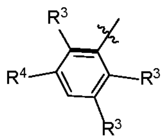

.

In some embodiments, the structural moiety

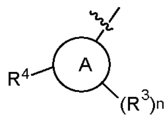

is selected from

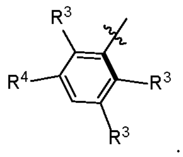

In some embodiments, the structural moiety

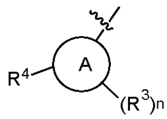

is selected from the group consisting of

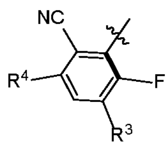

and

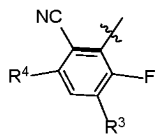

In some embodiments, the structural moiety

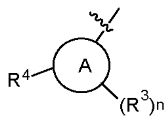

is selected from

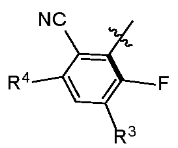

In some embodiments, the heteroatom in heterocyclyl, heteroaryl, or benzo 5- to 7-membered heterocycloalkenyl in ring A is selected from the group consisting of nitrogen, oxygen, sulfur, phosphorus, and boron; or the heteroatom is selected from the group consisting of nitrogen, oxygen, and sulfur; or the heteroatom is selected from the group consisting of nitrogen and oxygen. In some embodiments, the number of heteroatoms may be selected from the group consisting of 1, 2, 3, 4, and 5. In some embodiments, the number of heteroatoms may be selected from the group consisting of 1, 2, and 3.

In some embodiments, $R^3$ are each independently selected from the group consisting of OH, $NH_2$, CN, halogen, and the following groups optionally substituted with one or more $R^5$: $C_{1-10}$ alkyl, $C_{1-10}$ alkoxy, $C_{1-10}$ alkyl S-, $C_{1-10}$ alkyl NH-, ($C_{1-10}$ alkyl)$_2$ N-, $C_{1-10}$ alkyl NHC(O)-, ($C_{1-10}$ alkyl)$_2$ NC(O)-, $C_{1-10}$ alkyl C(O)NH-, $C_{1-10}$ alkyl OC(O)-, $C_{1-10}$ alkyl C(O)O-, $C_{2-10}$ alkenyl, $C_{2-10}$ alkynyl, $C_{3-10}$ cycloalkyl, 3- to 10-membered heterocycloalkyl, $C_{6-10}$ aryl, or 5- to 10-membered heteroaryl.

In some embodiments, $R^3$ are each independently selected from the group consisting of OH, $NH_2$, CN, halogen, and the following groups optionally substituted with one or more $R^5$: $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, or 5- to 10-membered heteroaryl.

In some embodiments, $R^3$ are each independently selected from the group consisting of OH, $NH_2$, CN, halogen, and the following groups optionally substituted with one or more $R^5$: $C_{1-6}$ alkyl, $C_{1-6}$ alkoxy, $C_{2-8}$ alkenyl, $C_{2-10}$ alkynyl, $C_{3-6}$ cycloalkyl, 3- to 6-membered heterocycloalkyl, $C_{6-10}$ aryl, or 5- to 6-membered heteroaryl.

In some embodiments, $R^3$ are each independently selected from the group consisting of OH, $NH_2$, CN, halogen, and the following groups optionally substituted with one or more $R^5$: $C_{1-6}$ alkyl, $C_{1-6}$ alkoxy, $C_{2-6}$ alkenyl, or $C_{2-8}$ alkynyl.

In some embodiments, $R^3$ are each independently selected from the group consisting of OH, $NH_2$, CN, halogen, $C_{2-6}$ alkenyl, and $C_{2-8}$ alkynyl, the $C_{2-6}$ alkenyl or $C_{2-8}$ alkynyl being optionally substituted with one or more $R^5$. In some embodiments, $R^3$ are each independently selected from the group consisting of OH, $NH_2$, CN, halogen, and $C_{2-8}$ alkynyl (or $C_{2-6}$ alkynyl), the $C_{2-8}$ alkynyl (or $C_{2-6}$ alkynyl) being optionally substituted with one or more $R^5$.

In some embodiments, $R^3$ are each independently selected from the group consisting of OH, $NH_2$, CN, halogen, $C_{2-3}$ alkenyl, and $C_{2-6}$ alkynyl, the $C_{2-3}$ alkenyl or $C_{2-6}$ alkynyl being optionally substituted with one or more $R^5$. In some embodiments, $R^3$ are each independently selected from the group consisting of OH, $NH_2$, CN, halogen, $C_{2-3}$ alkenyl, and $C_{2-4}$ alkynyl, the $C_{2-3}$ alkenyl or $C_{2-4}$ alkynyl being optionally substituted with one or more $R^5$.

In some embodiments, $R^3$ are each independently selected from the group consisting of OH, $NH_2$, CN, F, Cl, Br, I, and the following groups optionally substituted with one or more $R^5$: ethenyl, ethynyl, propynyl, butynyl, dimethylbutynyl (e.g., 3,3-dimethylbutynyl), or methylpenynyl (e.g., 4-methylpenynyl). In some embodiments, $R^3$ are each independently selected from the group consisting of OH, $NH_2$, CN, F, Cl, Br, I, and the following groups optionally substituted with one or more $R^5$: ethynyl, propynyl, butynyl, dimethylbutynyl (e.g., 3,3-dimethylbutynyl), or methylpenynyl (e.g., 4-methylpenynyl).

In some embodiments, $R^3$ are each independently selected from the group consisting of OH, $NH_2$, CN, F, Cl, Br, I, ethenyl, ethynyl, propynyl (e.g., 1-propynyl), butynyl (e.g., 1-butynyl or 2-butynyl), dimethylbutynyl (e.g., 3,3-dimethylbutynyl), and methylpentynyl (e.g., 4-methylpentynyl or 4-methyl-1-pentynyl), the ethenyl, ethynyl, propynyl (e.g., 1-propynyl), butynyl (e.g., 1-butynyl or 2-butynyl), dimethylbutynyl (e.g., 3,3-dimethylbutynyl), or methylpentynyl (e.g., 4-methylpentynyl or 4-methyl-1-pentynyl) being optionally substituted with one or more groups selected from the group consisting of OH, $NH_2$, CN, halogen (e.g., fluorine, chlorine, bromine, or iodine), methoxy, ethoxy, cyclopropyl, cyclopentyl, cyclohexyl, phenyl, thienyl, pyrazolyl, pyrimidinyl, pyrazinyl, furanyl, and pyrrolyl, further optionally, the methoxy, ethoxy, cyclopropyl, cyclopentyl, cyclohexyl, phenyl, thienyl, pyrazolyl, pyrimidinyl, pyrazinyl, furanyl, or pyrrolyl being substituted with one or more $R^d$.

In some embodiments, $R^3$ are each independently selected from the group consisting of $C_{2-6}$ alkenyl and $C_{2-8}$ alkynyl, the $C_{2-6}$ alkenyl or $C_{2-8}$ alkynyl being optionally substituted with one or more $R^5$. In some embodiments, $R^3$ are each independently selected from the group consisting of $C_{2-3}$ alkenyl and $C_{2-6}$ alkynyl, the $C_{2-3}$ alkenyl or $C_{2-6}$ alkynyl being optionally substituted with one or more $R^5$. In some embodiments, $R^3$ are each independently selected from the group consisting of $C_{2-3}$ alkenyl and $C_{2-4}$ alkynyl, the $C_{2-3}$ alkenyl or $C_{2-4}$ alkynyl being optionally substituted with one or more $R^5$.

In some embodiments, $R^5$ is selected from the group consisting of OH, $NH_2$, CN, halogen, and the following groups optionally substituted with one or more $R^d$: $C_{1-8}$ alkyl, $C_{1-8}$ alkoxy, $C_{1-8}$ alkyl S-, $C_{1-8}$ alkyl NH-, $(C_{1-8}$ alkyl$)_2$ N-, $C_{2-8}$ alkenyl, $C_{3-8}$ cycloalkyl, 3- to 8-membered heterocycloalkyl, $C_{6-8}$ aryl, or 5- to 8-membered heteroaryl.

In some embodiments, $R^5$ is selected from the group consisting of OH, $NH_2$, CN, halogen, and the following groups optionally substituted with one or more $R^d$: $C_{1-6}$ alkyl, $C_{1-6}$ alkoxy, $C_{1-6}$ alkyl S-, $C_{1-6}$ alkyl NH-, $(C_{1-6}$ alkyl$)_2$ N-, $C_{2-6}$ alkenyl, $C_{3-6}$ cycloalkyl, 3- to 6-membered heterocycloalkyl, $C_{6-8}$ aryl, or 5- to 6-membered heteroaryl.

In some embodiments, $R^5$ is selected from the group consisting of OH, $NH_2$, CN, halogen, and the following groups optionally substituted with one or more $R^d$: $C_{1-4}$ alkyl, $C_{1-4}$ alkoxy, $C_{1-4}$ alkyl S-, $C_{1-4}$ alkyl NH-, $(C_{1-4}$ alkyl$)_2$ N-, $C_{2-4}$ alkenyl, $C_{3-6}$ cycloalkyl, 3- to 6-membered heterocycloalkyl, phenyl, or 5- to 6-membered heteroaryl.

In some embodiments, $R^5$ is selected from the group consisting of OH, $NH_2$, CN, fluorine, chlorine, bromine, and the following groups optionally substituted with one or more $R^d$: $C_{1-3}$ alkoxy, $C_{3-6}$ cycloalkyl, 4- to 6-membered heterocycloalkyl, phenyl, or 5- to 6-membered heteroaryl.

In some embodiments, $R^5$ is selected from the group consisting of OH, $NH_2$, CN, fluorine, chlorine, and the following groups optionally substituted with one or more $R^d$: $C_{1-2}$ alkoxy, $C_{3-6}$ cycloalkyl, phenyl, and 5- to 6-membered heteroaryl. In some embodiments, $R^5$ is selected from the group consisting of OH, $NH_2$, CN, fluorine, chlorine, bromine, and the following groups optionally substituted with one or more $R^d$: methoxy, ethoxy, cyclopropyl, cyclobutyl, cyclopentyl, azetidinyl, piperidinyl, dioxanyl, piperazinyl, morpholinyl, tetrahydropyranyl, cyclohexyl, phenyl, pyrrolyl, furanyl, thienyl, pyrazolyl, imidazolyl, pyridazinyl, pyrimidinyl, or pyrazinyl.

In some embodiments, $R^5$ is selected from the group consisting of OH, fluorine, and the following groups optionally substituted with one or more $R^d$: methoxy, cyclopropyl, cyclopentyl, cyclohexyl, phenyl, pyrrolyl, furanyl, thienyl, pyrazolyl, pyrimidinyl, or pyrazinyl.

In some embodiments, $R^5$ is selected from the group consisting of the following groups optionally substituted with one or more $R^d$: $C_{3-6}$ cycloalkyl, phenyl, and 5- to 6-membered heteroaryl.

In some embodiments, $R^5$ is selected from the group consisting of the following groups optionally substituted with one or more $R^d$: cyclopropyl, cyclopentyl, cyclohexyl, phenyl, pyrrolyl, furanyl, thienyl, pyrazolyl, pyrimidinyl, or pyrazinyl.

In some embodiments, $R^d$ is selected from the group consisting of OH, $NH_2$, CN, halogen, $C_{1-6}$ alkyl, $C_{1-6}$ alkoxy, $C_{1-6}$ alkyl S-, $C_{1-6}$ alkyl NH-, and $(C_{1-6}$ alkyl$)_2$ N-, the $C_{1-6}$ alkyl, $C_{1-6}$ alkoxy, $C_{1-6}$ alkyl S-, $C_{1-6}$ alkyl NH-, or $(C_{1-6}$ alkyl$)_2$ N-

being optionally substituted with one or more groups selected from the group consisting of OH, $NH_2$, CN, or halogen.

In some embodiments, $R^d$ is selected from the group consisting of OH, $NH_2$, CN, halogen, $C_{1-4}$ alkyl, and $C_{1-4}$ haloalkyl.

In some embodiments, $R^d$ is selected from the group consisting of OH, $NH_2$, CN, F, Cl, Br, $C_{1-3}$ alkyl, and $C_{1-3}$ haloalkyl. In some embodiments, $R^d$ is selected from the group consisting of CN, $C_{1-3}$ alkyl, and $C_{1-3}$ haloalkyl. In some embodiments, $R^d$ is selected from the group consisting of OH, CN, F, Cl, Br, I, methyl, ethyl, and trifluoromethyl.

In some embodiments, $R^d$ is selected from the group consisting of CN, methyl, and trifluoromethyl.

In some embodiments, $R^3$ are each independently selected from the group consisting of OH, CN, F, Cl, propynyl (1-propynyl), butynyl (2-butynyl),

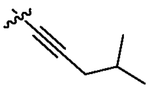, 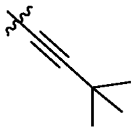, 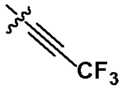, 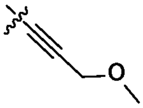, 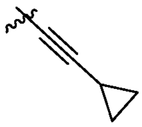,

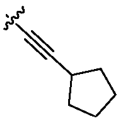, 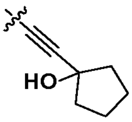, 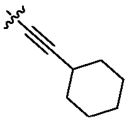, 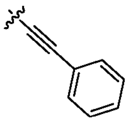, 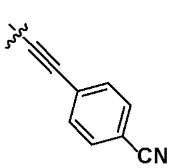, 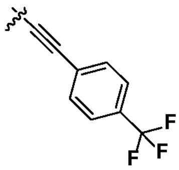, 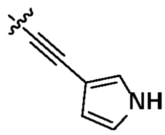,

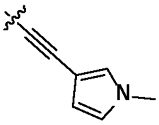, 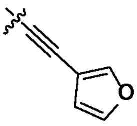, 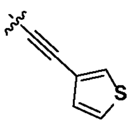, 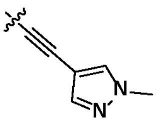, 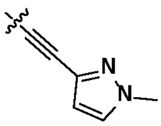, 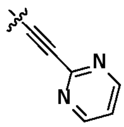, 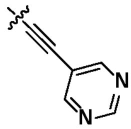,

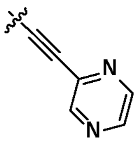,

ethenyl,

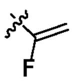, 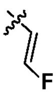, 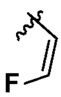, 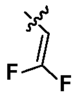, and 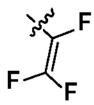.

In some embodiments, the structural moiety

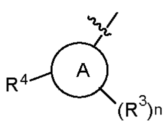

is selected from the group consisting of

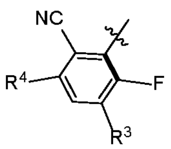 and 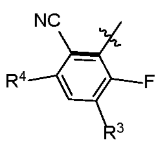,

and $R^3$ is selected from the group consisting of Cl, 1-propynyl, 2-butynyl,

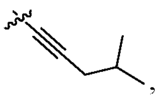, 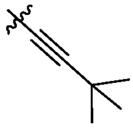, 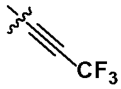, 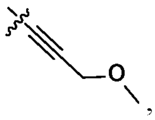, 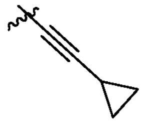, 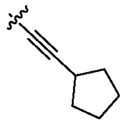, 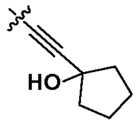, 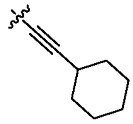,

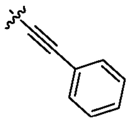, 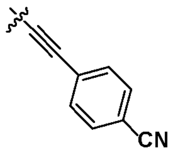, 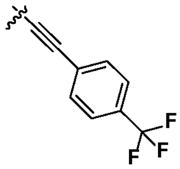, 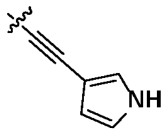, 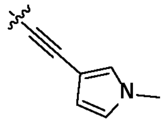, 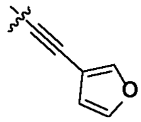, 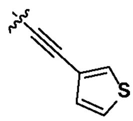,

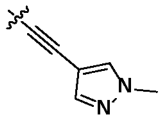, 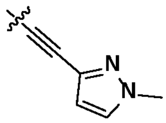, 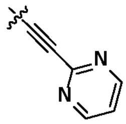, 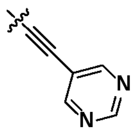, 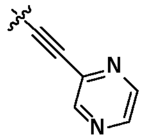,

ethenyl,

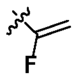, 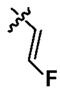, 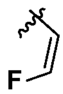,

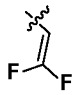, and 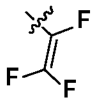.

In some other embodiments, $R^3$ are each independently selected from the group consisting of OH, $NH_2$, CN, halogen, $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, and 5- to 10-membered heteroaryl, the $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, or 5- to 10-membered heteroaryl being optionally substituted with one or more groups selected from the group consisting of OH, $NH_2$, CN, and halogen. In some other embodiments, $R^3$ are each independently selected from the group consisting of OH, $NH_2$, CN, halogen, $C_{1-6}$ alkyl, $C_{1-6}$ alkoxy, $C_{2-6}$ alkenyl, $C_{2-6}$ alkynyl, $C_{3-6}$ cycloalkyl, 3- to 6-membered heterocycloalkyl, $C_{6-10}$ aryl, and 5- to 6-membered heteroaryl, the $C_{1-6}$ alkyl, $C_{1-6}$ alkoxy, $C_{2-6}$ alkenyl, $C_{2-6}$ alkynyl, $C_{3-6}$ cycloalkyl, 3- to 6-membered heterocycloalkyl, $C_{6-10}$ aryl, or 5- to 6-membered heteroaryl being optionally substituted with one or more groups selected from the group consisting of OH, $NH_2$, CN, and halogen.

In some other embodiments, $R^3$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl, the $C_{1-6}$ alkyl, $C_{1-6}$ alkoxy, $C_{2-6}$ alkenyl, or $C_{2-6}$ alkynyl being optionally substituted with one or more groups selected from the group consisting of OH, $NH_2$, CN, and halogen.

In some other embodiments, $R^3$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl.

In some other embodiments, $R^3$ are each independently selected from the group consisting of OH, $NH_2$, CN, halogen, $C_{2-6}$ alkenyl, and $C_{2-6}$ alkynyl.

In some other embodiments, $R^3$ are each independently selected from the group consisting of CN, halogen, $C_{2-6}$ alkenyl, and $C_{2-6}$ alkynyl.

In some other embodiments, $R^3$ are each independently selected from the group consisting of CN, halogen, $C_{2-4}$ alkenyl, and $C_{2-4}$ alkynyl.

In some other embodiments, $R^3$ are each independently selected from the group consisting of CN, halogen, $C_{2-3}$ alkenyl, and $C_{2-3}$ alkynyl.

In some other embodiments, $R^3$ are each independently selected from the group consisting of CN, fluorine, chlorine, ethenyl, and propynyl (1-propynyl).

In some embodiments, n is selected from the group consisting of 1, 2, and 3. In some embodiments, n is selected from the group consisting of 2, and 3. In some embodiments, n is 3.

In some embodiments, $R^4$ is selected from being absent. In some embodiments, $R^4$ is selected from -X-cycB.

In some embodiments, X is selected from the group consisting of NH, N($C_{1-6}$ alkyl), O, and S. In some embodiments, X is selected from the group consisting of NH, N($C_{1-3}$ alkyl), O, and S.

In some embodiments, X is selected from the group consisting of NH, O, and S. In some embodiments, X is selected from the group consisting of NH and O. In some embodiments, X is selected from O.

In some embodiments, cycB is selected from the group consisting of $C_{3-6}$ cycloalkyl, 3- to 6-membered heterocycloalkyl, $C_{6-10}$ aryl, and 5- to 6-membered heteroaryl, the $C_{3-6}$ cycloalkyl, 3- to 6-membered heterocycloalkyl, $C_{6-10}$ aryl, or 5- to 6-membered heteroaryl being optionally substituted with one or more $R^c$.

In some embodiments, cycB is selected from the group consisting of $C_{3-10}$ cycloalkyl and 3- to 10-membered heterocycloalkyl, the $C_{3-10}$ cycloalkyl or 3- to 10-membered heterocycloalkyl being optionally substituted with one or more $R^c$.

In some embodiments, cycB is selected from the group consisting of $C_{3-6}$ cycloalkyl and 3- to 6-membered heterocycloalkyl, the $C_{3-6}$ cycloalkyl or 3- to 6-membered heterocycloalkyl being optionally substituted with one or more $R^c$.

In some embodiments, cycB is selected from the group consisting of $C_{3-4}$ cycloalkyl and 3- to 4-membered heterocycloalkyl, the $C_{3-4}$ cycloalkyl or 3- to 4-membered heterocycloalkyl being optionally substituted with one or more $R^c$.

In some embodiments, cycB is selected from $C_{3-10}$ cycloalkyl, the $C_{3-10}$ cycloalkyl being optionally substituted with one or more $R^c$. In some embodiments, cycB is selected from $C_{3-6}$ cycloalkyl, the $C_{3-6}$ cycloalkyl being optionally substituted with one or more $R^c$. In some embodiments, cycB is selected from $C_{3-4}$ cycloalkyl, the $C_{3-4}$ cycloalkyl being optionally substituted with one or more $R^c$. In some embodiments, cycB is selected from cyclopropyl, the cyclopropyl being optionally substituted with one or more $R^c$. In some embodiments, cycB is cyclopropyl.

In some embodiments, $R^c$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl, the $C_{1-6}$ alkyl, $C_{1-6}$ alkoxy, $C_{2-6}$ alkenyl, or $C_{2-6}$ alkynyl being optionally substituted with one or more groups selected from the group consisting of OH, $NH_2$, CN, and halogen. In some embodiments, $R^c$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl.

In some embodiments, $R^c$ are each independently selected from the group consisting of OH, $NH_2$, CN, halogen, and $C_{1-6}$ alkyl.

In some embodiments, $R^c$ are each independently selected from the group consisting of halogen and $C_{1-6}$ alkyl. In some embodiments, $R^c$ are each independently selected from the group consisting of halogen (e.g., fluorine, chlorine, bromine, or iodine) and $C_{1-3}$ alkyl.

In some embodiments, the structural moiety

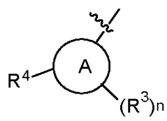

is selected from the group consisting of

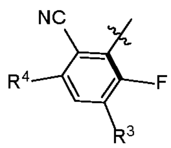

and

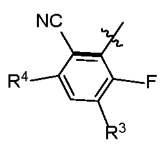

,

and $R^4$ is -O-cyclopropyl.

In some embodiments, the compound of formula I, the stereoisomer thereof, or the pharmaceutically acceptable salt thereof is selected from the group consisting of compounds of formulas II, III, IV, II-1, II-1A, II-1B, III-1, III-1A, III-1B, IV-1, IV-1A, IV-1B, VI, VI-1A, VI-1B, VI-1C, VI-1D, VI-1E, VII, VII-1, VII-1A, and VII-1B, stereoisomers thereof, or pharmaceutically acceptable salts thereof,

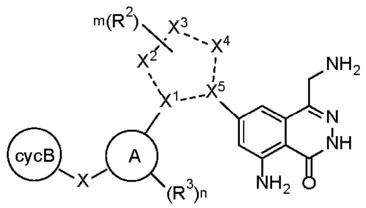
II

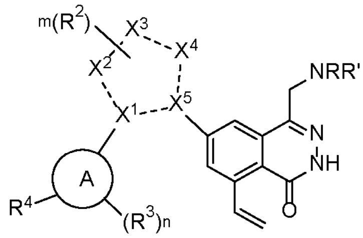
III

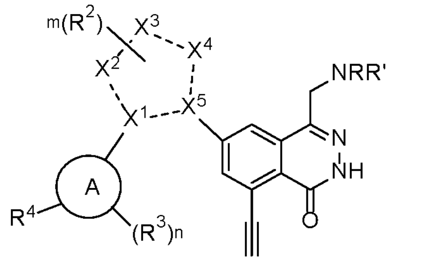
IV

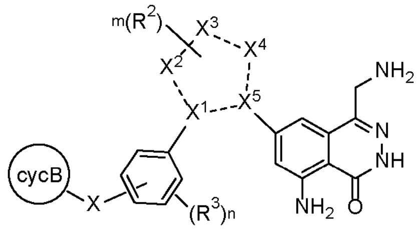
II-1

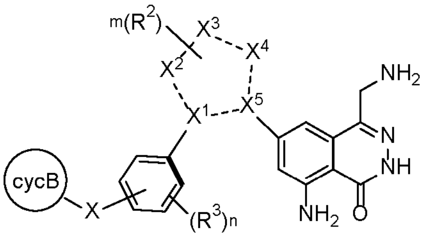
II-1A

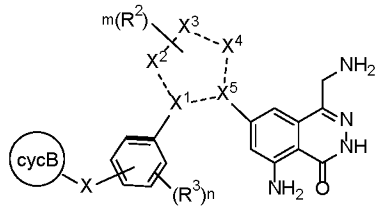
II-1B

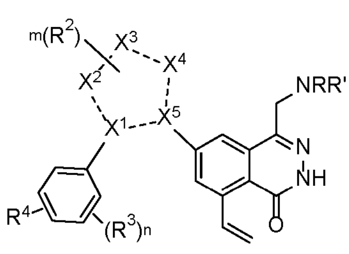
III-1

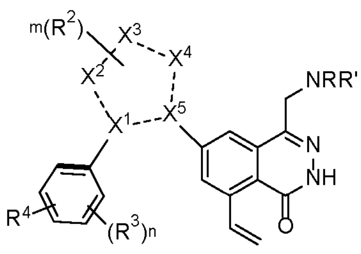
III-1A

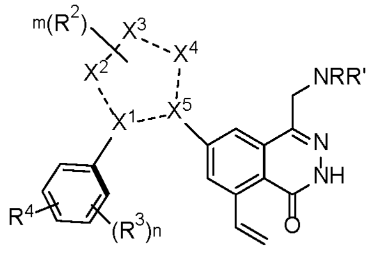
III-1B

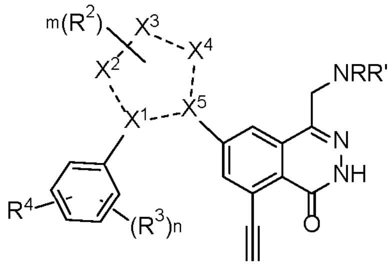
IV-1

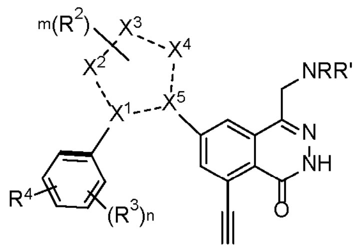
IV-1A

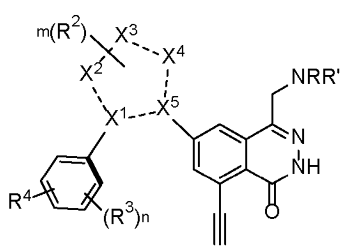
IV-1B

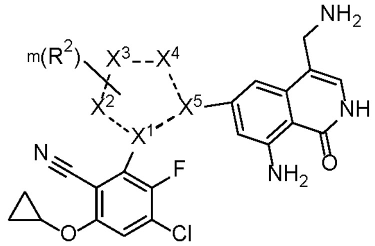
VI

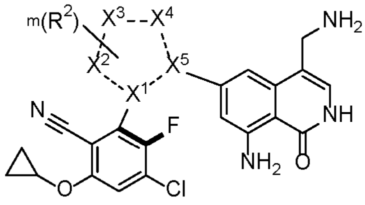
VI-1A

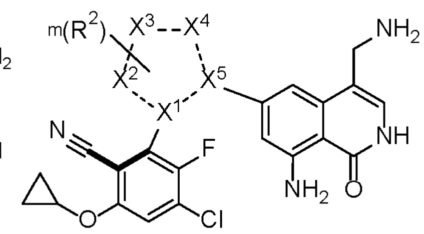
VI-1B

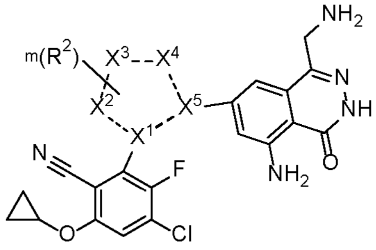

VI-1C

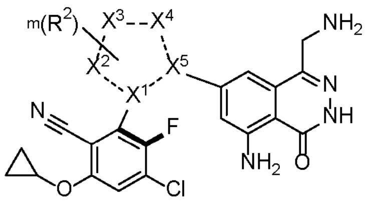

VI-1D

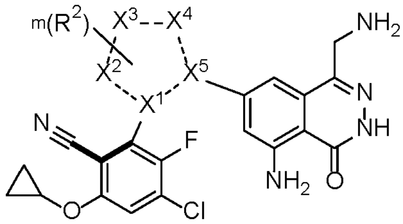

VI-1E

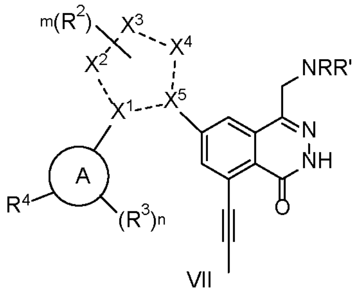

VII

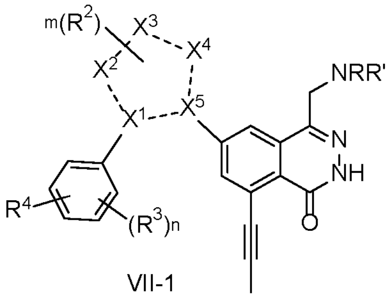

VII-1

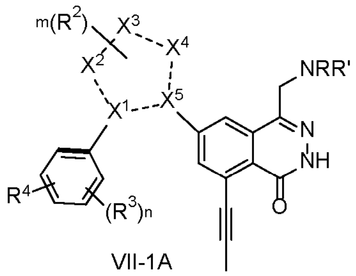

VII-1A

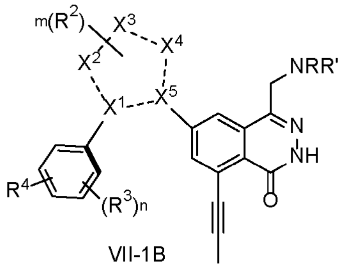

VII-1B

wherein R, R', $X^1$, $X^2$, $X^3$, $X^4$, $X^5$, $R^2$, m, ring A, $R^3$, n, X, cycB, and $R^4$ are as defined in the present disclosure.

In some embodiments, the compound of formula I, the stereoisomer thereof, or the pharmaceutically acceptable salt thereof is selected from a compound of formula V, a stereoisomer thereof, or a pharmaceutically acceptable salt thereof,

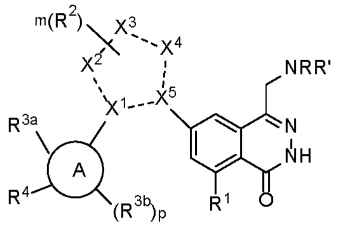

V

wherein R, R', $X^1$, $X^2$, $X^3$, $X^4$, $X^5$, $R^2$, m, ring A, and $R^4$ are as defined in the present disclosure;

$R^1$ is selected from the group consisting of hydrogen, halogen, OH, $C_{1-10}$ alkyl, halogenated $C_{1-10}$ alkyl, $C_{1-10}$ alkoxy, $C_{3-10}$ cycloalkyl, 3- to 10-membered heterocycloalkyl, 3- to 10-membered heterocycloalkenyl, $C_{2-10}$ alkenyl, $C_{2-10}$ alkynyl, $-NHR^a$, and $-NR^aR^b$;

p is selected from the group consisting of 0, 1, 2, 3, and 4;

$R^{3a}$ is selected from the group consisting of $C_{2-10}$ alkenyl and $C_{2-10}$ alkynyl, the $C_{2-10}$ alkenyl or $C_{2-10}$ alkynyl being optionally substituted with one or more $R^5$;

$R^5$ is selected from the group consisting of OH, $NH_2$, CN, halogen, and the following groups optionally substituted with one or more $R^d$: $C_{1-10}$ alkyl, $C_{1-10}$ alkoxy, $C_{1-10}$ alkyl S-, $C_{1-10}$ alkyl NH-, $(C_{1-10}$ alkyl$)_2$N-, $C_{2-10}$ alkenyl, $C_{3-10}$ cycloalkyl, 3- to 10-membered heterocycloalkyl, $C_{6-10}$ aryl, or 5- to 10-membered heteroaryl;

$R^d$ is selected from the group consisting of OH, $NH_2$, CN, COOH, CHO, halogen, $C_{1-10}$ alkyl, $C_{1-10}$ alkoxy, $C_{1-10}$ alkyl

S-, $C_{1-10}$ alkyl NH-, and $(C_{1-10}\ alkyl)_2$ N-, the $C_{1-10}$ alkyl, $C_{1-10}$ alkoxy, $C_{1-10}$ alkyl S-, $C_{1-10}$ alkyl NH-, or $(C_{1-10}\ alkyl)_2$ N-being optionally substituted with one or more groups selected from the group consisting of OH, $NH_2$, CN, and halogen; $R^{3b}$ are each independently selected from the group consisting of OH, $NH_2$, CN, halogen, $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, and 5- to 10-membered heteroaryl, the $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, or 5- to 10-membered heteroaryl being optionally substituted with one or more groups selected from the group consisting of OH, $NH_2$, CN, and halogen.

In some embodiments, the compound of formula I, the stereoisomer thereof, or the pharmaceutically acceptable salt thereof is selected from the group consisting of compounds of formulas V-1, V-2, and V-3, stereoisomers thereof, or pharmaceutically acceptable salts thereof,

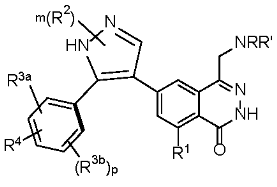
V-1

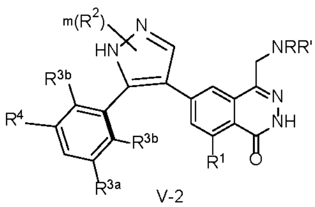
V-2

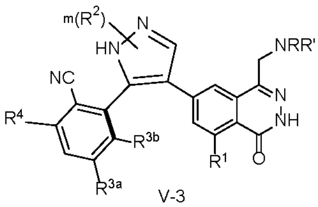
V-3

wherein $R^1$, R, R', $R^2$, m, $R^{3a}$, $R^{3b}$, p, and $R^4$ are as defined in the present disclosure.

In some embodiments, the structural moiety

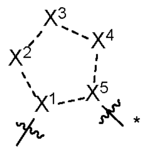

is as defined in the present application.

In some embodiments, the structural moiety

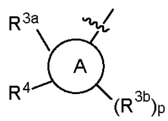

is selected from the group consisting of

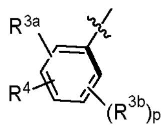

and

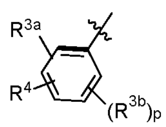

.

In some embodiments, the structural moiety

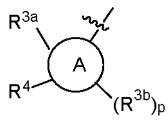

is selected from the group consisting of

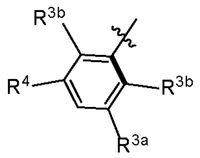 and 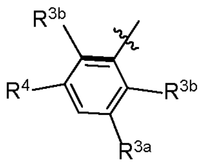 .

In some embodiments, the structural moiety

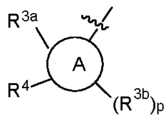

is selected from the group consisting of

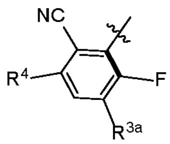 and 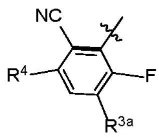 .

In some specific embodiments, the structural moiety

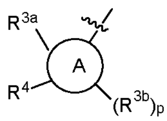

is selected from

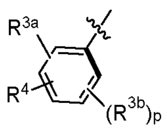 .

In some embodiments, the structural moiety

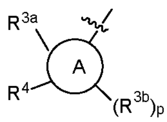

is selected from

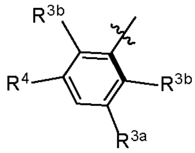 .

In some embodiments, the structural moiety

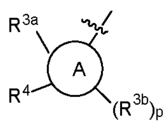 , 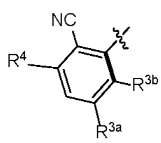 , or 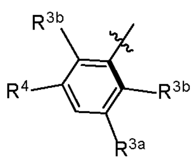

is selected from

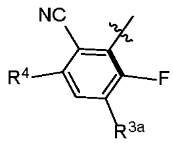

In some embodiments, $R^1$ is selected from the group consisting of hydrogen, halogen, OH, $C_{1-6}$ alkyl, halogenated $C_{1-6}$ alkyl, $C_{1-6}$ alkoxy, $C_{3-6}$ cycloalkyl, 3- to 6-membered heterocycloalkyl, 3- to 6-membered heterocycloalkenyl, $C_{2-6}$ alkenyl, $C_{2-6}$ alkynyl, $-NHR^a$, and $-NR^aR^b$.

In some embodiments, $R^1$ is selected from the group consisting of hydrogen, halogen, OH, $C_{1-6}$ alkyl, $C_{2-6}$ alkenyl, $C_{2-6}$ alkynyl, $-NHR^a$, and $-NR^aR^b$.

In some embodiments, $R^1$ is selected from the group consisting of hydrogen, halogen, $NH_2$, $C_{2-6}$ alkenyl, and $C_{2-6}$ alkynyl.

In some embodiments, $R^1$ is selected from the group consisting of hydrogen, F, Cl, Br, I, $NH_2$, $C_{2-4}$ alkenyl, and $C_{2-4}$ alkynyl.

In some embodiments, $R^1$ is selected from the group consisting of hydrogen, F, Cl, $NH_2$, $C_{2-3}$ alkenyl, and $C_{2-3}$ alkynyl.

In some embodiments, $R^1$ is selected from the group consisting of hydrogen, Cl, $NH_2$, ethenyl, ethynyl, and propynyl.

In some other embodiments, $R^1$ is selected from the group consisting of hydrogen, $NH_2$, $C_{2-6}$ alkenyl, and $C_{2-6}$ alkynyl.

In some other embodiments, $R^1$ is selected from the group consisting of hydrogen, $NH_2$, $C_{2-4}$ alkenyl, and $C_{2-4}$ alkynyl.

In some other embodiments, $R^1$ is selected from the group consisting of hydrogen, $NH_2$, $C_{2-3}$ alkenyl, and $C_{2-3}$ alkynyl.

In some other embodiments, $R^1$ is selected from the group consisting of hydrogen, $NH_2$, ethenyl, and ethynyl.

In some other specific embodiments, $R^1$ is selected from the group consisting of hydrogen, halogen, OH, $C_{1-6}$ alkyl, halogenated $C_{1-6}$ alkyl, $C_{1-6}$ alkoxy, $C_{3-6}$ cycloalkyl, 3- to 6-membered heterocycloalkyl, and 3- to 6-membered heterocycloalkenyl. In some specific embodiments, $R^1$ is selected from the group consisting of hydrogen, halogen, OH, and $C_{1-6}$ alkyl. In some specific embodiments, $R^1$ is selected from hydrogen.

In some other specific embodiments, $R^1$ is selected from the group consisting of $C_{2-6}$ alkenyl, $C_{2-6}$ alkynyl, $-NHR^a$, and $-NR^aR^b$. In some specific embodiments, $R^1$ is selected from the group consisting of $NH_2$, $C_{2-6}$ alkenyl, and $C_{2-6}$ alkynyl. In some specific embodiments, $R^1$ is selected from the group consisting of $NH_2$, $C_{2-4}$ alkenyl, and $C_{2-4}$ alkynyl. In some specific embodiments, $R^1$ is selected from the group consisting of $NH_2$, $C_{2-3}$ alkenyl, and $C_{2-3}$ alkynyl. In some embodiments, $R^1$ is selected from the group consisting of $NH_2$, ethenyl, and ethynyl.

In some specific embodiments, $R^1$ is selected from the group consisting of $C_{2-6}$ alkenyl and $C_{2-6}$ alkynyl. In some specific embodiments, $R^1$ is selected from the group consisting of $C_{2-4}$ alkenyl and $C_{2-4}$ alkynyl. In some specific embodiments, $R^1$ is selected from the group consisting of $C_{2-3}$ alkenyl and $C_{2-3}$ alkynyl. In some embodiments, $R^1$ is selected from the group consisting of ethenyl and ethynyl. In some specific embodiments, $R^1$ is selected from the group consisting of $-NHR^a$ and $-NR^aR^b$. In some specific embodiments, $R^1$ is selected from $NH_2$.

In some embodiments, p is selected from the group consisting of 0, 1, 2, and 3. In some embodiments, p is selected from the group consisting of 0, 1, and 2.

In some embodiments, $R^{3a}$ is selected from the group consisting of $C_{2-6}$ alkenyl and $C_{2-6}$ alkynyl, the $C_{2-6}$ alkenyl or $C_{2-6}$ alkynyl being optionally substituted with one or more $R^5$.

In some embodiments, $R^{3a}$ is selected from the group consisting of $C_{2-4}$ alkenyl and $C_{2-4}$ alkynyl, the $C_{2-4}$ alkenyl or $C_{2-4}$ alkynyl being optionally substituted with one or more $R^5$.

In some embodiments, $R^5$ or $R^d$ is as defined in the present disclosure.

In some embodiments, $R^{3a}$ is selected from the group consisting of propynyl, butynyl,

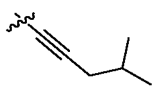

,

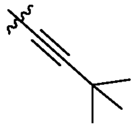, 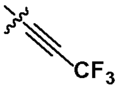, 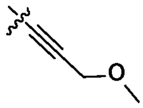, 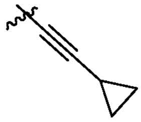, 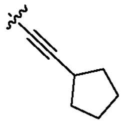, 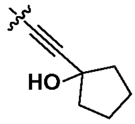, 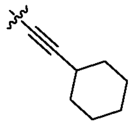, 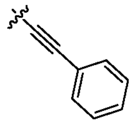,

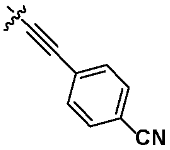, 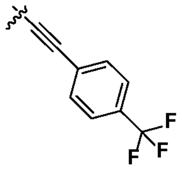, 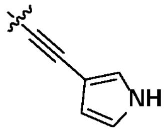, 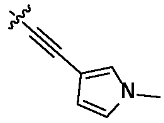, 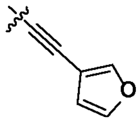, 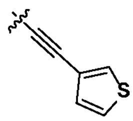,

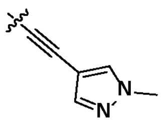, 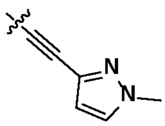, 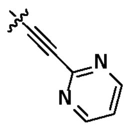, 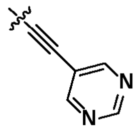, 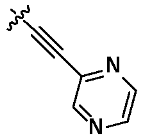,

ethenyl,

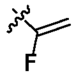, 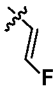, 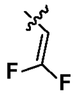,

and

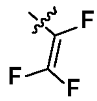.

In some other embodiments, $R^{3a}$ is selected from the group consisting of $C_{2-10}$ alkenyl and $C_{2-10}$ alkynyl, the $C_{2-10}$ alkenyl or $C_{2-10}$ alkynyl being optionally substituted with one or more groups selected from the group consisting of OH, $NH_2$, CN, and halogen.

In some other embodiments, $R^{3a}$ is selected from the group consisting of $C_{2-6}$ alkenyl and $C_{2-6}$ alkynyl, the $C_{2-6}$ alkenyl or $C_{2-6}$ alkynyl being optionally substituted with one or more groups selected from the group consisting of OH, $NH_2$, CN, and halogen.

In some other embodiments, $R^{3a}$ is selected from the group consisting of $C_{2-4}$ alkenyl and $C_{2-4}$ alkynyl, the $C_{2-4}$ alkenyl or $C_{2-4}$ alkynyl being optionally substituted with one or more groups selected from the group consisting of OH, $NH_2$, CN, and halogen.

In some other embodiments, $R^{3a}$ is selected from the group consisting of $C_{2-6}$ alkenyl and $C_{2-6}$ alkynyl.

In some other embodiments, $R^{3a}$ is selected from the group consisting of $C_{2-4}$ alkenyl and $C_{2-4}$ alkynyl.

In some other embodiments, $R^{3a}$ is selected from the group consisting of $C_{2-3}$ alkenyl and $C_{2-3}$ alkynyl.

In some other embodiments, $R^{3a}$ is selected from the group consisting of ethenyl and propynyl.

In some embodiments, $R^{3b}$ are each independently selected from the group consisting of OH, $NH_2$, CN, halogen, $C_{1-6}$ alkyl, $C_{1-6}$ alkoxy, $C_{2-6}$ alkenyl, $C_{2-6}$ alkynyl, $C_{3-6}$ cycloalkyl, 3- to 6-membered heterocycloalkyl, $C_{6-10}$ aryl, and 5- to 6-membered heteroaryl, the $C_{1-6}$ alkyl, $C_{1-6}$ alkoxy, $C_{2-6}$ alkenyl, $C_{2-6}$ alkynyl, $C_{3-6}$ cycloalkyl, 3- to 6-membered heterocycloalkyl, $C_{6-10}$ aryl, or 5- to 6-membered heteroaryl being optionally substituted with one or more groups selected from the group consisting of OH, $NH_2$, CN, and halogen.

In some embodiments, $R^{3b}$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl, the $C_{1-6}$ alkyl, $C_{1-6}$ alkoxy, $C_{2-6}$ alkenyl, and $C_{2-6}$ alkynyl being optionally substituted with one or more groups selected from the group consisting of OH, $NH_2$, CN, and halogen.

In some embodiments, $R^{3b}$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl.

In some embodiments, $R^{3b}$ are each independently selected from the group consisting of OH, $NH_2$, CN, halogen, $C_{2-6}$ alkenyl, and $C_{2-6}$ alkynyl.

In some embodiments, $R^{3b}$ are each independently selected from the group consisting of CN, halogen, $C_{2-6}$

alkenyl, and $C_{2-6}$ alkynyl.

In some embodiments, $R^{3b}$ are each independently selected from the group consisting of CN, halogen, $C_{2-4}$ alkenyl, and $C_{2-4}$ alkynyl.

In some embodiments, $R^{3b}$ are each independently selected from the group consisting of CN, halogen, $C_{2-3}$ alkenyl, and $C_{2-3}$ alkynyl.

In some embodiments, $R^{3b}$ are each independently selected from the group consisting of OH, $NH_2$, CN, halogen, $C_{1-6}$ alkyl, and $C_{1-6}$ alkoxy, the $C_{1-6}$ alkyl or $C_{1-6}$ alkoxy being optionally substituted with one or more groups selected from the group consisting of OH, $NH_2$, CN, and halogen.

In some embodiments, $R^{3b}$ are each independently selected from the group consisting of OH, $NH_2$, CN, halogen, $C_{1-6}$ alkyl, and $C_{1-6}$ alkoxy.

In some embodiments, $R^{3b}$ are each independently selected from the group consisting of OH, $NH_2$, CN, halogen, $C_{1-4}$ alkyl, and $C_{1-4}$ alkoxy.

In some embodiments, $R^{3b}$ are each independently selected from the group consisting of OH, $NH_2$, CN, halogen, and $C_{1-4}$ alkyl.

In some embodiments, $R^{3b}$ are each independently selected from the group consisting of OH, $NH_2$, CN, and halogen.

In some embodiments, $R^{3b}$ are each independently selected from the group consisting of CN and halogen.

In some embodiments, $R^{3b}$ are each independently selected from the group consisting of CN, fluorine, and chlorine.

It should be understood that any embodiment of the compounds of the present disclosure as described above and any specific substituents set forth herein with respect to particular R, R', $R^1$, $R^a$, $R^b$, $X^1$, $X^2$, $X^3$, $X^4$, $X^5$, $R^2$, m, ring A, $R^3$, $R^5$, $R^d$, n, $R^4$, X, cycB, $R^c$, the structural moiety

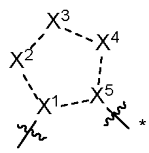

,

the structural moiety

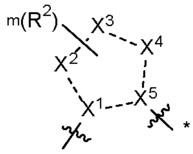

,

the structural moiety

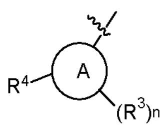

,

the structural moiety

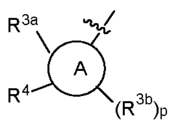

,

$R^{3a}$, and $R^{3b}$ of the compounds of the present disclosure as described above may be independently combined with other embodiments of the present disclosure and/or substituents of the compounds to form embodiments of the present disclosure not specifically set forth above. Furthermore, where substituent ranges are disclosed in the detailed embodiments and/or claims with respect to any particular R, R', $R^1$, $R^a$, $R^b$, $X^1$, $X^2$, $X^3$, $X^4$, $X^5$, $R^2$, m, ring A, $R^3$, $R^5$, $R^d$, n, $R^4$, X, cycB, $R^c$, the structural moiety

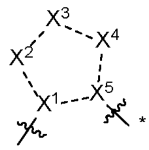

,

the structural moiety

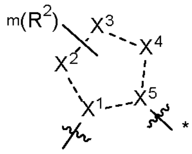

,

the structural moiety

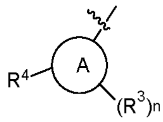

,

the structural moiety

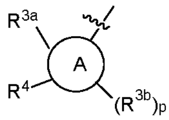

,

$R^{3a}$, and $R^{3b}$ substituents, it should be understood that one or more substituents may be deleted from the ranges, and the remaining substituent ranges should also be considered the embodiments of the present disclosure. It should be understood that when the substituent $R^2$ is present (i.e., when m is not 0), the substitution occurs at the position $X^2$, $X^3$, or $X^4$.

The present disclosure relates to a compound of the following formula, a stereoisomer thereof, or a pharmaceutically acceptable salt thereof:

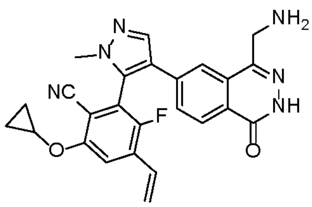 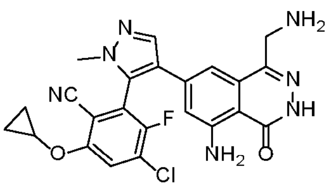 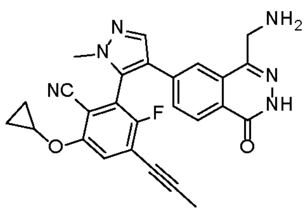 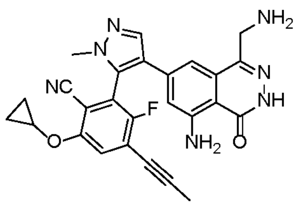

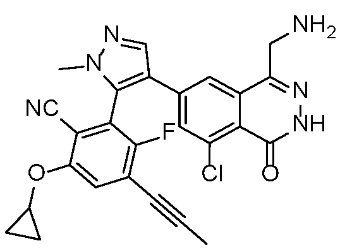 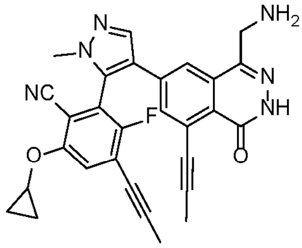 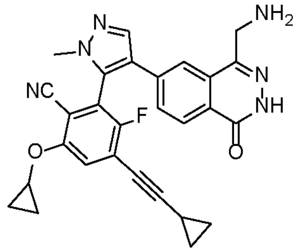 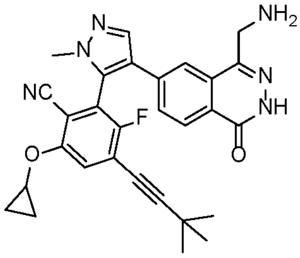

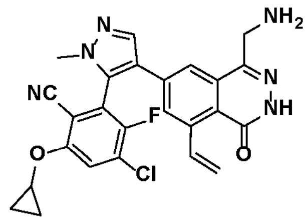
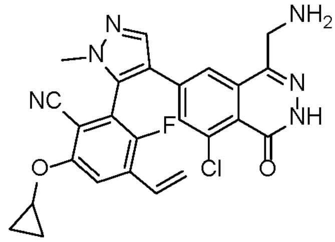
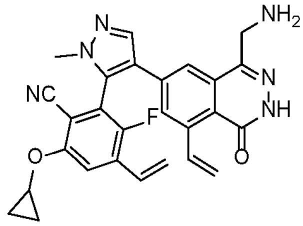

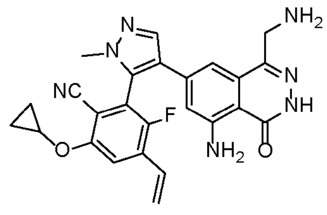
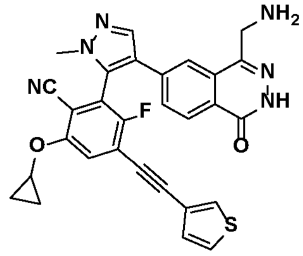
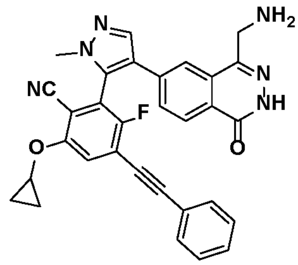

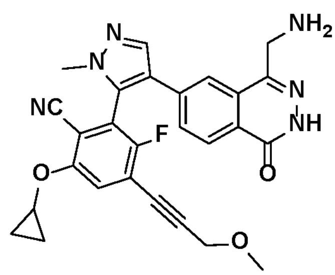
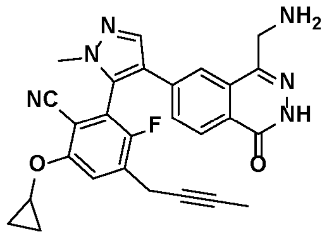
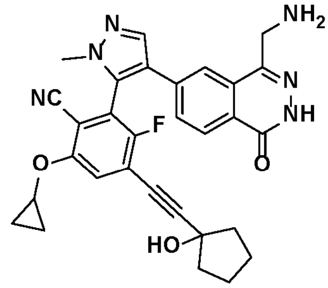

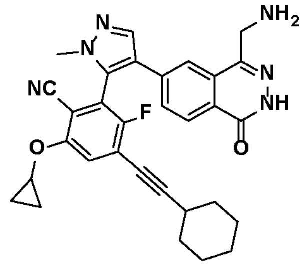
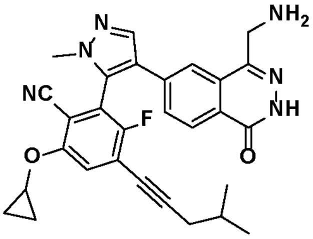
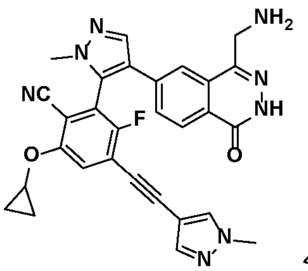
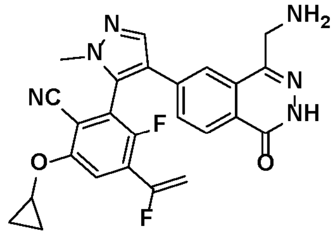

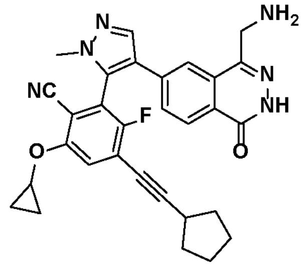
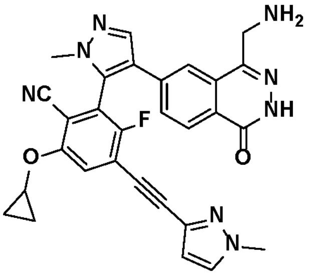
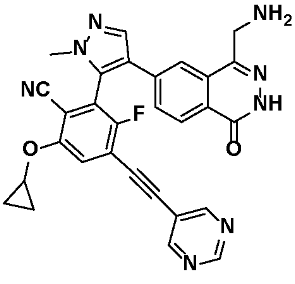
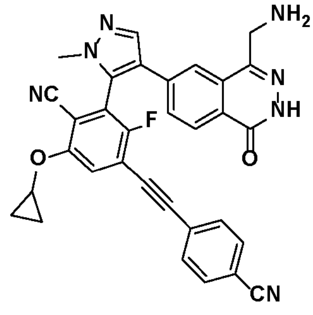

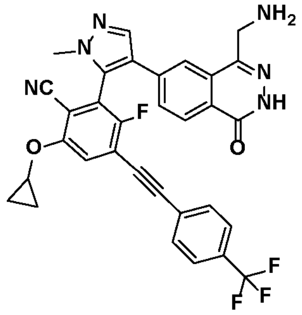
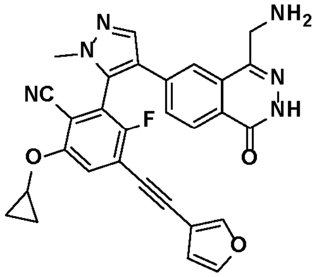
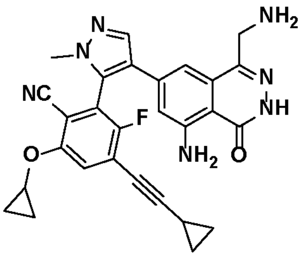
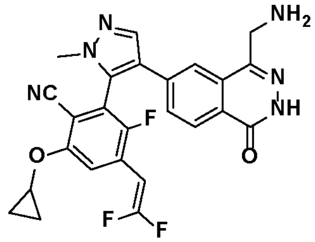

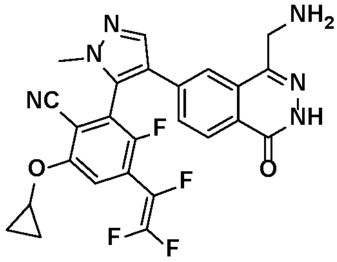

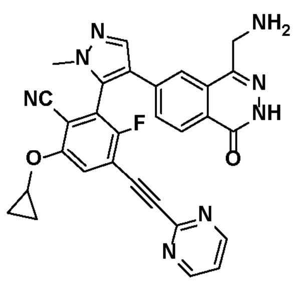
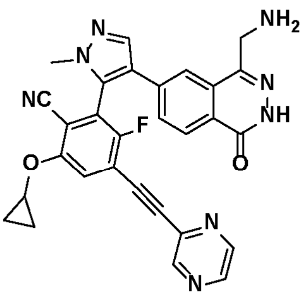
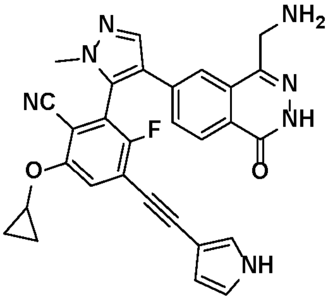
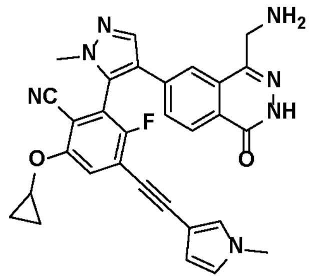

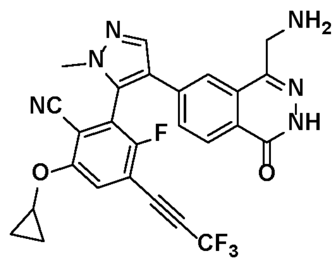
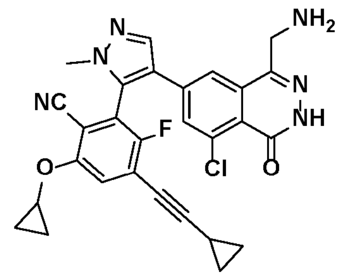
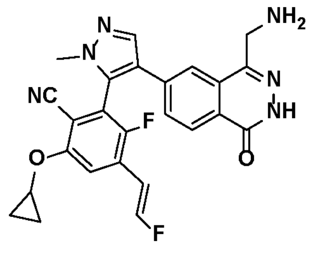

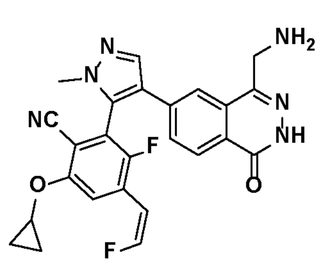
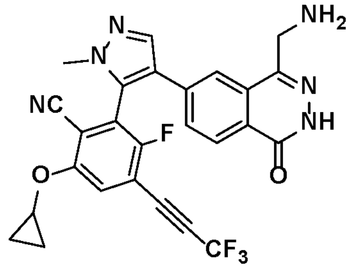

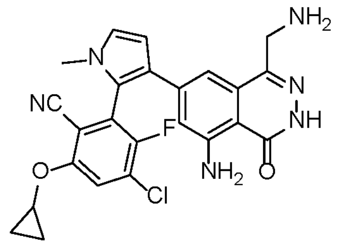 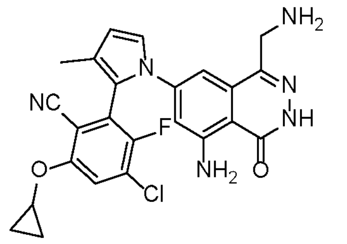 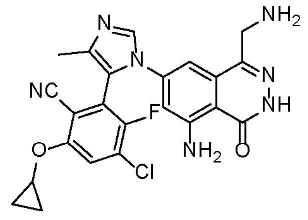

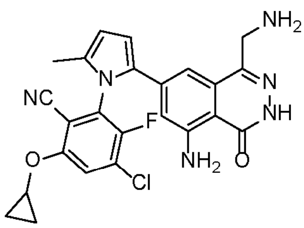 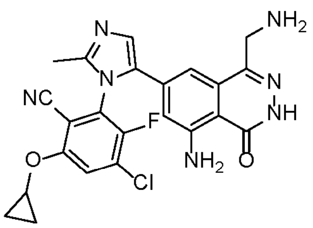 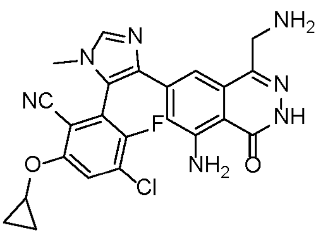 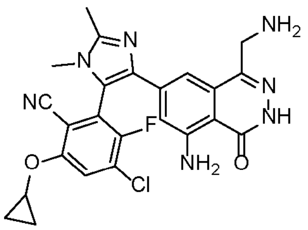

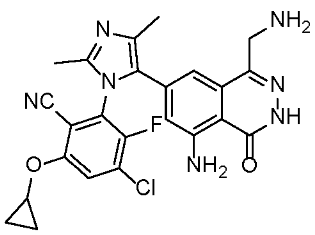 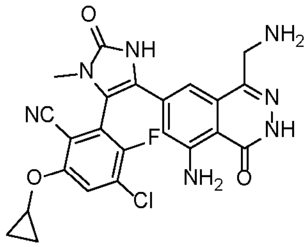 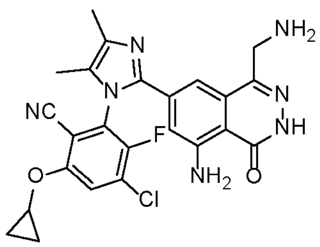 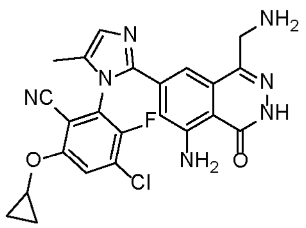

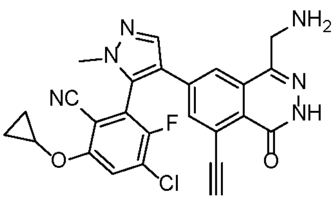 , 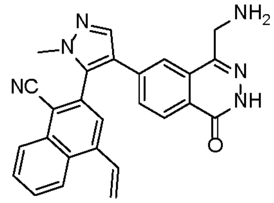 , or 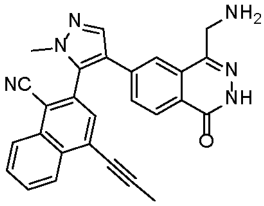 ;

or compounds of the following formulas or pharmaceutically acceptable salts thereof:

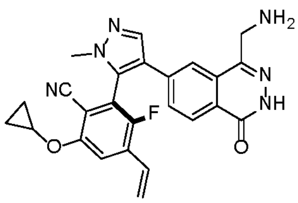 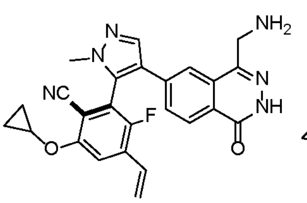 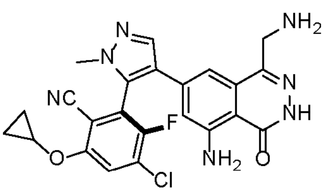 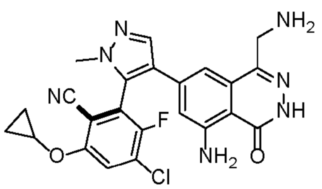

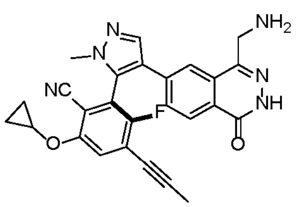 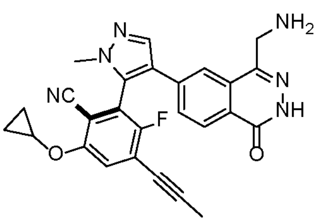 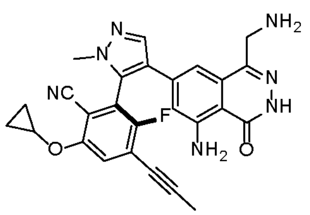 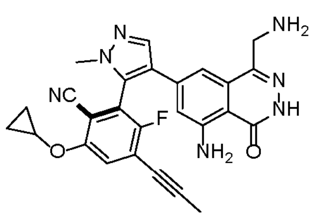

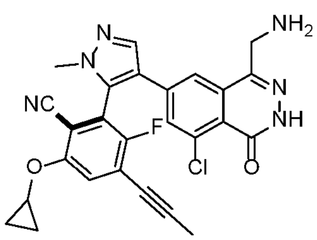
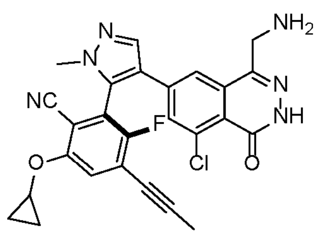
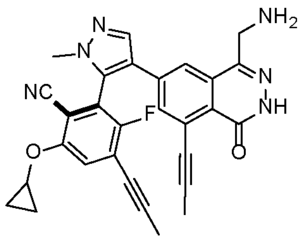
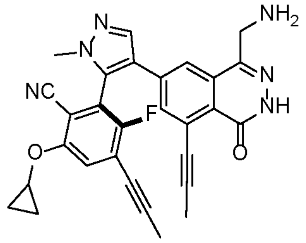
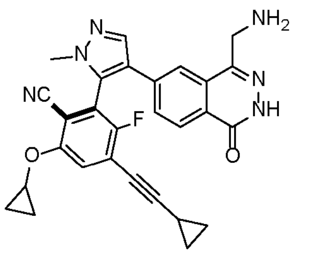
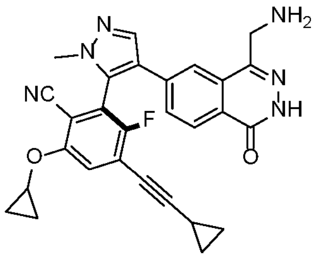
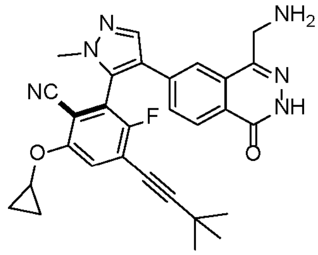
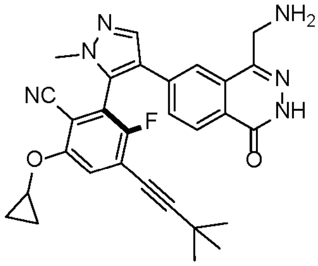
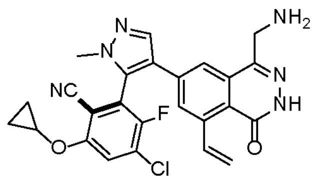
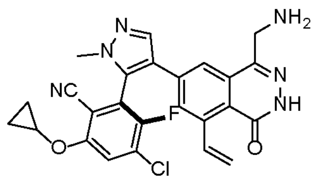
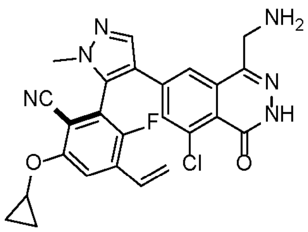
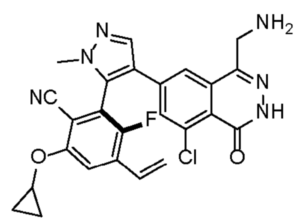
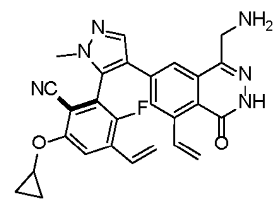
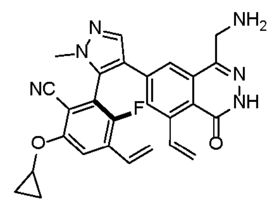
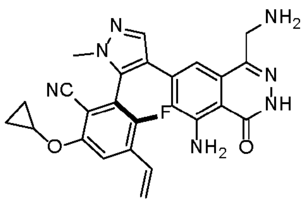
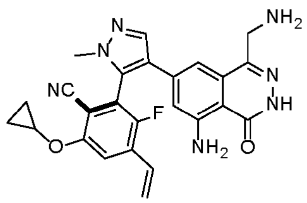
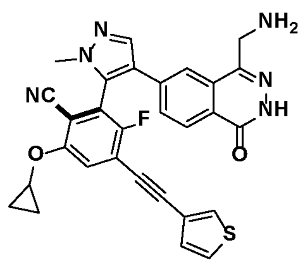
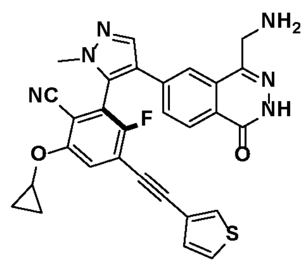
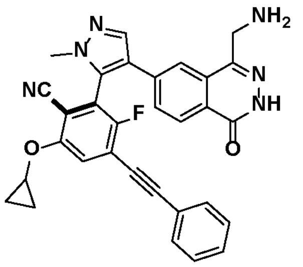
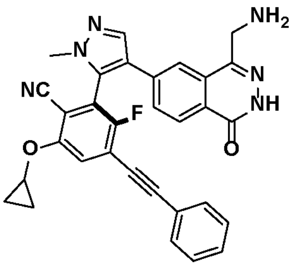
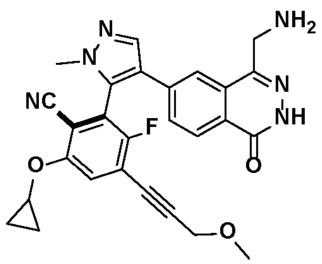
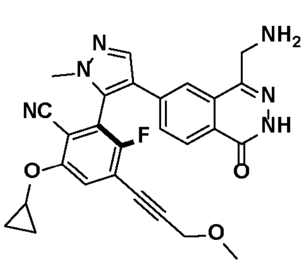

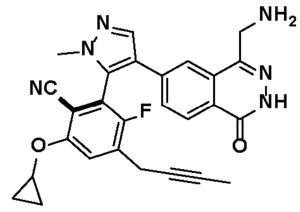
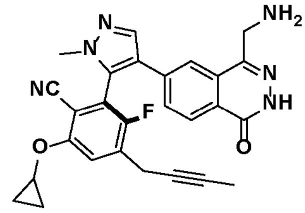
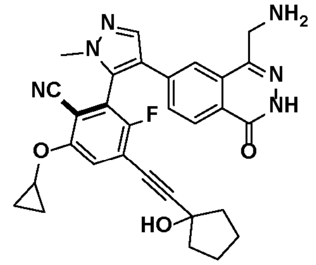
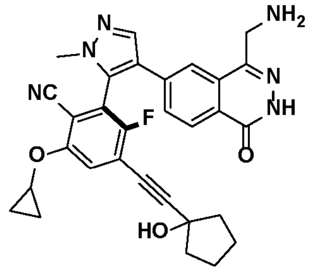

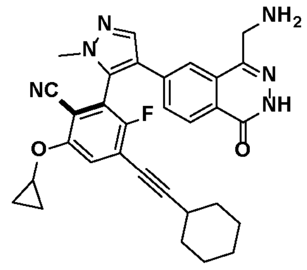
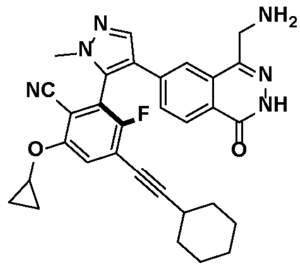
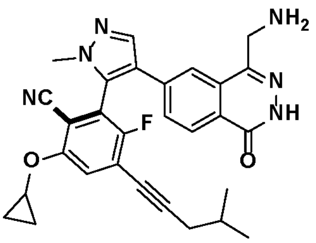
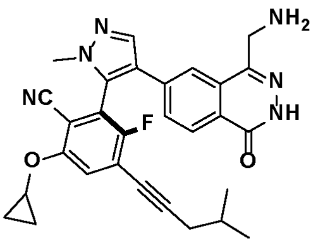

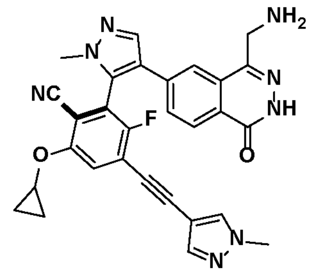
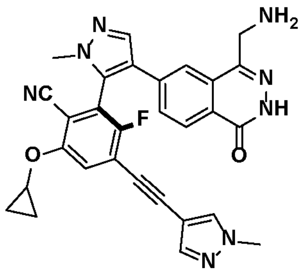
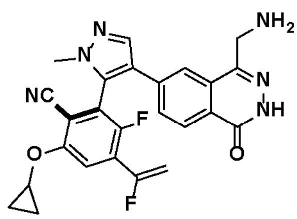
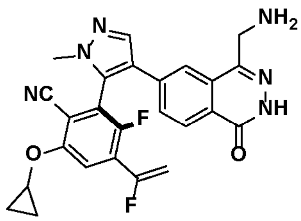

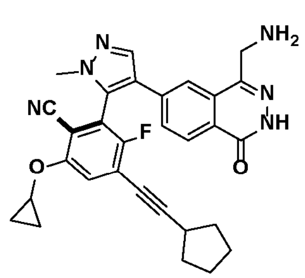
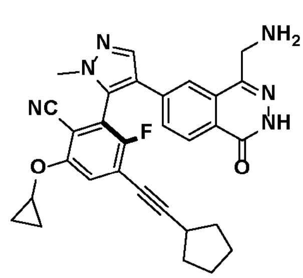
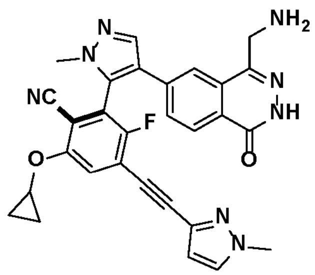
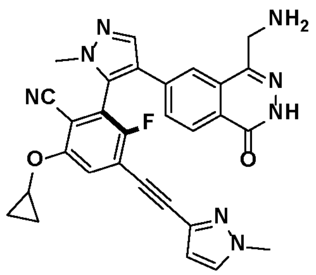

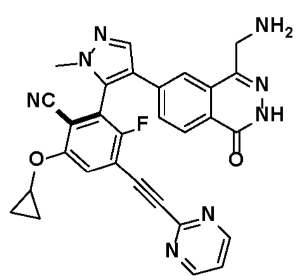
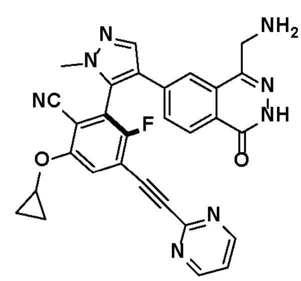
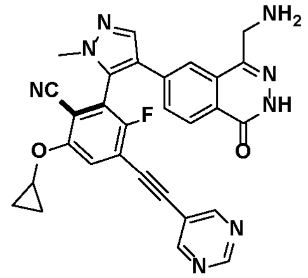
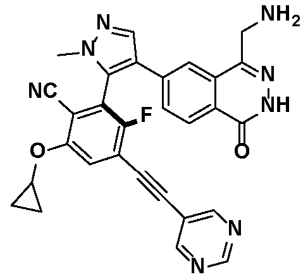

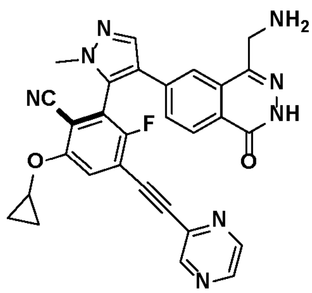

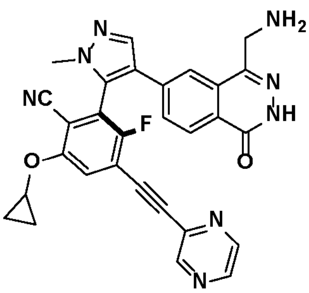

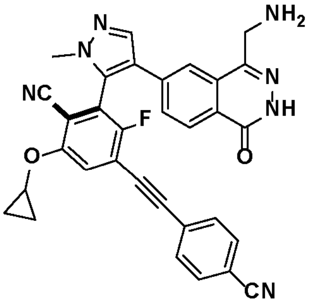

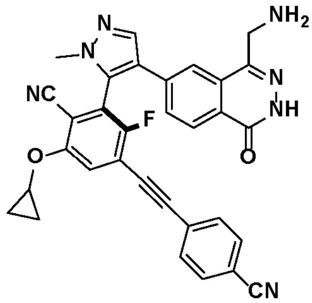

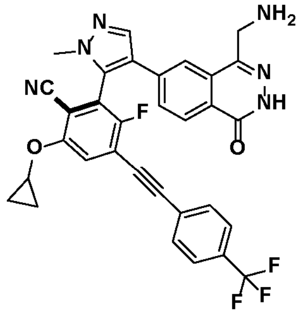

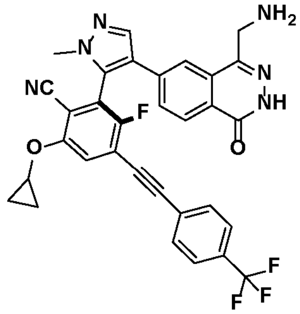

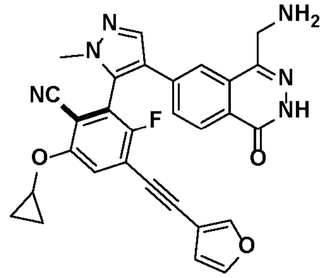

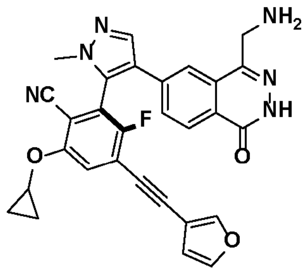

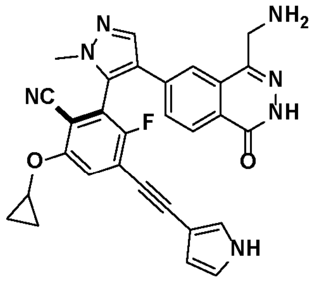

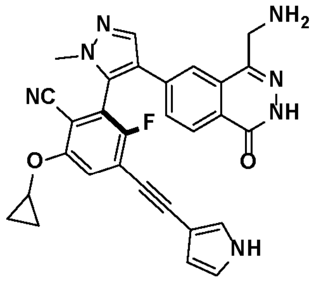

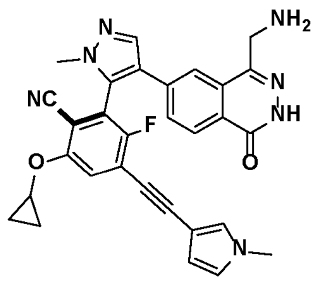

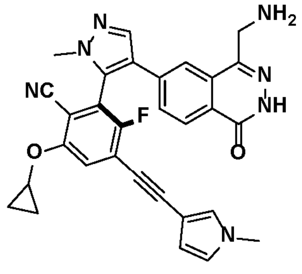

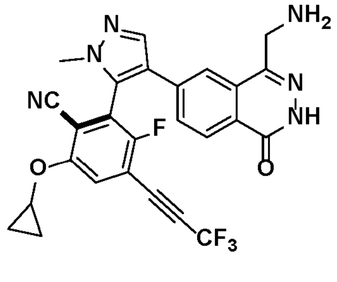

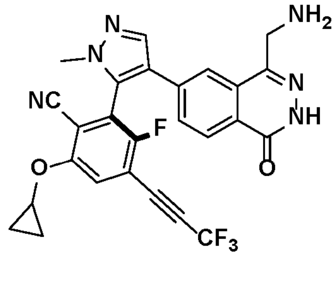

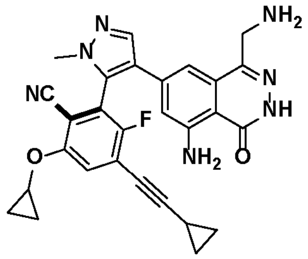

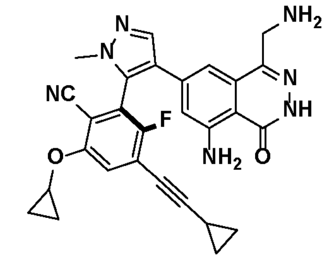

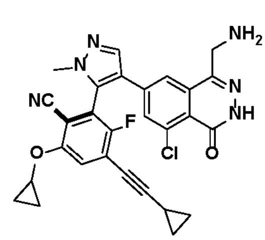

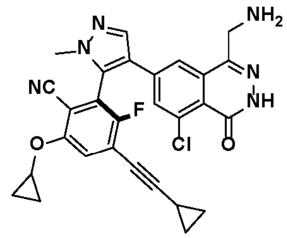

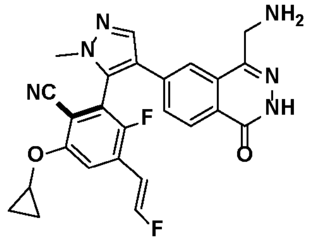
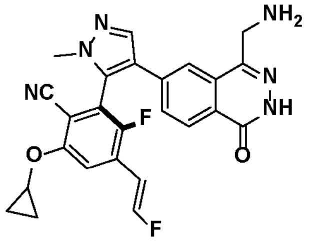
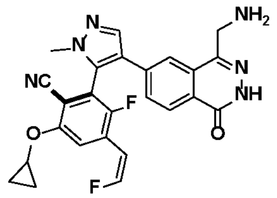
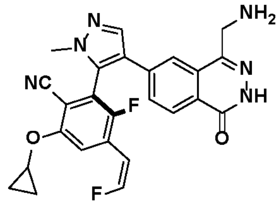

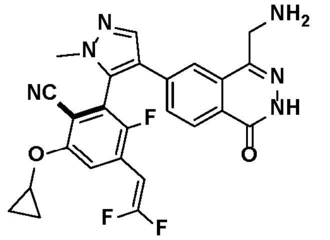
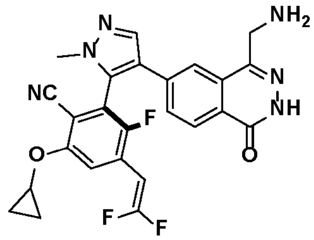
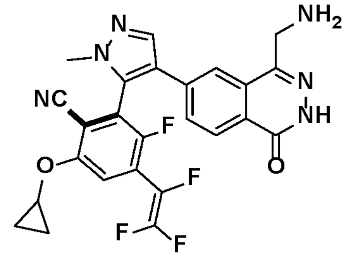

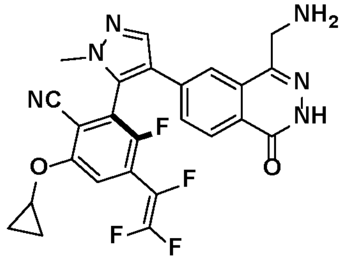
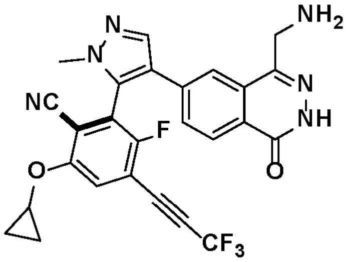
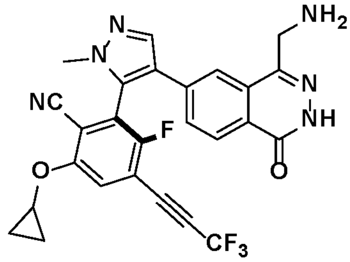

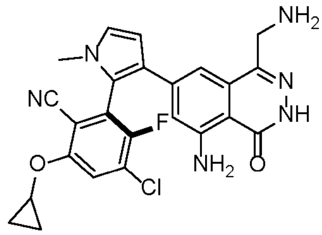
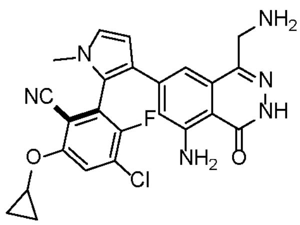
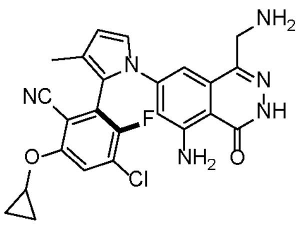
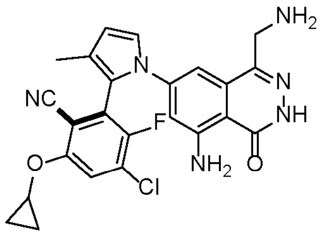

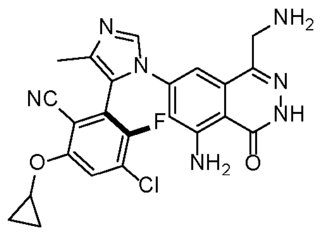
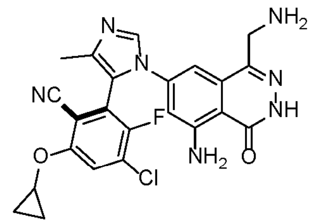
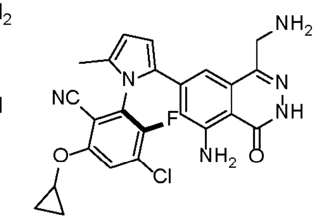
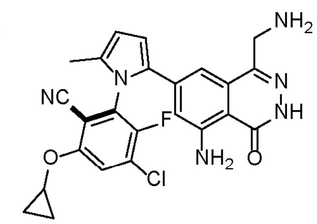

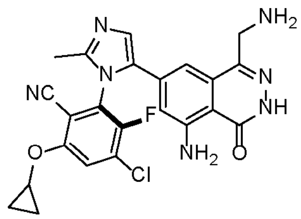
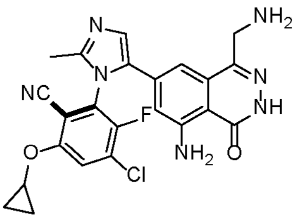
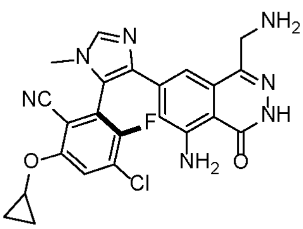
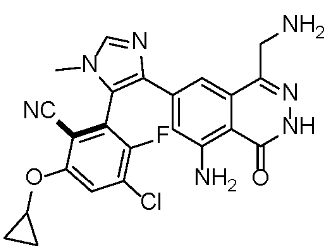

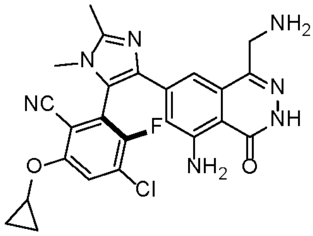
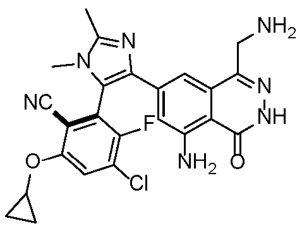
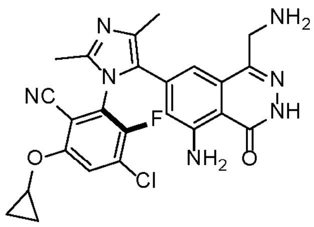
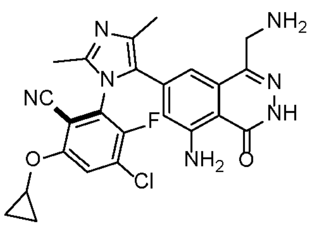
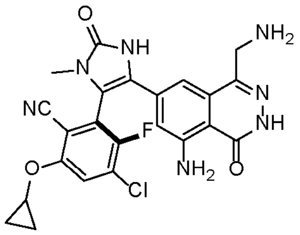
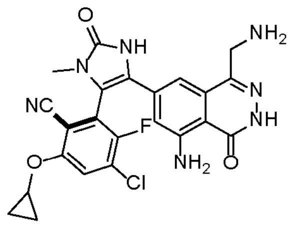
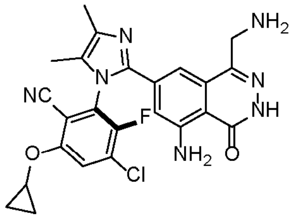
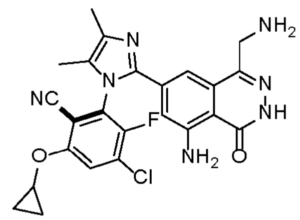
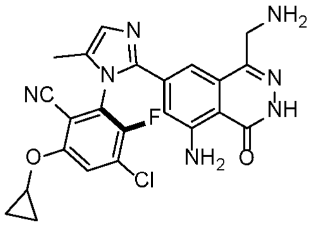
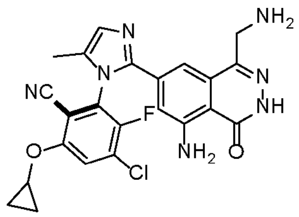
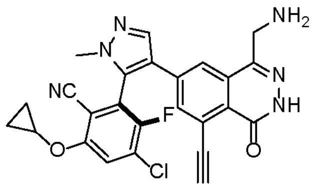
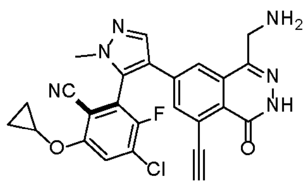
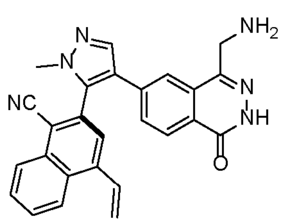
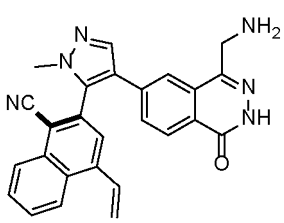
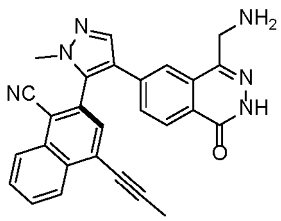
or
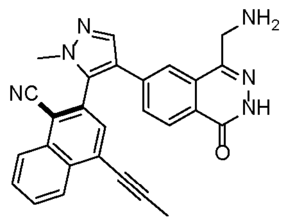
.

In another aspect, the present disclosure relates to a pharmaceutical composition comprising the compound, the stereoisomer thereof, or the pharmaceutically acceptable salt thereof of the present disclosure described above. The pharmaceutical composition of the present disclosure also comprises a pharmaceutically acceptable excipient. In another aspect, the present disclosure relates to use of the compound, or the stereoisomer thereof, or the pharmaceutically acceptable salt thereof, or the pharmaceutical composition thereof described above for preparing a medicament for preventing or treating a PRMT5-related disease.

The present disclosure relates to a method for treating or preventing a PRMT5-related disease in a mammal, comprising administering to a mammal, preferably a human, in need thereof a therapeutically effective amount of the compound, or the pharmaceutically acceptable salt thereof, or the stereoisomer thereof, or the pharmaceutical composition thereof of the present disclosure described above.

The present disclosure relates to a method for treating or preventing a PRMT5-related disease, comprising administering to a mammal, preferably a human, in need thereof a therapeutically effective amount of the compound, or the pharmaceutically acceptable salt thereof, or the stereoisomer thereof, or the pharmaceutical composition thereof of the present disclosure described above.

In another aspect, the present disclosure relates to use of the compound, the stereoisomer thereof, or the pharmaceutically acceptable salt thereof, or the pharmaceutical composition thereof described above for preventing or treating a PRMT5-related disease.

In another aspect, the present disclosure relates to the compound, or the stereoisomer thereof, or the pharmaceutically acceptable salt thereof, or the pharmaceutical composition thereof of the present disclosure described above for use in preventing or treating a PRMT5-related disease.

In some specific embodiments, the PRMT5-related disease described above is selected from the group consisting of disorders treated by degrading or inhibiting a PRMT5 protein; in some specific embodiments, the PRMT5-related disease is selected from the group consisting of cancers; in some more specific embodiments, the

PRMT5-related disease is selected from colon cancer.

In some embodiments, the present disclosure encompasses the variables defined above and embodiments thereof, as well as any combination thereof.

Technical effects

The compounds of the present disclosure have excellent *in vitro* protein binding activity, and have a binding effect on hPRMT5 & hMEP50 (plus MTA). The compounds of the present disclosure have an MTA-synergistic inhibitory effect on PRMT5, have proliferation inhibitory activity against HCT116 MTAP-/- cells, and selective inhibitory activity against HCT116 MTAP-/- cells compared with HCT116 WT, and have stable *in vitro* metabolism, and good *in vivo* pharmacokinetic properties and *in vivo* drug effect.

Definitions

Unless otherwise stated, the following terms used in the present disclosure shall have the following meanings. A certain term, unless otherwise specifically defined, should not be considered uncertain or unclear, but construed according to its common meaning in the art. When referring to a trade name, it is intended to refer to its corresponding commercial product or its active ingredient.

In some embodiments, the "one or more" is selected from the group consisting of one, two, three, four, five, and six. In some embodiments, the "one or more" is selected from the group consisting of one, two, and three. In some embodiments, the "one or more" is selected from the group consisting of one and two.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted, and oxo is not possible in an aromatic group.

The term "optional" or "optionally" means that the subsequently described event or circumstance may or may not occur. The description includes instances where the event or circumstance occurs and instances where it does not. For example, ethyl "optionally" substituted with halogen means that the ethyl may be unsubstituted ($CH_2CH_3$), monosubstituted (e.g., $CH_2CH_2F$), polysubstituted (e.g., $CHFCH_2F$ and $CH_2CHF_2$), or fully substituted ($CF_2CF_3$). It can be understood by those skilled in the art that for any groups comprising one or more substituents, no substitutions or substitution modes that are impossible to spatially exist and/or synthesize will be introduced. Non-limiting examples of the "substituent" described herein include, but are not limited to, -OH, -SH, halogen, - $NH_2$, nitro, nitroso, -CN, an azide group, a sulfoxide group, a sulfone group, a sulfonamide group, carboxy, a carboxaldehyde group, an imine group, alkyl, haloalkyl, cycloalkyl, halo-cycloalkyl, alkenyl, halo-alkenyl, cycloalkenyl, halo-cycloalkenyl, alkynyl, halo-alkynyl, cycloalkynyl, halo-cycloalkynyl, heteroalkyl, halo-heteroalkyl, alkoxy, alkylthio, aryl, aryloxy, arylthio, arylalkyl, arylalkoxy, arylalkylthio, heteroaryl, heteroaryloxy, heteroarylthio, heteroarylalkyl, heteroarylalkoxy, heteroarylalkylthio, heterocyclyl, heterocyclyloxy, heterocyclylthio, heterocyclylalkyl, heterocyclylalkoxy, heterocyclylalkylthio, acyl, acyloxy, a carbamate group, an amido group, ureido, an epoxy group, and an ester group, the groups being optionally substituted with one or more substituents selected from the group consisting of the following substituents: oxo, hydroxy, amino, nitro, halogen, cyano, alkyl, alkenyl, alkynyl, alkoxy, haloalkoxy, alkylamino, dialkylamino, haloalkylamino, halodialkylamino, carboxy, -C(O)O-alkyl, -OC(O)-alkyl, -C(O)$NH_2$, -C(O)NH-alkyl, - C(O)N$(alkyl)_2$, -NHC(O)-alkyl, -C(O)-alkyl, -S(O)-alkyl, -S$(O)_2$-alkyl, -S$(O)_2$$NH_2$, -S$(O)_2$NH-alkyl, - S$(O)_2$N$(alkyl)_2$, cycloalkyl, cycloalkylalkyl, cycloalkyloxy, heterocyclyl, heterocyclylalkyl, heterocyclyloxy, heterocycloalkyl, heterocycloalkylalkyl, heterocycloalkyloxy, heteroaryl, heteroarylalkyl, heteroaryloxy, aryl, arylalkyl, and aryloxy.

$C_{m-n}$ used herein means that the moiety has an integer number of carbon atoms in the given range. For example, "$C_{1-6}$" means that the group may have 1 carbon atom, 2 carbon atoms, 3 carbon atoms, 4 carbon atoms, 5 carbon atoms, or 6 carbon atoms.

When any variable (e.g., R) occurs once or more times in the constitution or structure of a compound, the definition of the variable in each case is independent. For example, if a group comprises 2 R, the definition of each R is independent.

When a bond is crosslinked to two atoms of a ring (including monocyclic, fused, or spiro ring), the bond may be bonded to any atom on the ring (including monocyclic, fused, or spiro ring).

The term "halo-" or "halogen" refers to fluorine, chlorine, bromine, and iodine.

The term "hydroxy" refers to an -OH group.

The term "amino" refers to an -$NH_2$ group.

The term "alkyl" refers to hydrocarbyl of general formula $C_nH_{2n+1}$, typically containing 1-12, 1-10, 1-8, 1-6, 1-4, or 1-3 carbon atoms. The alkyl may be linear or branched. For example, the term "$C_{1-6}$ alkyl" refers to alkyl containing 1-6 carbon atoms (for example, methyl, ethyl, n-propyl, isopropyl, n-butyl, isobutyl, sec-butyl, *tert*-butyl, n-pentyl, 1-methylbutyl, 2-methylbutyl, 3-methylbutyl, neopentyl, hexyl, 2-methylpentyl, and the like). Similarly, the alkyl moiety (i.e., alkyl) of

alkoxy, alkylamino, dialkylamino, alkylsulfonyl, and alkylthio has the same definition as those described above.

The term "alkoxy" refers to -O- alkyl.

The term "alkenyl" refers to linear or branched unsaturated aliphatic hydrocarbyl consisting of carbon atoms and hydrogen atoms and having at least one double bond, typically containing 2-12, 2-10, 2-8, 2-6, 2-4, or 2-3 carbon atoms. Non-limiting examples of the alkenyl include, but are not limited to, ethenyl, 1-propenyl, 2-propenyl, 1-butenyl, isobutenyl, 1,3-butadienyl, and the like.

The term "alkynyl" refers to linear or branched unsaturated aliphatic hydrocarbyl consisting of carbon atoms and hydrogen atoms and having at least one triple bond, typically containing 2-12, 2-10, 2-8, 2-6, 2-4, or 2-3 carbon atoms. Non-limiting examples of the alkynyl include, but are not limited to, ethynyl ($-C\equiv CH$), 1-propynyl ($-C\equiv C-CH_3$), 2-propynyl ($-CH_2-C\equiv CH$), 2-butynyl ($-CH_2C\equiv C-CH_3$), 3,3-dimethylbutynyl, 1,3-butadiynyl ($-C\equiv C-C\equiv CH$), 4-methyl-1-pentynyl, and the like.

The term "cycloalkenyl" refers to a non-aromatic carbocyclic ring that is not fully saturated and may exist as a monocyclic ring, a bicyclic bridged ring, or a spiro ring. Unless otherwise indicated, the carbocyclic ring is typically a 3- to 10-membered ring (e.g., a 4- to 10-membered ring, a 4- to 8-membered ring, a 5- to 7-membered ring, or a 5- to 6-membered ring). Non-limiting examples of the cycloalkenyl include, but are not limited to, cyclopentenyl, cyclopentadienyl, cyclohexenyl, cyclohexadienyl, cycloheptenyl, cycloheptadienyl, and the like. The term "cycloalkyl" refers to a carbocyclic ring that is fully saturated and may exist as a monocyclic ring, a bridged ring, or a spiro ring. Unless otherwise indicated, the carbocyclic ring is typically a 3- to 10-membered ring (e.g., a 5- to 8-membered ring, a 3- to 8-membered ring, or a 3- to 6-membered ring). Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like.

The term "heterocycloalkyl" refers to a cyclic group that is fully saturated and may exist as a monocyclic ring, a bridged ring, or a spiro ring. Unless otherwise indicated, the heterocyclic ring is typically a 3- to 15-membered ring, a 3- to 12-membered ring, a 3- to 10-membered ring, a 3- to 8-membered ring, a 3- to 7-membered ring, a 3- to 6-membered ring, a 5- to 6-membered ring, or a 3- to 5-membered ring containing 1-3 heteroatoms independently selected from the group consisting of boron, sulfur, oxygen, and/or nitrogen (preferably 1, 2, or 3 heteroatoms, preferably heteroatoms selected from the group consisting of nitrogen, oxygen, and sulfur). Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. Monocyclic heterocycloalkyl having 5 or 6 ring atoms is preferred. The term "heterocycloalkenyl" includes cycloalkenyl in which up to 3 carbon atoms, or up to 2 carbon atoms, or 1 carbon atom, are each independently replaced by boron, oxygen, S(O), or nitrogen, provided that at least one cycloalkenyl carbon-carbon double bond is retained. The heterocycloalkenyl refers to a cyclic group that may exist as a monocyclic ring, a bridged ring, or a spiro ring, and may be a 3- to 13-membered ring (e.g., a 5- to 13-membered ring, a 5- to 8-membered ring, or a 3- to 10-membered ring).

The term "heterocyclyl" refers to a non-aromatic ring that is fully saturated or partially unsaturated (but not a fully unsaturated heteroaromatic group) and may exist as a monocyclic ring, a bridged ring, or a spiro ring. Unless otherwise indicated, the heterocyclic ring is typically a 3- to 20-membered (or 3- to 17-membered, or 3- to 13-membered, or 3- to 7-membered, or 5- to 15-membered, or 5- to 10-membered) ring containing 1-3 heteroatoms independently selected from the group consisting of boron, sulfur, oxygen, and/or nitrogen (preferably 1, 2, or 3 heteroatoms, preferably heteroatoms selected from the group consisting of nitrogen, oxygen, and sulfur). Non-limiting examples of the heterocyclyl include, but are not limited to, oxiranyl, tetrahydrofuranyl, dihydrofuranyl, pyrrolidinyl, N-methylpyrrolidinyl, dihydropyrrolyl, piperidinyl, piperazinyl, pyrazolidinyl, 4H-pyranyl, morpholinyl, sulfomorpholinyl, tetrahydrothienyl, and the like. The heterocyclyl may be, for example, heterocycloalkenyl, heterocycloalkyl, or benzoheterocycloalkenyl (not a fully saturated heteroaromatic group). The term "aryl" refers to an all-carbon aromatic monocyclic or fused polycyclic group having a conjugated $\pi$-electron system. For example, the aryl may have 6-20 carbon atoms, 6-14 carbon atoms, 6-12 carbon atoms, or 6-10 carbon atoms. Non-limiting examples of the aryl include phenyl, naphthyl, anthryl, 1,2,3,4-tetrahydronaphthalene, and the like.

The term "heteroaryl" refers to a monocyclic or fused polycyclic system that contains at least one ring atom selected from the group consisting of nitrogen, oxygen, and S, with the remaining ring atoms being C, and that has at least one aromatic ring. Preferably, the heteroaryl has a single 4- to 8-membered ring, in particular a 5- to 8-membered ring or a 5- to 6-membered ring, or has a plurality of fused rings containing 6-14 ring atoms, in particular 6-10 or 5-10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The term "treat", "treating", or "treatment" refers to administering the compound or formulation described in the

present disclosure to ameliorate or eliminate a disease or one or more symptoms related to the disease, and includes:

(i) inhibiting a disease or disease state, i.e., arresting its progression; and
(ii) alleviating a disease or disease state, i.e., causing its regression.

The term "prevent", "preventing", or "prevention" means administering the compound or formulation described in the present disclosure to prevent a disease or one or more symptoms related to the disease, and includes preventing the occurrence of the disease or disease state in a mammal, particularly when such a mammal is predisposed to the disease state but has not yet been diagnosed with it.

The term "therapeutically effective amount" refers to an amount of the compound of the present disclosure for (i) treating or preventing a specific disease, condition, or disorder; (ii) relieving, ameliorating, or eliminating one or more symptoms of a specific disease, condition, or disorder, or (iii) preventing or delaying onset of one or more symptoms of the specific disease, condition, or disorder described herein. The amount of the compound of the present disclosure composing the "therapeutically effective amount" varies dependently on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/hazard ratio.

A pharmaceutically acceptable salt, for example, may be a metal salt, an ammonium salt, a salt formed with an organic base, a salt formed with an inorganic acid, a salt formed with an organic acid, and a salt formed with a basic or acidic amino acid.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism. The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, and water.

The word "comprise" and variations thereof such as "comprises" or "comprising" will be understood in an open, non-exclusive sense, i.e., "including but not limited to".

The compounds and intermediates of the present disclosure may also exist in different tautomeric forms, and all such forms are included within the scope of the present disclosure. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerization and imine-enamine isomerization. A specific example of a proton tautomer is an imidazole moiety where a proton can transfer between two ring nitrogen atoms. A valence tautomer includes the interconversion via recombination of some bonding electrons.

The present disclosure also includes isotopically labeled compounds of the present disclosure, which are identical to those recited herein but have one or more atoms replaced with an atom having an atomic mass or mass number different from the atomic mass or mass number generally found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Certain isotopically labeled compounds of the present disclosure (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$, and $^{18}F$, can be used in positron emission tomography (PET) studies to determine substrate occupancy. The isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., $^{2}H$) may provide certain therapeutic advantages (e.g., increased *in vivo* half-life or reduced dosage) resulting from greater metabolic stability and hence may be preferred in some circumstances in which deuterium substitution may be partial or complete, wherein partial deuterium substitution refers to substitution of at least one hydrogen with at least one deuterium.

The compounds of the present disclosure can be asymmetrical, for example, has one or more stereoisomers. Unless otherwise stated, all stereoisomers are included, for example, enantiomers and diastereoisomers. Certain compounds of the present application may exist as atropisomers, which are conformational isomers. The atropisomers

occur when rotation around a single bond in a molecule is prevented or greatly slowed due to steric interactions with other parts of the molecule. The compounds of the present disclosure may include all atropisomers, which may be pure individual atropisomers, or enriched in one of the atropisomers, or a nonspecific mixture of the atropisomers. If the rotational potential around a single bond is high enough and the interconversion between conformations is slow enough, separation of isomers may be allowed. The bond I indicates that the steric orientation of this side is outward. For example,

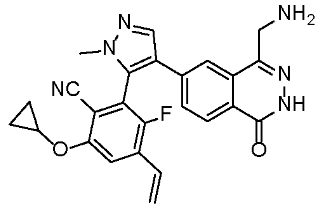

exists as atropisomer 1

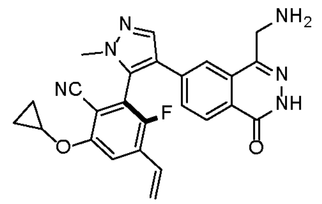

and atropisomer 2

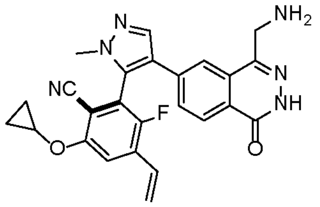
.
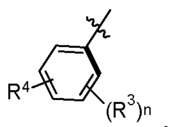
,
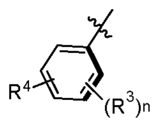
,

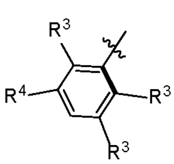
,
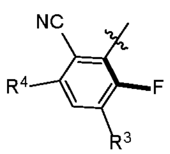
,
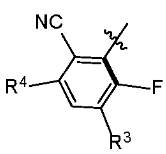
,
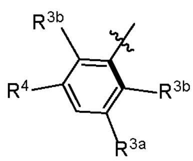
,
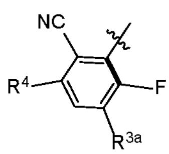
,

and the like shown in the present disclosure all represent the same configuration, in particular the same configuration as the isomer 1 compound described above.

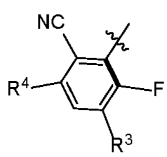

is connected to the adjacent structural moiety

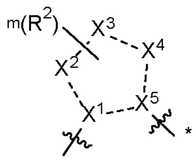

to form, for example, the following structure

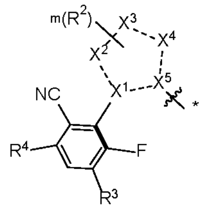

, or

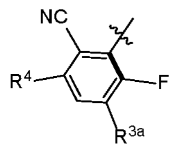

is connected to the adjacent structural moiety

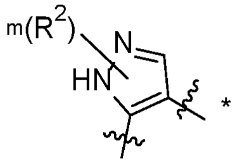

to form, for example, the following structure

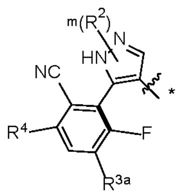

.

The connections of the other structural moieties are similar to the above.

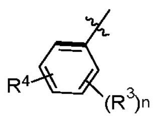

,

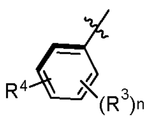

,

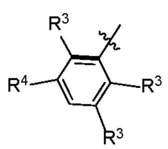

,

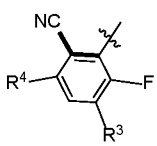

,

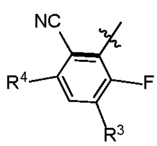

,

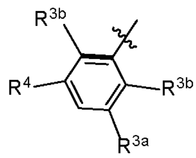

,

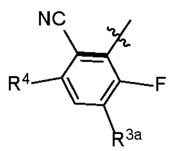

,

and the like shown in the present disclosure all represent the same configuration, i.e., the same configuration as the isomer 2 compound described above.

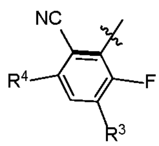

is connected to the adjacent structural moiety

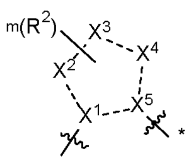

to form, for example, the following structure

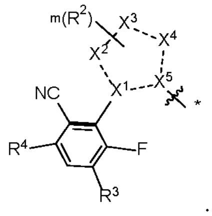

The connections between the other structural moieties and the adjacent structural moieties are similar to the above. The compounds containing asymmetric carbon atoms of the present disclosure can be isolated in an optically active pure form or in a racemic form. The optically active pure form may be separated from a racemic mixture or may be synthesized using a chiral raw material or a chiral reagent.

The pharmaceutical composition of the present disclosure can be prepared by combining the compound of the present disclosure with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present disclosure include, but are not limited to, oral, rectal, topical, inhalational, parenteral, sublingual, intravaginal, intranasal, intraocular, intraperitoneal, intramuscular, subcutaneous, and intravenous administration.

The pharmaceutical composition of the present disclosure can be manufactured using methods well known in the art, such as conventional mixing, dissolving, granulating, dragee-making, levigating, emulsifying, and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compound of the present disclosure to be formulated into tablets, pills, pastilles, dragees, capsules, liquids, gels, slurries, suspensions, etc., for oral administration to a patient.

A solid oral composition can be prepared by conventional mixing, filling, or tableting. For example, the solid oral composition can be obtained by the following method: mixing the active compounds with solid excipients, optionally grinding the resulting mixture, adding additional suitable excipients if desired, and processing the mixture into granules to obtain the core parts of tablets or dragees. Suitable excipients include, but are not limited to: binders, diluents, disintegrants, lubricants, glidants, sweeteners, flavoring agents, and the like.

The pharmaceutical composition may also be suitable for parenteral administration, such as a sterile solution, a suspension, or a lyophilized product in a suitable unit dosage form.

In all of the administration methods of the compound of general formula I described herein, the daily dose administered is from 0.01 mg/kg of body weight to 200 mg/kg of body weight, given in individual or separated doses.

The compounds of the present disclosure can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present disclosure.

The chemical reactions of the embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., amino in the present disclosure). For example, reference may be made to Greene's Protective Groups in Organic Synthesis (4th Ed.) Hoboken, New Jersey: John Wiley & Sons, Inc.

The compounds of the present disclosure may be prepared using the following routes, in conjunction with techniques known in the art:

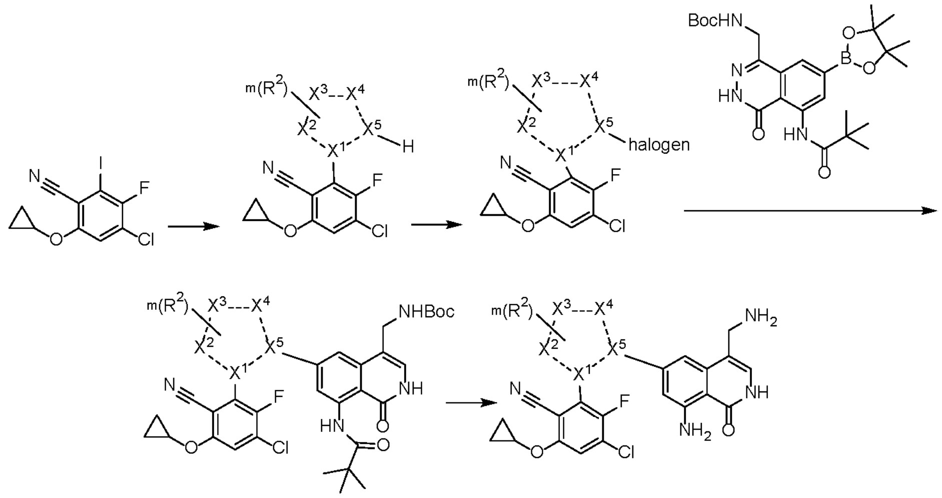

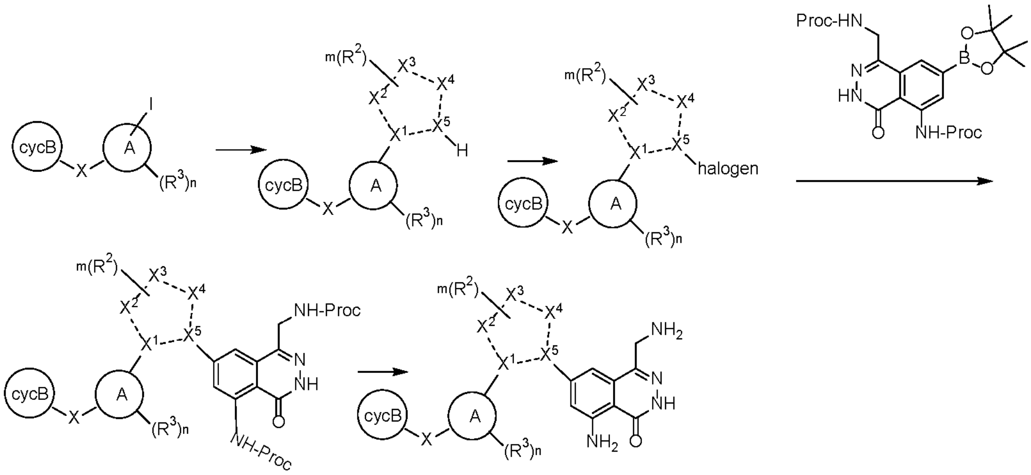

wherein halogen represents a halogen element; proc represents a protecting group such as Boc or Piv; $X^1$, $X^2$, $X^3$, $X^4$, $X^5$, ring A, $R^3$, n, $R^2$, m, X, and cycB are as defined in the present disclosure.

The following abbreviations are used in the present disclosure:
NIS represents *N*-iodosuccinimide; EA represents ethyl acetate; LDA represents lithium diisopropylamide; BuOK represents potassium *tert*-butoxide; THF represents tetrahydrofuran; Pd(dtbpf)$Cl_2$ represents 1,1'-bis(di-*tert*-butylphosphino)ferrocene dichloropalladium; DMF represents *N,N*-dimethylformamide; Boc represents *tert*-butyloxycarbonyl; Bu represents *tert*-butyl; Piv represents pivaloyl; HATU represents O-(7-azabenzotriazol-1-yl)-*N*,*N*,*N*',*N*'-tetramethyluronium hexafluorophosphate; DIPEA represents diisopropylethylamine; cataCXium A Pd G3 represents mesylate[(di(1-adamantyl)-n-butylphosphine)-2-(2'-amino-1,1'-biphenyl)]palladium(II); Pd(dppf)$Cl_2$ represents [1,1'-bis(diphenylphosphino)ferrocene]dichloropalladium (II); MTA represents 2-methylthioadenosine; DMSO represents dimethyl sulfoxide.

## DETAILED DESCRIPTION

For clarity, the present disclosure is further described with the following examples, which are, however, not intended to limit the scope of the present disclosure. All the reagents used in the present disclosure are commercially available and can be used without further purification.

**Example 1:**

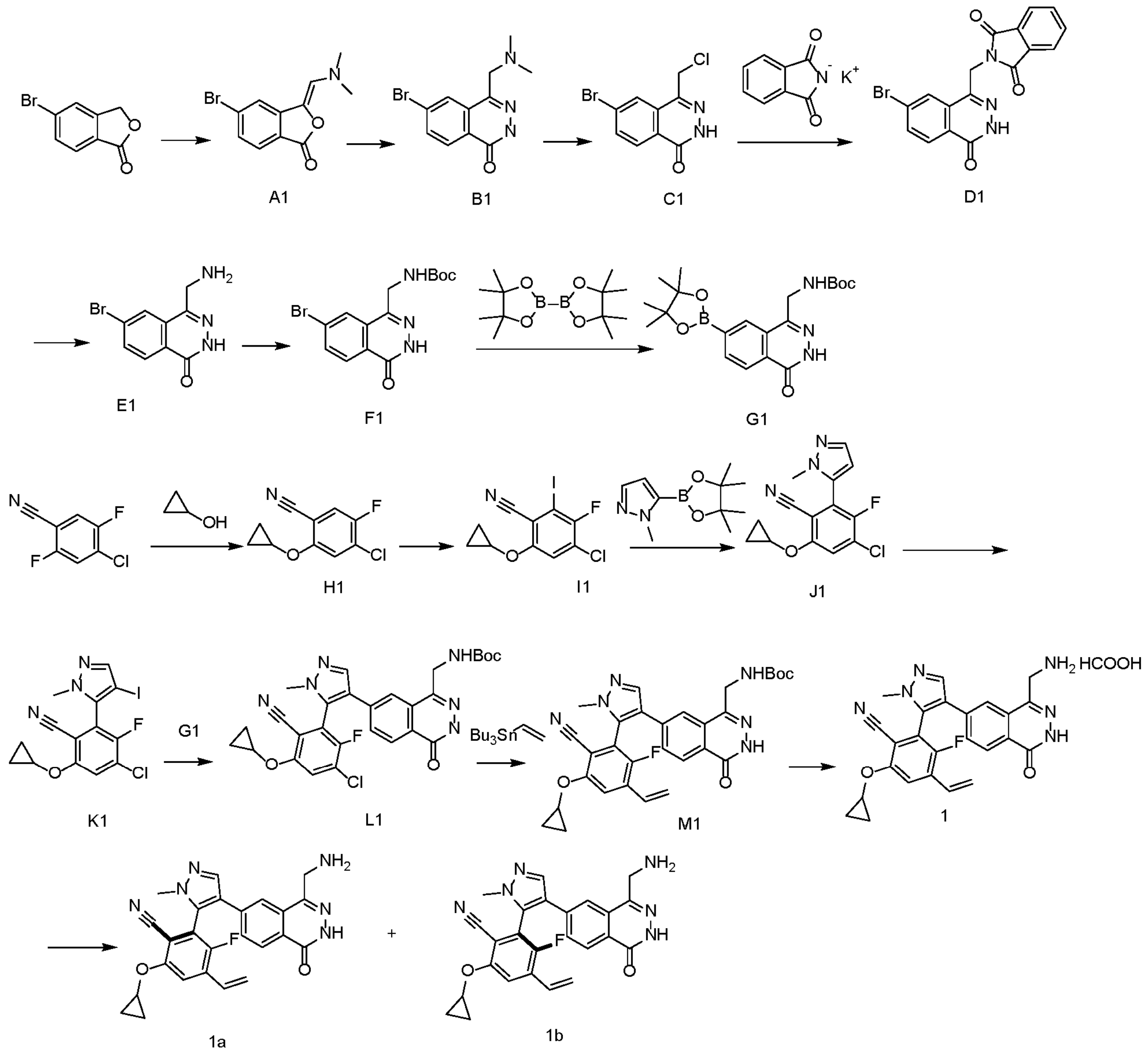


A1 B1 C1 D1 E1 F1 G1 H1 I1 J1 K1 L1 M1 1 1a + 1b

**(1) Preparation method for compound A1:**

5-Bromophenhthalide (80.0 g) and potassium *tert*-butoxide (4.2 g) were dissolved in *N,N*-dimethylformamide dimethyl acetal (2880. g). The reaction solution was stirred at 110 °C for about 20 h under nitrogen atmosphere. After the reaction was completed, the reaction solution was cooled to room temperature, and distilled under reduced pressure until no liquid flowed out. The resulting crude product was first slurried with petroleum ether at 25 °C, then slurried with ethyl acetate at 80 °C, and filtered under vacuum. The filter cake was concentrated under reduced pressure to dryness to give compound **A1** (60.0 g in total), ESI-MS: m/z = 267.9 $[M+H]^+$.

**(2) Preparation method for compound B1:**

Compound **Al(60.0** g) and hydrazine hydrate (18.6 mL) were dissolved in 1.0 L of ethanol. The reaction solution was stirred at 70 °C for about 12 h under nitrogen atmosphere. After the reaction was completed, the reaction solution was filtered under vacuum. The resulting filter cake was the target product **B1** (55.0 g in total), which was directly used in the next step without further purification. ESI-MS: m/z = 282.0 $[M+H]^+$.

**(3) Preparation method for compound C1:**

Compound **B1** (55.0 g) was dissolved in 680 mL of tetrahydrofuran under nitrogen atmosphere. Isobutyl chloroformate (32.0 g) was slowly added at 0 °C. After the dropwise addition, the reaction solution was stirred in a

reaction flask at room temperature for 6 h, and then the reaction was stopped. The reaction flask was placed in an ice-water bath, and 920 mL of hydrogen chloride (0.5 M) solution was added to quench the reaction. After the addition, the reaction solution was filtered under vacuum. The filter cake was rinsed with tetrahydrofuran and dried under reduced pressure to give compound **C1** (45.0 g in total), which was directly used in the next step without further purification. ESI-MS: m/z = 272.9 $[M+H]^+$.

**(4) Preparation method for compound D1:**

Compound **C1** (45.0 g) and potassium phthalimide (31.3 g) were dissolved in 890 mL of DMF. The reaction solution was stirred at room temperature for about 1.5 h. After the reaction was completed, 550 mL of hydrogen chloride (0.5 M) solution was added to the reaction system to quench the reaction. The reaction solution was filtered under vacuum. The filter cake was rinsed with a saturated sodium bicarbonate solution and pure water in sequence. The resulting crude product was slurried in ethanol at 70 °C and filtered under vacuum. The resulting filter cake was the target product **D1** (48.0 g in total), which was directly used in the next step without further purification. ESI-MS: m/z = 383.9 $[M+H]^+$.

**(5) Preparation method for compound E1:**

Compound **D1** (48.0 g) and hydrazine hydrate (25.6 mL) were dissolved in 100 mL of ethanol. The reaction solution was stirred at 80 °C for 2 h. After the reaction was completed, the reaction system was concentrated to dryness. The resulting crude product was dissolved in ethyl acetate and filtered under vacuum. The resulting filter cake was the target product **E1** (30.0 g in total), which was directly used in the next step without further purification. ESI-MS: m/z =253.9 $[M+H]^+$.

**(6) Preparation method for compound F1:**

Compound **E1** (30.0 g), di-*tert*-butyl dicarbonate (51.5 g), and triethylamine (35.8 g) were dissolved in 630 mL of dichloromethane. The reaction solution was stirred at room temperature for 2 h. After the reaction was completed, the reaction solution was filtered under vacuum. The resulting filter cake was slurried in dichloromethane and filtered under vacuum. The resulting filter cake was the target product **F1** (55.0 g in total), which was directly used in the next step without further purification. ESI-MS: m/z =354.0 $[M+H]^+$.

**(7) Preparation method for compound G1:**

Compound **F1** (25.0 g), bis(pinacolato)diboron (20.2 g), Pd(dtbpf)$Cl_2$ (3.9 g), and potassium acetate (15.6 g) were dissolved in 1.0 L of a 1,4-dioxane solution. The reaction solution was stirred at 100 °C for about 2 h under nitrogen atmosphere. After the reaction was completed, the reaction solution was cooled to room temperature and filtered under vacuum. The resulting filter cake was slurried in a mixed solvent of petroleum ether and ethyl acetate for 1 h and filtered under vacuum. The resulting filter cake was the target product **G1** (20.0 g in total), which was directly used in the next step without further purification.

**(8) Preparation method for compound H1:**

4-Chloro-2,5-difluorobenzonitrile (93.5 g) and cyclopropylalcohol (31.3 g) were dissolved in 935 mL of tetrahydrofuran. The reaction solution was cooled to -25 to -15 °C. BuOK-THF (594 mL, 594 mmol) was added dropwise. After 30 min of dropwise addition, the reaction solution was reacted at the same temperature for 2 h until the reaction was completed. Purified water (935 mL) was added at 0-10 °C for quenching, and the reaction solution was extracted with EA, washed with saturated brine, dried, and concentrated to dryness at 45-55 °C to give compound **H1** (113 g in total). ESI-MS: m/z = 212 $[M+H]^+$.

**(9) Preparation method for compound 11:**

Compound **H1** (110 g) was dissolved in tetrahydrofuran (1650 mL). The reaction solution was purged with $N_2$, and then cooled to -80 to -70 °C. A solution of 2 N LDA in tetrahydrofuran (390 mL, 780 mmol) was added dropwise. After 20 min of dropwise addition, a solution of $I_2$ (264 g, 1040 mmol) in tetrahydrofuran (1650 mL) was added dropwise. After 30 min of dropwise addition, the reaction solution was heated to 20-30 °C and reacted for above 1 h until compound **H1** was completely reacted. Water was added for quenching, and the reaction solution was extracted with EA (ethyl acetate), washed with saturated brine, dried, and concentrated to dryness at 40-50 °C to give a crude product, which was subjected to column chromatography to give compound **I1**, (105 g in total), ESI-MS: m/z = 339 $[M+H]^+$.

**(10) Preparation method for compound J1:**

Compound **I1** (100g), pyrazole 5-borate (123 g), sodium carbonate (94.4 g), and 1,1'-bis(di-*tert*-butylphosphine) ferrocene dichloropalladium (19.4 g) were dissolved in dioxane-water (5:1) (2000 mL). The reaction solution was purged with $N_2$, and then heated to 85 °C and reacted for above 5 h. The reaction solution was cooled to room temperature, concentrated to dryness at 40-50 °C and extracted with water and EA (ethyl acetate). The organic phase was isolated, washed with water and saturated brine, dried, and concentrated to dryness at 40-50 °C to give a crude product, which was subjected to column chromatography to give compound **J1** (41 g in total), ESI-MS: m/z = 292 $[M+H]^+$.

**(11) Preparation method for compound K1:**

Compound **J1** (40 g) and NIS (61.8 g) were dissolved in glacial acetic acid (800 mL). The reaction solution was heated to 80 °C and reacted for above 2 h until compound **J1** was completely reacted. The reaction solution was cooled to room temperature, concentrated to dryness at 45-55 °C, and extracted with water and EA. The organic phase was isolated, washed with a saturated sodium bicarbonate solution and saturated brine, dried, and concentrated to dryness at 40-50 °C to give a crude product, which was subject to column chromatography to give compound **K1**, (43 g in total), ESI-MS: m/z = 418 $[M+H]^+$.

**(12) Preparation method for compound L1:**

Compound **K1** (17.3 g), compound **G1** (20 g), sodium carbonate (17.6 g), and tetrakis(triphenylphosphine) palladium (7.19 g) were dissolved in dioxane-water (5:1) (1730 mL). The reaction solution was purged with $N_2$, heated to 85 °C, and reacted for 1 h until the reaction was completed. The reaction solution was cooled to room temperature, concentrated to dryness at 45-55 °C, and extracted with water and EA. The organic phase was isolated, washed with water and saturated brined, dried, and concentrated to dryness at 40-50 °C to give compound **L1** (25.5 g), ESI-MS: m/z = 565 $[M+H]^+$.

**(13) Preparation method for compound M1:**

Compound **L1** (282 mg), tributylvinyltin (475.5 mg), tetrakis(triphenylphosphine)palladium (100 mg), and cesium fluoride (380 mg) were stirred in dioxane (15 mL) and reacted at 100 °C under nitrogen atmosphere. After the reaction was completed, the reaction solution was filtered under vacuum and concentrated under reduced pressure to give compound **M1** (500 mg), which was directly used in the next step without further purification. LC-MS: m/z 557.18 $(M+H)^+$.

**(14) Preparation method for compound 1:**

Compound **M1** (500 mg) was dissolved in 4 mol/L hydrogen chloride in ethyl acetate (10 mL). The reaction solution was reacted at room temperature. After the reaction was completed, the reaction solution was concentrated under reduced pressure, and separated and purified by preparative liquid phase chromatography (liquid phase chromatography conditions: column: Novasep C18 250 × 50 mm, 10 μm; mobile phase: A: 0.05% formic acid-water, C: acetonitrile, gradient: 10% C-40% C (0-60 min); flow rate: 50 mL/min; wavelength: 254 nm, retention time: 24.09 min) to give compound **1** (90 mg). LC-MS: m/z 457.30 $(M+H)^+$. $^1$H NMR (500 MHz, DMSO-*d6*) δ 12.67 (s, 1H), 8.31 (d, *J*= 7.9 Hz, 2H), 8.15 (d, *J* = 8.3 Hz, 1H), 7.93 (d, *J* = 5.8 Hz, 1H), 7.78 (s, 1H), 7.58 (d, *J* = 8.3 Hz, 1H), 6.94 (dd, *J* = 17.6, 11.2 Hz, 1H), 6.30 (d, *J* = 17.6 Hz, 1H), 5.77 (d, *J* = 11.2 Hz, 1H), 4.23 (d, *J* = 2.6 Hz, 1H), 4.06 (s, 2H), 3.77 (s, 3H), 1.02-0.88 (m, 2H), 0.81 (d, *J* = 6.6 Hz, 2H).

**(15) Preparation method for compounds 1a and 1b:**

Compound 1 was further subjected to chiral resolution (liquid phase chromatography conditions: column: YMC Cellulose-SC, 30 × 250 mm, 10 μm; A: 0.05% diethylamine (dichloromethane: n-hexane = 1:1), B: 0.1% diethylamine-methanol; gradient: 20-80 B-40 min; λ = 254 nm, V = 30 mL/min) to give compounds **1a** and **1b** in sequence.

**Example 2: Preparation of Compounds 2, 2a, and 2b**

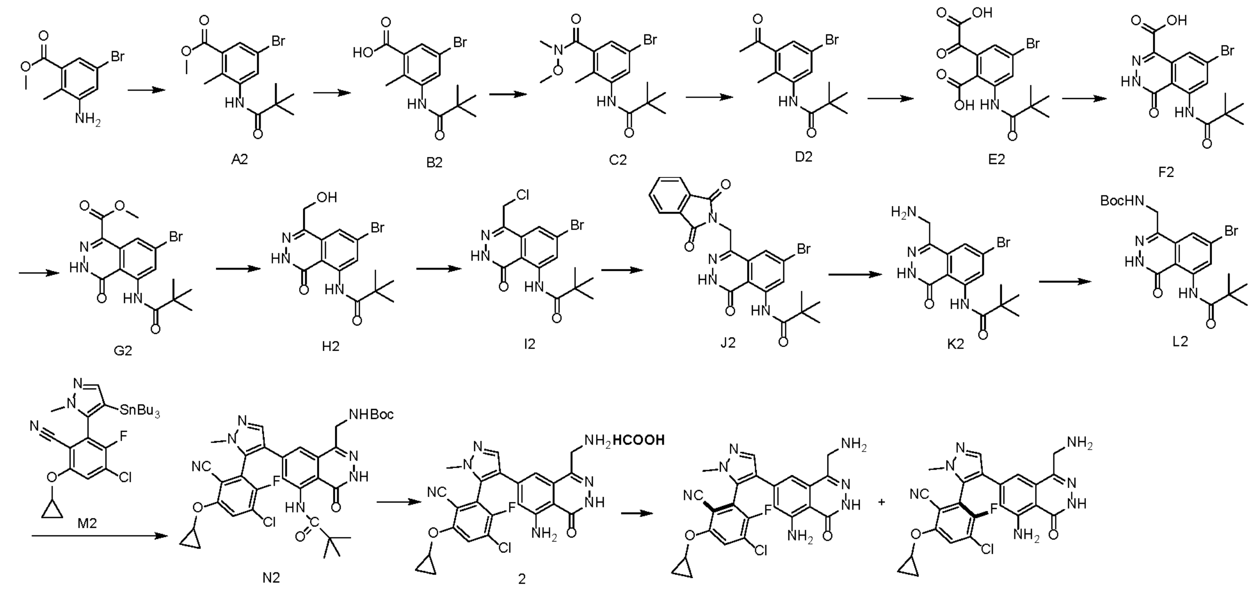


**(1) Preparation method for compound A2:**

2-Methyl-3-amino-5-bromobenzoate (80 g) and triethylamine (50 mL) were dispersed in tetrahydrofuran (800 mL). The reaction solution was cooled to 0 °C, and then pivaloyl chloride (44 mL) was added dropwise. After the dropwise addition, the reaction solution was stirred at room temperature for 5 h. After the reaction was completed, water (800 mL) was added to the reaction system to quench the reaction, and the reaction solution was extracted with ethyl acetate (800 mL). The resulting organic phase was washed with water and then with saturated brine. The resulting organic phase was concentrated to dryness to give compound **A2** (117 g). ESI-MS: m/z = 328.17 $[M+H]^+$.

**(2) Preparation method for compound B2:**

Compound **A2** (117 g) was dissolved in tetrahydrofuran (400 mL), and a solution of sodium hydroxide (57 g) in water (100 mL) was added at room temperature. After the dropwise addition, the reaction solution was stirred at room temperature overnight. After the reaction was completed, the reaction system was concentrated to remove tetrahydrofuran, and a 1 N hydrochloric acid solution was added to the reaction system to adjust the pH to 5-6. The reaction solution was filtered under vacuum. The resulting filter cake was compound **B2** (120 g). ESI-MS: m/z = 314.17 $[M+H]^+$.

**(3) Preparation method for compound** C2:

Compound **B2** (120 g), dimethylhydroxylamine hydrochloride (56 g), HATU (175 g), and DIPEA (99 g) were dispersed in *N,N*-dimethylformamide (1.5 L). The reaction solution was stirred at room temperature for 2 h and then the reaction was stopped. Water was added to the reaction system to quench the reaction, and the reaction solution was extracted with ethyl acetate. The resulting aqueous phase was extracted with ethyl acetate. The resulting organic phases were combined, washed with water, and finally washed with saturated brine. The resulting organic phase was concentrated to dryness to give compound **C2** (90 g in total). ESI-MS: m/z = 357.23 $[M+H]^+$.

**(4) Preparation method for compound D2:**

Methyl magnesium bromide (3 M, 253 mL) was dissolved in a tetrahydrofuran solution (0.8 L). The reaction system was purged with nitrogen. A solution of compound **C2** (90 g) in tetrahydrofuran (0.2L) was slowly added to the reaction system at 0 °C. After the addition, the reaction solution was stirred at room temperature for 3 h and then the reaction was stopped. Water was added to quench the reaction, and the reaction solution was extracted with ethyl acetate. The resulting organic phase was washed with water and finally washed with saturated brine. The organic phase was concentrated to dryness to give compound **D2** (75 g in total). ESI-MS: m/z = 312.16 $[M+H]^+$.

**(5) Preparation method for compound E2:**

Compound **D2** (75 g), potassium carbonate (66 g), and potassium permanganate (265 g) were dispersed in water

(0.8 L). The reaction solution was stirred at 50 °C for 5 h and then the reaction was stopped. A saturated solution of sodium thiosulfate was added to quench the reaction, and the reaction solution was filtered under vacuum. The resulting organic phase was adjusted to pH 2 with 2 N HCl, and a mixture of ethyl acetate and tetrahydrofuran (v:v = 10:1, 1.2 L × 3) was added. The resulting organic phases were combined and finally washed with saturated brine. The organic phase was concentrated to dryness to give compound **E2** (47 g in total).

**(6) Preparation method for compound F2:**

Compound **E2** (47 g) and hydrazine hydrate (7.7 g) were dispersed in ethanol (470 mL). The reaction solution was stirred at 75 °C for 5 h and then the reaction was stopped. The reaction system was cooled and then filtered under vacuum. The resulting filter cake was compound **F2** (10 g in total).

**(7) Preparation method for compound G2:**

Compound **F2** (10 g) and a solution of hydrogen chloride in methanol (4 M, 136 mL) were dispersed in methanol (136 mL). The reaction solution was stirred at 70 °C for 36 h and then the reaction was stopped. The reaction system was concentrated to dryness, and then water (35 mL) was added. The resulting mixture was adjusted to pH 8 with a 1 N NaOH solution, stirred for 0.5 h, and filtered under vacuum. The resulting filter cake was first washed with water (15 mL) and then with ethanol (30 mL). The resulting filter cake was compound **G2** (11 g in total).

**(8) Preparation method for compound H2:**

Compound **G2** (4 g) was dispersed in ethanol (60 mL), and sodium borohydride (740 mg) was added in portions at 0 °C, followed by calcium chloride (1.3 g). After the addition, the reaction system was transferred to room temperature, stirred for 1 h, and then the reaction was stopped. A saturated ammonium chloride solution (200 mL) was added to quench the reaction. Then, the reaction system was concentrated to dryness to remove ethanol, diluted with water (200 mL), and then filtered under vacuum. The filter cake was washed with water. The resulting crude product was slurried with methanol for 10 h, and filtered under vacuum. The resulting filter cake was compound **H2** (3 g in total).

**(9) Preparation method for compound I2:**

Compound **H2** (3 g) was dispersed in thionyl chloride (33 mL). The reaction solution was stirred at 70 °C for 2 h, and then the reaction was stopped. The reaction system was concentrated to dryness, slurried with petroleum ether for 0.5 h, and then filtered under vacuum. The filter cake was compound **I2** (2.8 g in total).

**(10) Preparation method for compound J2:**

Compound **I2** (2.8 g) and potassium phthalimide (1.7 g) were dispersed in *N,N*-dimethylformamide (75 mL). The reaction solution was stirred at room temperature for 1 h, and then the reaction was stopped. The reaction system was washed with HCl (0.5 M, 30 mL), and filtered under vacuum. The resulting filter cake was washed with a saturated sodium bicarbonate solution, and finally washed with pure water. The resulting filter cake was slurried in ethanol at 70 °C for 1 h, and filtered under vacuum. The resulting filter cake was compound **J2** (600 mg in total).

**(11) Preparation method for compound K2:**

Compound **J2** (600 mg) and hydrazine hydrate (320 mg) were dispersed in ethanol (5 mL). The reaction solution was stirred at 80 °C for 2 h, and then the reaction was stopped. The reaction system was concentrated to dryness. The resulting crude product was washed with water, slurried with ethyl acetate at room temperature for 2 h, and directly filtered under vacuum. The resulting filter cake was compound **K2,** (410 mg in total).

**(12) Preparation method for compound L2:**

Compound **K2** (410 mg), triethylamine (351 mg), and $(Boc)_2O$ (506 mg) were dispersed in dichloromethane (10 mL). The reaction solution was stirred at room temperature for 2 h, and then the reaction was stopped. The reaction system was directly concentrated to dryness, and purified by column chromatography to give compound **L2,** (380 mg in total).

**(13) Preparation method for compound M2:**

Compound **K1** (2.4 g) in Example 1, hexabutylditin (10.0 g), lithium chloride (1.3 g), $Pd_2(dba)_3$ (0.5 g), and tricyclohexylphosphine (320 mg) were dispersed in 1,4-dioxane (30 mL). The reaction solution was stirred at 100 °C for 6 h under nitrogen atmosphere, and then the reaction was stopped. The reaction system was filtered under vacuum. The resulting filtrate was concentrated to dryness. The resulting crude product was subjected to column chromatography to give compound **M2** (1.5 g in total).

**(14) Preparation method for compound N2:**

Compound **L2** (226 mg), compound **M2** (872 mg), cesium fluoride (160 mg), copper(I) iodide (120 mg), and tetrakis(triphenylphosphine)palladium (120 mg) were dispersed in *N,N*-dimethylformamide (20 mL). The reaction solution was stirred at 100 °C for 3 h under nitrogen atmosphere. Compound **M2** (872 mg) was additionally added. The reaction solution was stirred at the same temperature for 3 h, and then the reaction was stopped. The reaction solution was filtered under vacuum. The filter cake was rinsed with ethyl acetate. The resulting filtrate was concentrated to dryness to give crude product **N2** (1.8 g in total).

**(15) Preparation method for compound 2:**

Compound **N2** (1.8 g) was dissolved in hydrogen chloride/methanol (4 M, 10 mL). The reaction solution was stirred at 80 °C for 36 h, and then the reaction was stopped. The resulting crude product was concentrated to dryness, and separated and purified by preparative liquid phase chromatography (liquid phase chromatography conditions: column: YMC AQ C18 30 × 250 mm, 10 μm; mobile phase: A: 0.1% formic acid, B: acetonitrile, gradient: 15% B-35% B (0-90 min); flow rate: 25 mL/min; wavelength: 254 nm; retention time: 30.0 min) to give compound **2** (1.6 g in total). ESI-MS: m/z = 480.19 $[M+H]^+$. $^1H$ NMR (500 MHz, DMSO-$d_6$) δ 12.46 (s, 1H), 8.43 (t, *J* = 5.8 Hz, 3H), 8.19 (s, 1H), 7.99 (d, *J* = 6.0 Hz, 1H), 6.75 (d, *J* = 1.5 Hz, 1H), 6.52 (d, *J* = 1.4 Hz, 1H), 4.21 (td, *J* = 6.1, 3.0 Hz, 1H), 4.19-4.12 (m, 2H), 3.74 (s, 3H), 2.54 (s, 2H), 0.96-0.89 (m, 2H), 0.88-0.78 (m, 2H).

**(16) Preparation method for compounds 2a and 2b:**

Compound **2** was further subjected to chiral resolution.

**Example 3: Preparation of Compounds 3, 3a, and 3b**

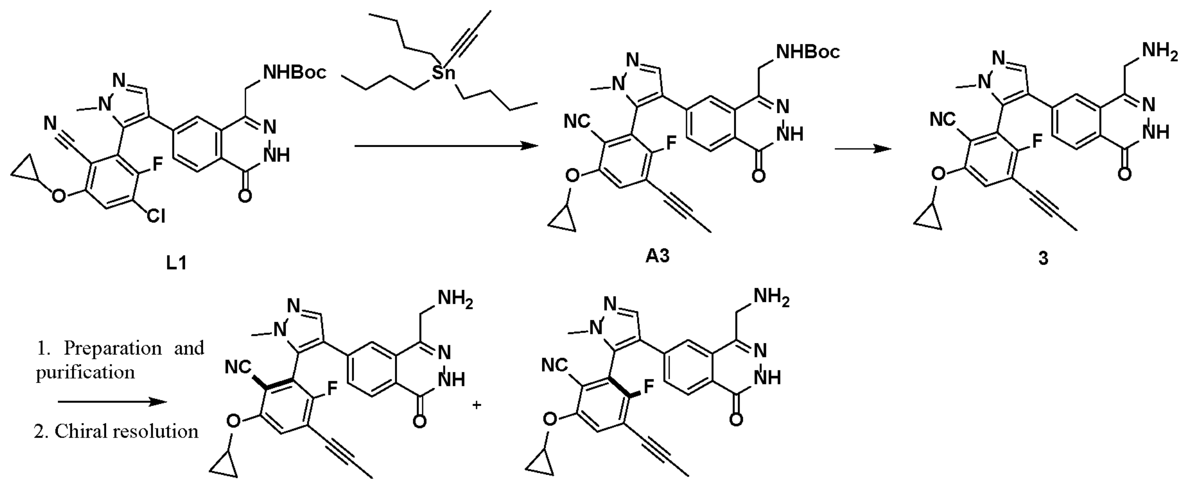


L1 A3 3

3a 3b

**(1) Preparation method for compound A3:**

Intermediate **L1** (3.10 g), tributylpropinyltin (5.43 g), tetrakis(triphenylphosphine)palladium (954 mg), and sodium carbonate (2.32 g) were dissolved in a mixed solvent (357 mL) of 1,4-dioxane and water (5:1). The reaction

solution was reacted at 85-90 °C for 7 h under nitrogen atmosphere.

After the reaction was completed, the reaction solution was filtered under vacuum, and concentrated at 45-55 °C until no liquid flowed out. The concentrate was completely dissolved with ethyl acetate (100 mL), and then washed with water and saturated brine. The organic phase was isolated, dried, and concentrated at 45-55 °C until no liquid existed to give intermediate **A3.** ESI-MS: m/z =569.30[M+H]$^+$.

**(2) Preparation method for compound 3:**

Intermediate **A3** (3.10 g) and trifluoroacetic acid (10.9 mL) were dissolved in dichloromethane (85 mL). The reaction solution was reacted at 10-30 °C for about 2 h.

After the reaction was completed, the filtrate was concentrated at 35-50 °C until no liquid flowed out. Dichloromethane (30 mL) was added, and the resulting mixture was concentrated to dryness by rotary evaporation. This process was repeated for 4 times (with rotary evaporation under high vacuum for above 30 min). The resulting crude product was separated and purified by preparative liquid phase chromatography (liquid phase chromatography conditions: column: Daisogel C18 50 × 250 mm, 10 μm; A: 0.1% formic acid, B: acetonitrile; gradient: 20% A-70% (0-40 min); λ: 254 nm, V: 60 mL/min) to give compound **3.** ESI-MS: m/z = 469.24[M+H]$^+$.

**(3) Preparation method for compounds 3a and 3b:**

Compound **3** was subjected to chiral resolution (liquid phase chromatography conditions: column: YMC Cellulose-SC 30 × 250 mm, 10 μm; A: 0.2% diethylamine-methanol, B: dichloromethane-n-hexane = 2:3; gradient: 20% A-80% B (0-40 min); λ: 254 nm, V: 40 mL/min) to give compounds **3a** and **3b** in sequence.

**3a:** ESI-MS: m/z = 469.24[M+H]$^+$, $^1$H NMR (500 MHz, DMSO) δ 12.53 (s, 1H), 8.35 (s, 2H), 8.27 (s, 1H), 8.15 (d, J = 8.3 Hz, 1H), 7.78 (d, J = 5.6 Hz, 1H), 7.73 (s, 1H), 7.63 (d, J = 8.3 Hz, 1H), 4.16 (d, J = 2.8 Hz, 1H), 3.88 (s, 2H), 3.76 (s, 3H), 2.17 (s, 3H), 0.89 (d, J = 5.8 Hz, 2H), 0.79 (d, J = 2.3 Hz, 2H).

**3b:** ESI-MS: m/z = 469.24[M+H]$^+$, $^1$H NMR (500 MHz, DMSO) δ 12.52 (s, 1H), 8.37 (s, 2H), 8.27 (s, 1H), 8.15 (d, J = 8.3 Hz, 1H), 7.78 (d, J = 5.6 Hz, 1H), 7.73 (s, 1H), 7.64 (dd, J = 8.3, 1.4 Hz, 1H), 4.18-4.14 (m, 1H), 3.87 (s, 2H), 3.76 (s, 3H), 2.17 (s, 3H), 0.89 (d, J = 6.0 Hz, 2H), 0.79 (d, J = 2.3 Hz, 2H).

**Example 4: Preparation of Compound 4**

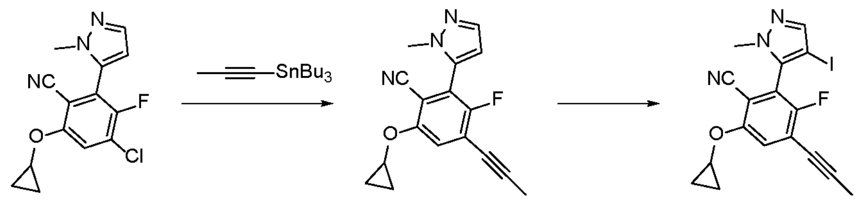


J1 A4 B4

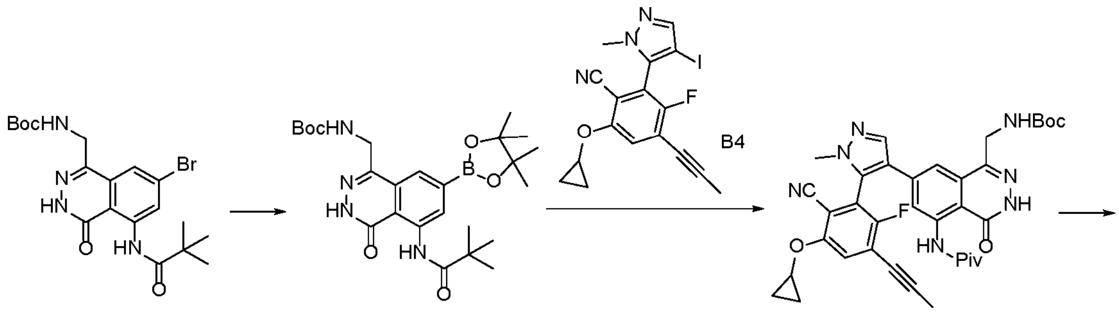


L2 C4 D4

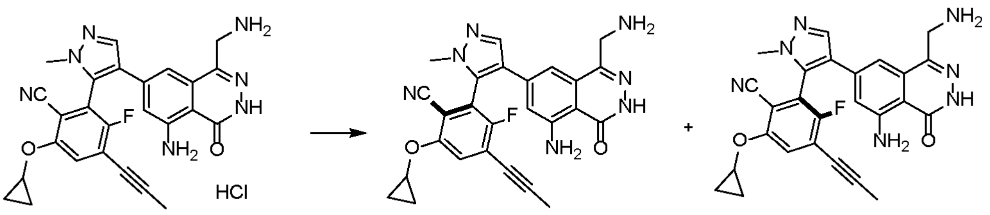


4 4a 4b

**(1) Preparation method for compound A4:**

Compound **J1** (1.5 g), tributylpropynylstannane (4.9 g), tetrakis(triphenylphosphine)palladium (1.2 g), and cesium fluoride (2.3 g) were stirred in a mixed solution of dioxane (20 mL) and water (4 mL) and reacted at 100 °C under nitrogen atmosphere. After the reaction was completed, the reaction solution was filtered under vacuum, concentrated under reduced pressure, and purified by silica gel column chromatography to give **A4** (1.4 g).

LC-MS: m/z 296.15 $(M+H)^+$.

**(2) Preparation method for compound B4:**

Compound **A4** (1.4 g) and *N*-iodosuccinimide (2.1 g) were stirred in acetic acid (15 mL) and reacted at 80 °C for 3 h under nitrogen atmosphere. After the reaction was completed, the reaction system was concentrated to dryness. Water (10 mL) and ethyl acetate (20 mL × 2) were added. The organic phases were combined, and washed with saturated sodium bicarbonate (20 mL) and saturated sodium chloride (20 mL) in sequence. The resulting organic phase was concentrated to dryness to give a crude product, which was subjected to column chromatography to give compound **B4** (1.2 g).

LC-MS: m/z 422.13 $(M+H)^+$.

**(3) Preparation method for compound C4:**

Compound **L2** (2.7 g), bis(pinacolato)diboron (3.1 g), $Pd(dppf)Cl_2*CH_2Cl_2$ (486 mg), and potassium acetate (1.8 g) were stirred in dioxane (60 mL) and reacted at 85 °C under nitrogen atmosphere. After the reaction was completed, the reaction solution was filtered under vacuum, and concentrated under reduced pressure to give a crude product of compound **C4.** The crude product was slurried with a mixed solvent of petroleum ether (40 mL) and ethyl acetate (4 mL) for 1 h, and filtered under vacuum. The resulting filter cake was compound **C4** (2.5 g in total).

LC-MS: m/z 419.17 $(M-82+1)^+$.

**(4) Preparation method for compound D4:**

Compound **C4** (800 mg), compound **B4** (741 mg), tetrakis(triphenylphosphine)palladium (555 mg), and sodium carbonate (678 mg) were stirred in a mixed solution of dioxane (20 mL) and water (4 mL), and reacted at 110 °C under nitrogen atmosphere. After the reaction was completed, the reaction solution was filtered under vacuum, and concentrated under reduced pressure to give a crude product of compound **D4,** which was subjected to column chromatography to give compound **D4** (120 mg).

LC-MS: m/z 668.33 $(M+H)^+$.

**(5) Preparation method for compound 4:**

Compound **D4** was dissolved in hydrochloric acid/methanol (10 mL). The reaction solution was stirred under reflux at 80 °C for 48 h. After the reaction was completed, the reaction solution was directly concentrated to dryness to give a crude product of compound **4** (80 mg). The crude product was separated and purified by preparative liquid phase chromatography (liquid phase chromatography conditions: column: YMC-SC 30 × 250 mm, 10 μm; A: *n*-hexane:dichloromethane = 3:1, B: 0.2% diethylamine/ethanol; gradient: 5% B-60% B (0-60 min); λ: 254 nm, V: 30 mL/min) to give compound 4 (30 mg).

LC-MS: m/z484.19 $(M+H)^+$.

$^1$H NMR (500 MHz, DMSO-d6) δ 12.22 (s, 1H), 8.29 (s, 1H), 8.08 (s, 1H), 7.75 (d, J = 5.6 Hz, 1H), 7.32 (s, 2H), 6.65 (d, J = 1.6 Hz, 1H), 6.62 (d, J = 1.4 Hz, 1H), 4.15 (tt, J = 6.0, 2.9 Hz, 1H), 3.83 (s, 2H), 3.72 (s, 3H), 2.17 (s, 3H), 0.92-0.88 (m, 2H), 0.82-0.78 (m, 2H).

**(6) Preparation of Compounds 4a and 4b**

Compound **4** was subjected to preparative liquid phase chromatography (YMC Cellulose-SC 5 μm, 30 × 250 chromatographic column; 0.1% diethylamine ethanol-*n*-hexane:dichloromethane = 3:1 (10% -50%/0-40 min)) to give compound **4a** and compound **4b** in sequence.

**4a:** LC-MS: m/z 484.19 $(M+H)^+$, $^1$H NMR (500 MHz, DMSO-d6) δ 12.10 (s, 1H), 8.06 (s, 1H), 7.77 (d, J = 5.6 Hz, 1H), 7.32 (s, 2H), 6.66 (dd, J = 40.5, 1.6 Hz, 2H), 4.16 (tt, J = 6.0, 2.9 Hz, 1H), 3.70 (d, J = 42.7 Hz, 5H), 2.19 (s, 3H), 0.95-0.78 (m, 4H).

**4b:** LC-MS: m/z 484.19 $(M+H)^+$, $^1$H NMR (500 MHz, DMSO-$d_6$) δ 12.08 (s, 1H), 8.05 (s, 1H), 7.77 (d, *J* = 5.7 Hz, 1H), 7.31 (s, 2H), 6.66 (dd, *J* = 48.7, 1.5 Hz, 2H), 4.15 (tt, *J* = 6.1, 2.9 Hz, 1H), 3.68 (d, *J* = 57.1 Hz, 5H), 2.19 (s, 3H), 0.94-0.78 (m, 4H).

**Example 5: Preparation of Compound 5**

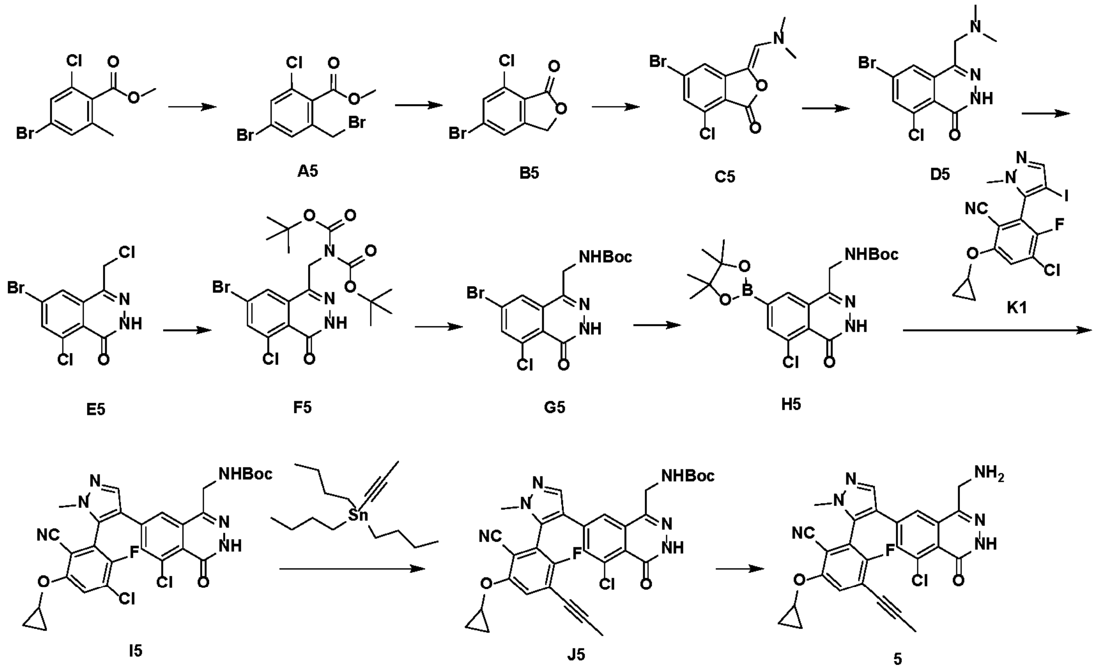


**(1) Preparation method for compound A5:**

Methyl 4-bromo-2-chloro-6-methylbenzoate (45.05 g), *N*-bromosuccinimide (36.74 g), and benzoyl peroxide (purity: 75% wt) (10.42 g) were dissolved in carbon tetrachloride (450 mL). The reaction solution was reacted at 85 °C for 12 h under nitrogen atmosphere. After the reaction was completed, the reaction solution was extracted with water (450 mL) and dichloromethane (900 mL). The organic phase was isolated and washed with water and saturated brine. The resulting organic phase filtrate was concentrated until no liquid existed to give intermediate **A5** (67 g).

**(2) Preparation method for compound B5:**

Intermediate **A5** (63.0 g) and calcium carbonate (111 g) were dissolved in a mixed solvent (2500 mL) of dioxane and water (1:1). The reaction solution was reacted at 90 °C for 12 h. After the reaction was completed, the reaction solution was filtered under vacuum. The filter cake was washed with a mixed solvent (400 mL) of dioxane and water (1:1), concentrated at 50-60 °C until no liquid flowed out, and extracted with dichloromethane. The organic phases were combined, washed with water and saturated brine, dried, and then concentrated to dryness to give intermediate **B5** (32.1 g).

**(3) Preparation method for compound C5:**

Intermediate **B5** (32.0 g) was dissolved in *N,N*-dimethylformamide dimethyl acetal (112 mL), and potassium *tert*-butoxide (1459 mg) was added. The reaction mixture was reacted at 105-110 °C for 5 h. After the reaction was completed, the reaction solution was concentrated at 50-60 °C until no liquid flowed out. The concentrate was slurried with petroleum ether (56 mL) at room temperature for 30 min, and filtered to remove liquid. The filter cake was slurried with ethyl acetate (112 mL) at 80 °C for 5 h, cooled, and filtered under vacuum to give intermediate **C5** (16.60 g), ESI-MS: m/z = 301.96 $[M+H]^+$.

**(4) Preparation method for compound D5:**

Intermediate **C5** (15.80 g) was dissolved in absolute ethanol (160 mL), and hydrazine hydrate (6.4 mL) was added. The reaction solution was purged with nitrogen, and then reacted at 80 °C for 10 h. After the reaction was completed, the reaction solution was stirred at room temperature for 1 h, stirred at -10 °C to 0 °C for 4 h, and filtered under vacuum to give intermediate **D5** (13.14 g), ESI-MS: m/z = 315.99 $[M+H]^+$.

**(5) Preparation method for compound E5:**

Intermediate **D5** (11.34 g) was dissolved in tetrahydrofuran (114 mL). The reaction solution was purged with nitrogen, and cooled to -5 °C to 0 °C. Isobutyl chloroformate (7.37 g) was added at the same temperature. After the addition, the resulting mixture was warm to 25 °C and reacted for 5 h. After the reaction was completed, the reaction solution was concentrated to dryness, slurried with 0.5 M hydrochloric acid (100 mL) and water (144 mL) in sequence, and filtered under vacuum to give intermediate **E5** (7.27 g), ESI-MS: m/z = 306.89 $[M+H]^+$.

**(6) Preparation method for compound F5:**

Bis(*tert*-butyloxycarbonyl)amine (4790 mg) was dissolved in tetrahydrofuran (129 mL). The reaction solution was purged with nitrogen, and then cooled to -30 °C. Lithium bis(trimethylsilyl)amide (1 N, 23.1 mL) was added dropwise. After the dropwise addition, the resulting mixture was stirred at the same temperature for 30 min. A solution of intermediate **E5** in tetrahydrofuran (6424 mg in 129 mL of tetrahydrofuran) was added dropwise. After the dropwise addition, the resulting mixture was heated to 25 °C and reacted for 3 h. After the reaction was completed, the reaction was quenched with a saturated ammonium chloride solution (100 mL), and the reaction solution was extracted with ethyl acetate. The organic phases were combined, washed with saturated brine, dried, and concentrated to dryness to give intermediate **F5** (9.96 g), ESI-MS: m/z = 488.14 $[M+H]^+$.

**(7) Preparation method for compound G5:**

Intermediate **F5** (9300 mg) was dissolved in acetonitrile (158 mL), and magnesium perchlorate hexahydrate (1266 mg) was added. The reaction solution was purged with nitrogen, and then heated to 50 °C and reacted for 5 h. After the reaction was completed, the reaction solution was cooled to room temperature. Water (158 mL) was added. The resulting mixture was stirred for 10 min, and then filtered under vacuum. The filter cake was washed with water, acetonitrile, and petroleum ether in sequence. The resulting filter cake was dried under reduced pressure to give intermediate **G5** (6.16 g), ESI-MS: m/z = 388.08 $[M+H]^+$.

**(8) Preparation method for compound H5:**

Intermediate **G5** (2322 mg), bis(pinacolato)diboron (2286 mg), [1,1'-bis (diphenylphosphino)ferrocene]palladium (II) chloride (219.6 mg), and potassium acetate (1767 mg) were dissolved in 1,4-dioxane (140 mL). The reaction solution was purged with nitrogen, and then heated to 100 °C and reacted for 6 h.

After the reaction was completed, the reaction solution was cooled and filtered under vacuum. The filtrate was concentrated to dryness to give a concentrate. The concentrate was dissolved with ethyl acetate (25 mL) under stirring, and water (25 mL) was added. The resulting mixture was stirred for 10 min, and filtered under vacuum. The filter cake was rinsed with petroleum ether to give intermediate **H5** (1830 mg), ESI-MS: m/z = 354.12 $[M-80]^+$.

**(9) Preparation method for compound I5**:

Intermediate **H5** (1559 mg), intermediate **K1** (1251 mg), tetrakis(triphenylphosphine)palladium (520 mg), and sodium carbonate (1272 mg) were dissolved in a mixed solvent (144 mL) of 1,4-dioxane and water (5:1). The reaction solution was reacted at 85-90 °C for 2 h under nitrogen atmosphere. After the reaction was completed, the reaction solution was filtered under vacuum, and concentrated at 45-55 °C until no liquid flowed out. The concentrate was completely dissolved with ethyl acetate (50 mL), and then washed with water and saturated brine. The organic phase was isolated, dried, then concentrated at 45-55 °C until no liquid existed, and purified by column chromatography to give intermediate **I5** (1060 mg), ESI-MS: m/z = 599.08$[M+H]^+$.

**(10) Preparation method for compound J5:**

Intermediate **I5** (300 mg), tributylpropinyltin (1186 mg), tetrakis(triphenylphosphine)palladium (166.4 mg), and sodium carbonate (458 mg) were dissolved in a mixed solvent (45 mL) of 1,4-dioxane and water (5:1). The reaction solution was reacted at 85-90 °C for 3 h under nitrogen atmosphere. After the reaction was completed, the reaction solution was filtered under vacuum, and concentrated at 45-55 °C until no liquid flowed out. The concentrate was completely dissolved with ethyl acetate (50 mL), and then washed with water and saturated brine. The organic phase was isolated, dried, and concentrated at 45-55 °C until no liquid existed to give intermediate **J5** (680 mg). ESI-MS: m/z = 603.35$[M+H]^+$.

**(1) Preparation method for compound 5:**

Intermediate **J5** (680 mg) and trifluoroacetic acid (2.1 mL) were dissolved in dichloromethane (30 mL). The reaction solution was reacted at 10-30 °C for about 2 h. After the reaction was completed, the reaction solution was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: column: PFP 30 × 250 mm, 10 μm; A: 50 mM ammonium acetate, B: methanol; gradient: 30% A-70% B (0-60 min); λ: 254 nm, V: 20 mL/min) to give compound **5.** ESI-MS: m/z = 503.22[M+H]$^+$.

$^1$H NMR (500 MHz, DMSO-$d_6$) δ 8.35 (s, 1H), 7.81 (d, $J$ = 5.6 Hz, 1H), 7.75 (d, $J$ = 1.8 Hz, 1H), 7.57 (d, $J$ = 1.7 Hz, 1H), 3.76 (s, 4H), 3.66-3.61 (m, 2H), 2.18 (s, 3H), 0.97-0.86 (m, 2H), 0.82-0.74 (m, 2H).

**Example 6: Preparation of Compound 6**

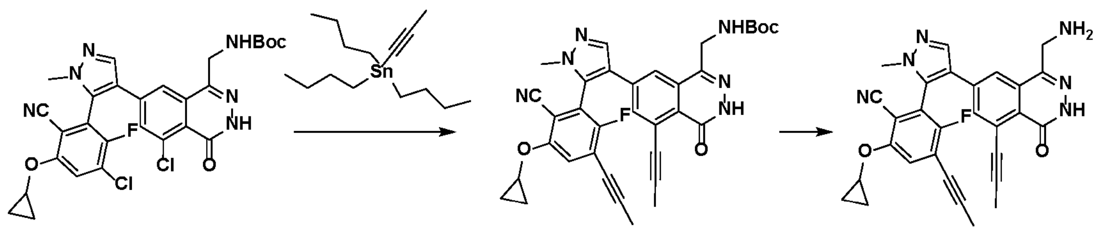

I5 A6 6

**(1) Preparation method for compound A6:**

Intermediate **I5** (300 mg), tributylpropinyltin (1186 mg), tetrakis(triphenylphosphine)palladium (166.4 mg), and sodium carbonate (458 mg) were dissolved in a mixed solvent (45 mL) of 1,4-dioxane and water (5:1). The reaction solution was reacted at 85-90 °C for 3 h under nitrogen atmosphere. After the reaction was completed, the reaction solution was filtered under vacuum. The filtrate was concentrated at 45-55 °C until no liquid flowed out. The concentrate was completely dissolved with ethyl acetate (50 mL), and then washed with water and saturated brine. The organic phase was isolated, dried, and concentrated at 45-55 °C until no liquid existed to give intermediate **A6** (680 mg). ESI-MS: m/z = 607.26[M+H]$^+$.

**(2) Preparation method for compound 6:**

Intermediate **A6** (680 mg) and trifluoroacetic acid (2.1 mL) were dissolved in dichloromethane (30 mL). The reaction solution was reacted at 10-30 °C for about 2 h.

After the reaction was completed, the filtrate was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: column: PFP 30 × 250 mm, 10 μm; A: 50 mM ammonium acetate, B: methanol; gradient: 70% A-100% B (0-60 min); λ: 254 nm, V: 20 mL/min) to give compound **6.** ESI-MS: m/z = 507.25[M+H]$^+$.

$^1$H NMR (500 MHz, DMSO-d6) δ 8.24 (s, 1H), 7.79 (d, J = 5.7 Hz, 1H), 7.41 (s, 1H), 7.30 (s, 1H), 6.35 (s, 1H), 4.16 (s, 1H), 3.77 (s, 3H), 3.57 (s, 1H), 2.24 (s, 3H), 2.18 (s, 3H), 0.90 (d, J = 6.0 Hz, 2H), 0.79 (d, J = 3.6 Hz, 2H).

**Example 7: Preparation of Compound 7**

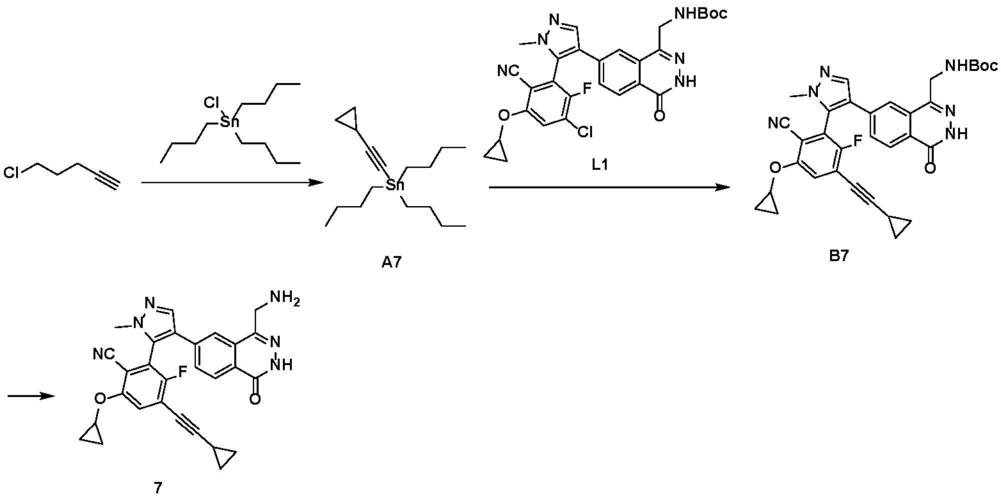

L1

A7

B7

7

**(1) Preparation method for compound A7:**

5-Chloro-1-pentyne (1.02 g) was dissolved in tetrahydrofuran (25 mL). The reaction solution was purged with nitrogen, and then cooled to -30 °C. A solution of n-butyllithium in *n*-hexane solution (2.5 M, 8.2 mL) was added dropwise. After the dropwise addition, the resulting mixture was heated to 10 °C and reacted for 1 h. Tributyltin chloride (3.26 g) was added, and the resulting mixture was heated to 65 °C and reacted for 4 h. After the reaction was completed, the reaction solution was washed with ethyl acetate (150 mL) and a saturated sodium bicarbonate solution (30 mL). The organic phase was isolated, washed with saturated brine, dried, and concentrated to dryness at 45-55 °C to give intermediate **A7** (3250 mg).

**(2) Preparation method for compound B7:**

Intermediate **L1** (169.3 mg), intermediate **A7** (1260 mg), tetrakis(triphenylphosphine)palladium (104.4 mg), and sodium carbonate (191 mg) were dissolved in a mixed solvent (14 mL) of 1,4-dioxane and water (v:v = 5:1). The reaction solution was reacted in a microwave reactor (130 °C, 100 W) for 1 h under nitrogen atmosphere. After the reaction was completed, the reaction solution was filtered under vacuum, and concentrated at 45-55 °C until no liquid flowed out. The concentrate was completely dissolved with ethyl acetate (20 mL), and then washed with water and saturated brine. The organic phase was isolated, dried, and concentrated at 45-55 °C until no liquid existed to give intermediate **B7** (280 mg). ESI-MS: m/z = 595.30[M+H]$^+$.

**(3) Preparation method for compound 7:**

Intermediate **B7** (280 mg) and trifluoroacetic acid (1.3 mL) were dissolved in dichloromethane (7.8 mL). The reaction solution was reacted at 10-30 °C for about 2 h. After the reaction was completed, the filtrate was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: column: YMC AQ C18 50 $\times$ 250 mm, 10 $\mu$m; A: acetonitrile, B: 0.1% ammonium hydroxide; gradient: 10% A-70% B (0-60 min); $\lambda$: 254 nm, V: 60 mL/min) to give compound 7. ESI-MS: m/z = 495.29[M+H]$^+$.

$^1$H NMR (500 MHz, DMSO-$d_6$) $\delta$ 12.58 (s, 1H), 8.27 (d, *J* = 9.3 Hz, 2H), 8.15 (d, *J* = 8.3 Hz, 1H), 7.93 (dd, *J* = 13.3, 5.7 Hz, 1H), 7.75 (dd, *J* = 5.8, 3.7 Hz, 2H), 7.59 (dd, *J* = 8.4, 1.7 Hz, 1H), 4.43 (dq, *J* = 6.6, 3.6, 3.1 Hz, 1H), 4.15 (tt, *J* = 6.2, 3.0 Hz, 2H), 3.96 (s, 2H), 1.68 (tt, *J* = 8.3, 5.0 Hz, 1H), 0.96-0.75 (m, 8H).

**Example 8: Preparation of Compound 8**

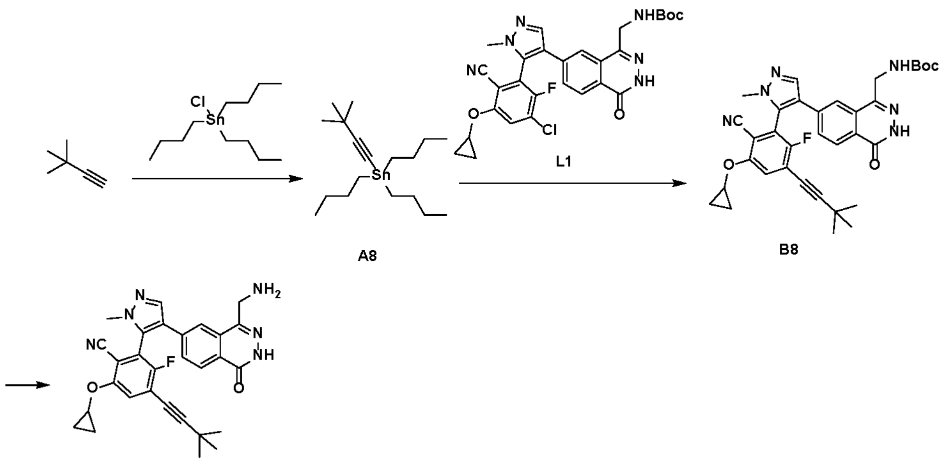

L1

A8

B8

8

**(1) Preparation method for compound A8:**

3,3-Dimethyl-1-butyne (1642 mg) was dissolved in tetrahydrofuran (197 mL). The reaction solution was purged with nitrogen, and then cooled to -78 °C. A solution of *n*-butyllithium in *n*-hexane (2.5 M, 8.0 mL) was added dropwise. After the dropwise addition, the resulting mixture was reacted at the same temperature for 1 h. Tributyltin chloride (6522 mg) was added dropwise. After the dropwise addition, the resulting mixture was reacted for 2 h. After the reaction was completed, the reaction was quenched with a saturated aqueous ammonium chloride solution (150 mL), and extracted with *n*-hexane. The organic phase was isolated, washed with saturated brine, dried over anhydrous magnesium sulfate, and concentrated to dryness at 45-55 °C to give intermediate **A8** (7230 mg).

**(2) Preparation method for compound B8:**

Intermediate **L1** (203.1 mg), intermediate **A8** (1608 mg), tetrakis(triphenylphosphine)palladium (124.8 mg), and sodium carbonate (229 mg) were dissolved in a mixed solvent (14 mL) of 1,4-dioxane and water (v:v = 5:1). The reaction solution was reacted in a microwave reactor (130 °C, 100 W) for 1 h under nitrogen atmosphere. After the reaction was completed, the reaction solution was filtered under vacuum. The filtrate was concentrated at 45-55 °C until no liquid flowed out. The concentrate was completely dissolved with ethyl acetate (20 mL), and then washed with water and saturated brine. The organic phase was isolated, dried, and concentrated at 45-55 °C until no liquid existed to give intermediate **B8** (255 mg). ESI-MS: m/z = 611.41$[M+H]^+$.

**(3) Preparation method for compound 8:**

Intermediate **B8** (255 mg) and trifluoroacetic acid (1.2 mL) were dissolved in dichloromethane (7.5 mL). The reaction solution was reacted at 10-30 °C for about 2 h. After the reaction was completed, the filtrate was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: column: YMC AQ C18 30 $\times$ 250 mm, 10 $\mu$m; A: acetonitrile, B: 30 mM ammonium acetate; gradient: 20% A-80% B (0-60 min); $\lambda$: 254 nm, V: 40 mL/min) to give compound **8.** ESI-MS: m/z = 511.29$[M+H]^+$.

$^1$H NMR (500 MHz, DMSO-$d_6$) $\delta$ 12.42 (s, 1H), 8.24 (s, 1H), 8.13 (d, *J* = 8.4 Hz, 1H), 7.73 (d, *J* = 1.7 Hz, 1H), 7.69 (d, *J* = 5.7 Hz, 1H), 7.64 (dd, *J* = 8.3, 1.7 Hz, 1H), 4.18 (dq, *J* = 6.0, 3.0 Hz, 1H), 3.77 (d, *J* = 1.3 Hz, 2H), 3.73 (s, 3H), 1.30 (s, 9H), 0.90-0.86 (m, 2H), 0.81-0.75 (m, 2H).

**Example 9: Preparation of Compound 9**

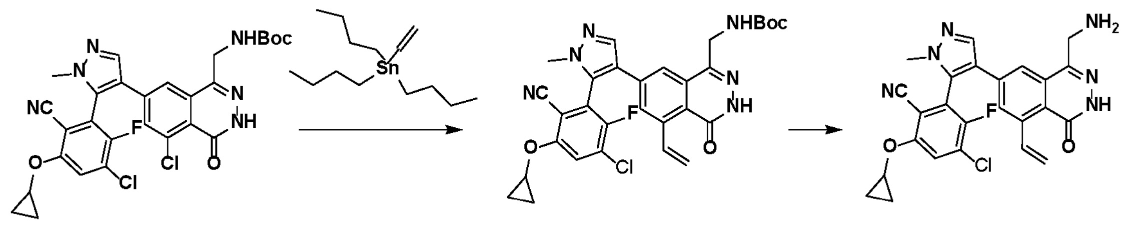

I5 A9 9

## (1) Preparation method for compound A9:

Intermediate **I5** (200 mg), tributylvinylene (916 mg), tetrakis(triphenylphosphine)palladium (112 mg), and sodium carbonate (408 mg) were dissolved in a mixed solvent (35 mL) of 1,4-dioxane and water (5:1). The reaction solution was reacted at 85-90 °C for 8 h under nitrogen atmosphere. After the reaction was completed, the reaction solution was filtered under vacuum, and concentrated at 45-55 °C until no liquid flowed out. The concentrate was completely dissolved with ethyl acetate (20 mL), and then washed with water and saturated brine. The organic phase was isolated, dried, and concentrated at 45-55 °C until no liquid existed to give intermediate **A9** (280 mg). ESI-MS: m/z = 591.31[M+H]$^+$.

## (2) Preparation method for compound 9:

Intermediate **A9** (280 mg) and trifluoroacetic acid (1.6 mL) were dissolved in dichloromethane (20 mL). The reaction solution was reacted at 10-30 °C for about 2 h. After the reaction was completed, the filtrate was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: column: Novasep C18 50 × 250 mm, 10 μm; A: 0.05% ammonium hydroxide, B: acetonitrile; gradient: 40% B-70% B (0-60 min); λ: 254 nm, V: 50 mL/min) to give compound **9.** ESI-MS: m/z = 491.32[M+H]$^+$.

## Example 10: Preparation of Compound 10

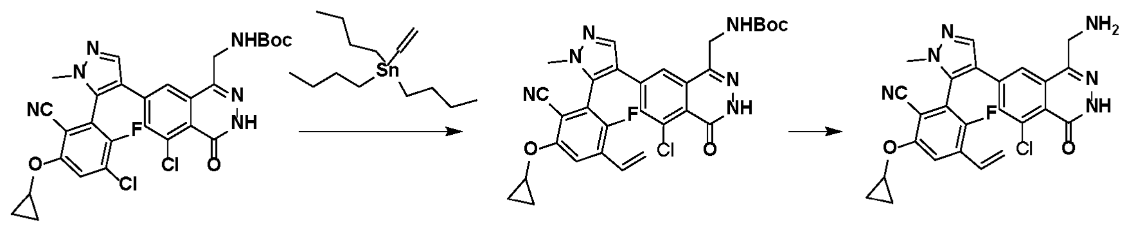

I5 A10 10

## (1) Preparation method for compound A10:

Intermediate **I5** (200 mg), tributylvinylene (916 mg), tetrakis(triphenylphosphine)palladium (112 mg), and sodium carbonate (408 mg) were dissolved in a mixed solvent (35 mL) of 1,4-dioxane and water (v:v = 5:1). The reaction solution was reacted at 85-90 °C for 8 h under nitrogen atmosphere. After the reaction was completed, the reaction solution was filtered under vacuum, and concentrated at 45-55 °C until no liquid flowed out. The concentrate was completely dissolved with ethyl acetate (20 mL), and then washed with water and saturated brine. The organic phase was isolated, dried, and concentrated at 45-55 °C until no liquid existed to give intermediate **A10** (280 mg). ESI-MS: m/z = 591.31[M+H]$^+$.

## (2) Preparation method for compound 10:

Intermediate **A10** (280 mg) and trifluoroacetic acid (1.6 mL) were dissolved in dichloromethane (20 mL). The reaction solution was reacted at 10-30 °C for about 2 h. After the reaction was completed, the filtrate was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase

chromatography conditions: column: Novasep C18 50 × 250 mm, 10 μm; A: 0.05% ammonium hydroxide, B: acetonitrile; gradient: 40% B-70% B (0-60 min); λ: 254 nm, V: 50 mL/min) to give compound **10.** ESI-MS: m/z = 491.32[M+H]+.

**Example 11: Preparation of Compound 11**

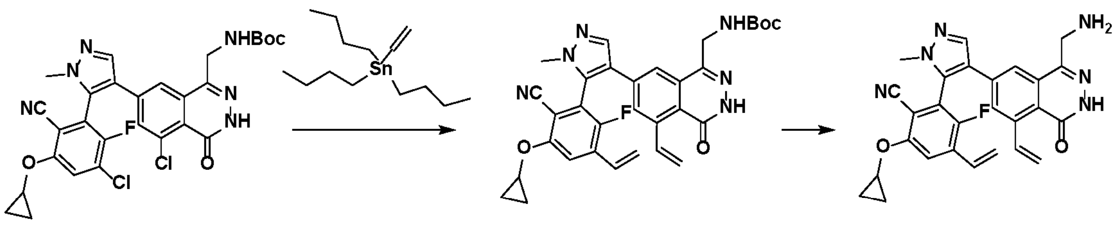

I5 A11 11

**(1) Preparation method for compound A11:**

Intermediate **I5** (200 mg), tributylvinylene (916 mg), tetrakis(triphenylphosphine)palladium (112 mg), and sodium carbonate (408 mg) were dissolved in a mixed solvent (35 mL) of 1,4-dioxane and water (5:1). The reaction solution was reacted at 85-90 °C for 8 h under nitrogen atmosphere. After the reaction was completed, the reaction solution was filtered under vacuum, and concentrated at 45-55 °C until no liquid flowed out. The concentrate was completely dissolved with ethyl acetate (20 mL), and then washed with water and saturated brine. The organic phase was isolated, dried, and concentrated at 45-55 °C until no liquid existed to give intermediate **A11** (280 mg). ESI-MS: m/z = 583.35[M+H]+.

**(2) Preparation method for compound 11:**

Intermediate **A11** (280 mg) and trifluoroacetic acid (1.6 mL) were dissolved in dichloromethane (20 mL). The reaction solution was reacted at 10-30 °C for about 2 h. After the reaction was completed, the filtrate was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: column: Novasep C18 50 × 250 mm, 10 μm; A: 0.05% ammonium hydroxide, B: acetonitrile; gradient: 40% B-70% B (0-60 min); λ: 254 nm, V: 50 mL/min) to give compound **11.** ESI-MS: m/z = 483.35[M+H]+.

$^1$H NMR (500 MHz, DMSO-$d_6$) δ 12.34 (s, 1H), 8.39 (s, 1H), 8.14 (dd, *J* = 17.6, 10.9 Hz, 1H), 7.96 (d, *J* = 5.8 Hz, 1H), 7.76-7.70 (m, 1H), 7.64 (s, 1H), 6.97 (dd, *J* = 17.6, 11.3 Hz, 1H), 6.31 (d, *J* = 17.6 Hz, 1H), 5.79 (d, *J* = 11.1 Hz, 1H), 5.33 (dd, *J* = 28.5, 14.3 Hz, 2H), 4.25 (dt, *J* = 6.1, 3.1 Hz, 1H), 3.77 (d, *J* = 19.6 Hz, 5H), 0.94 (d, *J* = 5.6 Hz, 2H), 0.75 (d, *J* = 13.3 Hz, 2H).

**Example 12: Preparation of Compound 12**

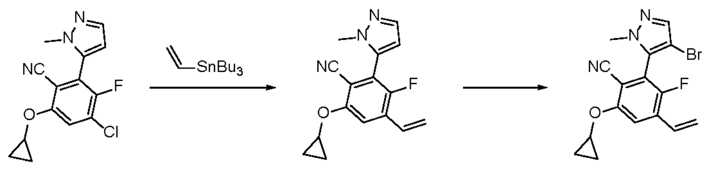

J1 A12 B12

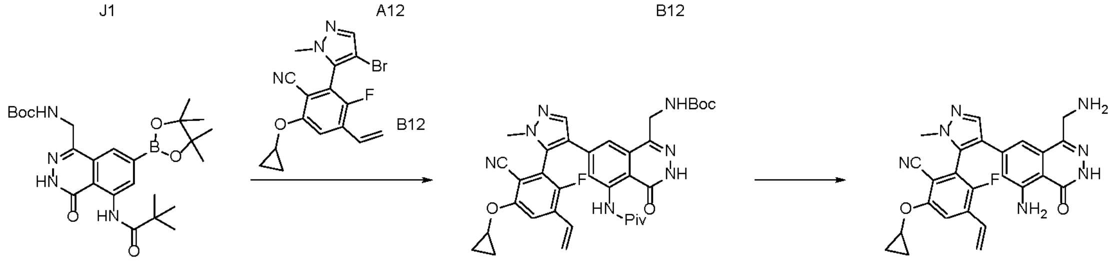


C4 C12 12

(1) Preparation method for compound **A12:**

Compound **J1** (1.5 g), tributylvinyltin (4.8 g), tetrakis(triphenylphosphine)palladium (1.2 g), and cesium fluoride (2.3 g) were stirred in a mixed solution of dioxane (20 mL) and water (4 mL) and reacted at 100 °C under nitrogen atmosphere. After the reaction was completed, the reaction solution was filtered under vacuum, concentrated under reduced pressure, and purified by column chromatography to give compound **A12** (1.1 g).
LC-MS: m/z 284.14 $(M+H)^+$.

(2) Preparation method for compound **B12:**

Compound **A12** (1.1 g) and *N*-bromosuccinimide (1.4 g) were stirred in acetonitrile (20 mL) at room temperature overnight. After the reaction was completed, water (10 mL) and ethyl acetate (20 mL) were added. The organic phases were combined, and washed with saturated sodium bicarbonate (20 mL) and saturated sodium chloride (20 mL) in sequence. The resulting organic phase was concentrated to dryness and purified by column chromatography to give compound **B12** (1.4 g).
LC-MS: m/z 361.96 $(M+H)^+$.

(3) Preparation method for compound **C12:**

Compound **C4** (600 mg), compound **B12** (434 mg), tetrakis(triphenylphosphine)palladium (208 mg), and sodium carbonate (730 mg) were stirred in a mixed solution of dioxane (20 mL) and water (4 mL), and reacted at 110 °C under nitrogen atmosphere. After the reaction was completed, the reaction solution was filtered under vacuum, concentrated under reduced pressure, and purified by column chromatography to give compound **C12** (110 mg). LC-MS: m/z 678.35 $(M+Na)^+$.

(4) Preparation method for compound **12:**

Compound **C12** was dissolved in hydrochloric acid/methanol (10 mL). The reaction solution was stirred under reflux at 80 °C for 48 h. After the reaction was completed, the reaction solution was directly concentrated to dryness, and separated and purified by preparative liquid phase chromatography to give compound **12** (49 mg).
LC-MS: m/z 472.22 $(M+H)^+$.
$^1H$ NMR (500 MHz, DMSO-d6) δ 12.22 (s, 1H), 8.28 (s, 2H), 8.08 (s, 1H), 7.89 (d, J = 5.9 Hz, 1H), 7.31 (s, 2H), 6.94 (dd, J = 17.7, 11.2 Hz, 1H), 6.28 (d, J = 17.7 Hz, 1H), 5.76 (d, J = 11.3 Hz, 1H), 4.25-4.17 (m, 1H), 3.81 (s, 2H), 3.72 (s, 3H), 0.95-0.91 (m, 2H), 0.84-0.79 (m, 2H).

**Example 13: Preparation of Compound 13**

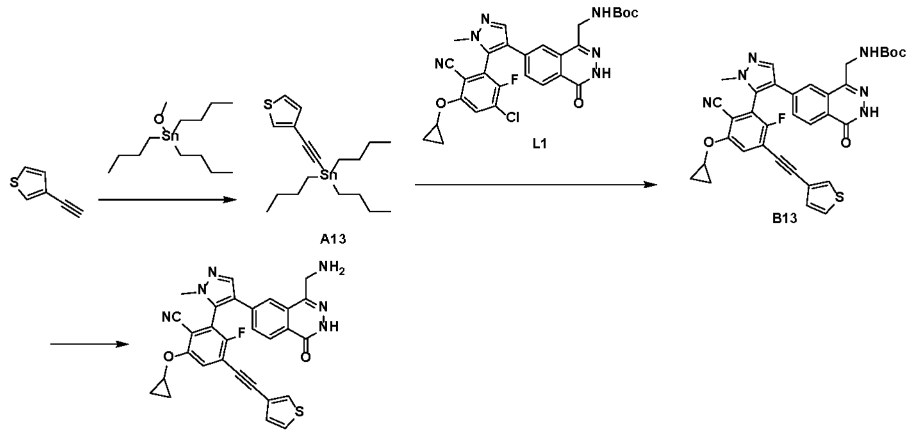

L1

A13

B13

13

### (1) Preparation method for compound A13:

3-Ethynyl thiophene(2700 mg), tri-n-butyltin methoxide (9640 mg), and zinc bromide (282 mg) were dissolved in tetrahydrofuran (27 mL). The reaction solution was reacted at 20-30 °C for 27 h. After the reaction was completed, water (40 mL) was added, and the reaction solution was extracted with ethyl acetate. The organic phase was isolated, washed with saturated brine (60 mL), dried, and filtered under vacuum. The filtrate was concentrated to dryness at 45-55 °C to give intermediate **A13** (8.10 g).

### (2) Preparation method for compound B13:

Intermediate **L1** (248 mg), intermediate **A13** (2532 mg), tetrakis(triphenylphosphine)palladium (152.4 mg), and sodium carbonate (280 mg) were dissolved in a mixed solvent (14.9 mL) of 1,4-dioxane and water (v:v = 5:1). The reaction solution was reacted in a microwave reactor (130 °C, 100 W) for 1 h under nitrogen atmosphere. After the reaction was completed, the reaction solution was filtered under vacuum, and concentrated at 45-55 °C. The concentrate was completely dissolved with ethyl acetate (20 mL), and then washed with water and saturated brine. The organic phase was isolated, dried, and concentrated at 45-55 °C to give intermediate **B13** (280 mg). ESI-MS: m/z = 637.26[M+H]$^+$.

### (3) Preparation method for compound 13:

Intermediate **B13** (280 mg) and trifluoroacetic acid (1.2 mL) were dissolved in dichloromethane (15.2 mL). The reaction solution was reacted at 10-30 °C for about 3.5 h. After the reaction was completed, the filtrate was concentrated at 35-50 °C until no liquid flowed out. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (chromatographic column: YMC AQ C18, 50 × 250 mm, 10 μm; mobile phase: A: acetonitrile, B: 0.1% FA; elution gradient: 17-47A-60 min; λ = 254 nm, V = 60 mL/min) to give compound **13** (72 mg). ESI-MS: m/z = 537.17 [M+H]$^+$.

$^1$H NMR (500 MHz, DMSO-d6) δ 12.43 (s, 1H), 8.27 (s, 1H), 8.21-7.98 (m, 2H), 7.92 (d, J = 5.5 Hz, 1H), 7.82-7.61 (m, 3H), 7.34 (d, J = 5.0 Hz, 1H), 4.20 (dq, J = 6.0, 3.0 Hz, 1H), 3.79 (d, J = 4.8 Hz, 5H), 1.03-0.75 (m, 4H).

### Example 14: Preparation of Compound 14

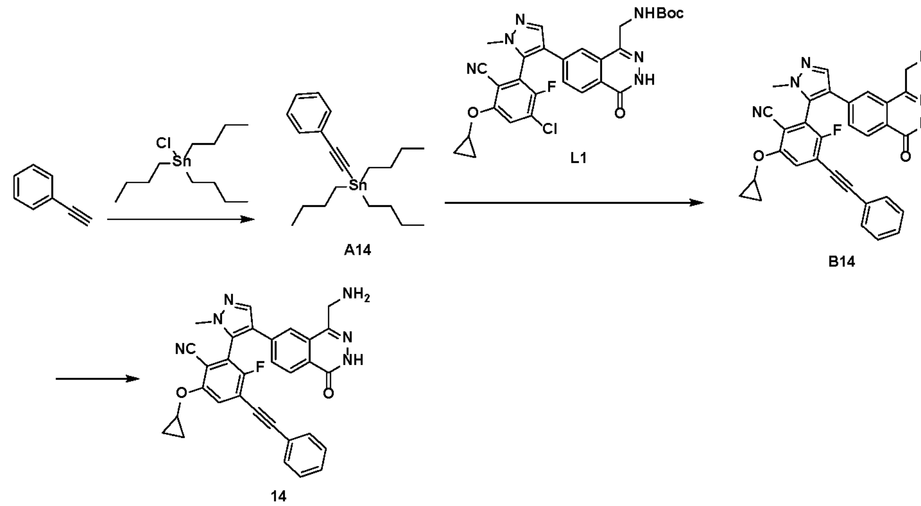

A14 L1 B14 14

**(1) Preparation method for compound A14:**

Phenylacetylene (2529 mg) was dissolved in tetrahydrofuran (46 mL). The reaction solution was purged with nitrogen, and then cooled to -78 °C. A solution of 1 N lithium (trimethylsilyl)amide in tetrahydrofuran (30.5 mL) was added dropwise. After the dropwise addition, the resulting mixture was stirred for 1 h. Tributyltin chloride (9946 mg) was added dropwise. After the dropwise addition, the resulting mixture was heated to 20-30 °C and reacted for 1.5 h. After the reaction was completed, the reaction was quenched with a saturated aqueous ammonium chloride solution (50 mL), and the reaction solution was extracted with ethyl acetate. The organic phase was isolated, washed with brine, dried, filtered under vacuum, and concentrated to dryness at 45-55 °C to give intermediate **A14** (9.80 g).

**(2) Preparation method for compound B14:**

Intermediate **L1** (282 mg), intermediate **A14** (2940 mg), tetrakis(triphenylphosphine)palladium (173.4 mg), and sodium carbonate (318 mg) were dissolved in a mixed solvent (14.1 mL) of 1,4-dioxane and water (v:v = 5:1). The reaction solution was reacted in a microwave reactor (130 °C, 100 W) for 1 h under nitrogen atmosphere. After the reaction was completed, the reaction solution was filtered under vacuum, and concentrated at 45-55 °C. The concentrate was completely dissolved with ethyl acetate (20 mL), and then washed with water and saturated brine. The organic phase was isolated, dried, and concentrated at 45-55 °C to give intermediate **B14** (350 mg). ESI-MS: m/z = 631.29[M+H]$^+$.

**(3) Preparation method for compound 14:**

Intermediate **B14** (350 mg) and trifluoroacetic acid (3.3 mL) were dissolved in dichloromethane (9.5 mL). The reaction solution was reacted at 10-30 °C for 5.0 h. After the reaction was completed, the filtrate was concentrated at 35-50 °C. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (chromatographic column: YMC AQ C18, 50 × 250 mm, 10 μm; mobile phase: A: acetonitrile, B: 0.1% FA; elution gradient: 15-45A-60 min; λ = 254 nm, V = 60 mL/min; desalting: chromatographic column: YMC, TA-C18, 30 × 250 mm, 10 μm, mobile phase: A: acetonitrile; B: 0.1% ammonium hydroxide, elution gradient: 5-5A-20 min-95-40 min; λ = 254 nm; V = 40 mL/min) to give compound **14** (32 mg). ESI-MS: m/z = 531.29[M+H]$^+$.

$^1$H NMR (500 MHz, DMSO-d6) δ 12.44 (s, 1H), 8.36-8.09 (m, 2H), 7.96 (d, J = 5.4 Hz, 1H), 7.84-7.57 (m, 4H), 7.49 (q, J = 8.1, 6.9 Hz, 3H), 4.21 (s, 1H), 3.80 (s, 5H), 0.98-0.78 (m, 4H).

**Example 15: Preparation of Compound 15**

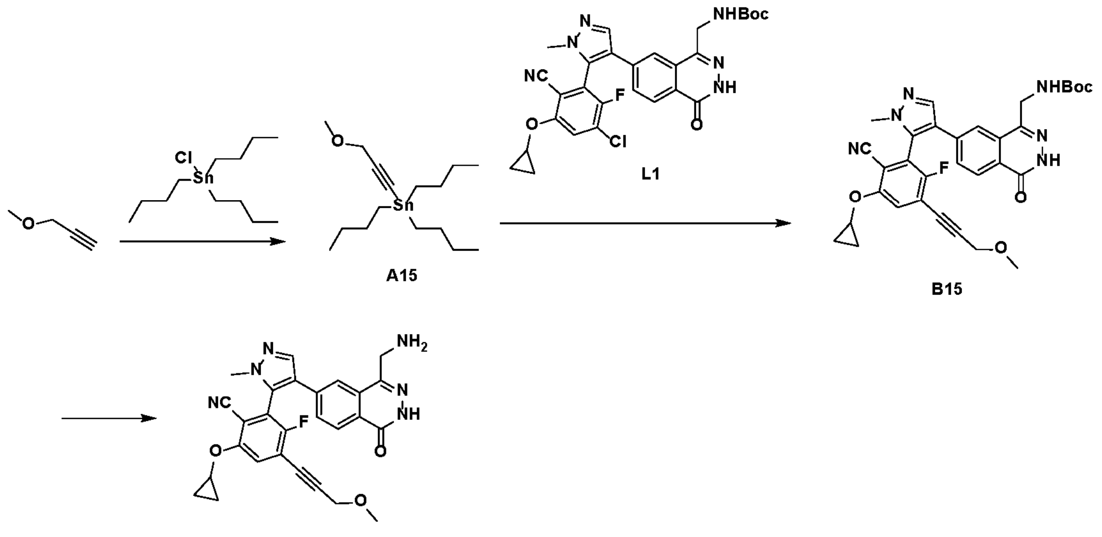

L1

A15

B15

15

**(1) Preparation method for compound A15:**

Methyl propinyl ether (1750 mg) was dissolved in tetrahydrofuran (53 mL). The reaction solution was purged with nitrogen, and then cooled to -78 °C. A solution of 2.5 N n-butyllithium in hexane (12 mL) was added dropwise. After the dropwise addition, the resulting mixture was stirred for 0.5 h. Tributyltin chloride (9790 mg) was added dropwise. After the dropwise addition, the resulting mixture was heated to 20-30 °C and reacted for 4 h. After the reaction was completed, the reaction was quenched with a saturated aqueous ammonium chloride solution (50 mL), and the reaction solution was extracted with ethyl acetate. The organic phase was isolated, washed with brine, dried, filtered under vacuum, and concentrated to dryness at 45-55 °C to give intermediate **A15** (9.00 g).

**(2) Preparation method for compound B15:**

Intermediate **L1** (225.7 mg), intermediate **A15** (2232 mg), tetrakis(triphenylphosphine)palladium (138.6 mg), and sodium carbonate (254.4 mg) were dissolved in a mixed solvent (34 mL) of 1,4-dioxane and water (v:v = 5:1). The reaction solution was reacted at 85-90 °C for 12 h under nitrogen atmosphere. After the reaction was completed, the reaction solution was filtered under vacuum, and concentrated at 45-55 °C. The concentrate was completely dissolved with ethyl acetate (20 mL), and then washed with water and saturated brine. The organic phase was isolated, dried, and concentrated at 45-55 °C to give intermediate **B15** (252 mg). ESI-MS: m/z = 599.30[M+H]$^+$.

**(3) Preparation method for compound 15:**

Intermediate **B15** (252 mg) and trifluoroacetic acid (2.4 mL) were dissolved in dichloromethane (6.8 mL). The reaction solution was reacted at 10-30 °C for 2 h. After the reaction was completed, the filtrate was concentrated at 35-50 °C. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (chromatographic column: YMC TA C18, 50 × 250 mm, 10 μm; mobile phase: A: acetonitrile, B: 10 mM ammonium acetate; elution gradient: 20-45A-60 min; λ = 254 nm, V = 60 mL/min, desalting with a 0.05% aqueous acetic acid solution, drying and concentration) to give compound 15 (10 mg). ESI-MS: m/z = 499.22[M+H]$^+$.

$^1$H NMR (500 MHz, DMSO-d6) δ 12.43 (s, 1H), 8.25 (s, 1H), 8.13 (dd, J = 8.5, 5.0 Hz, 1H), 7.79-7.58 (m, 2H), 4.42 (s, 1H), 4.16 (ddt, J = 10.4, 7.2, 3.7 Hz, 2H), 3.91-3.55 (m, 7H), 1.23 (d, J = 5.6 Hz, 2H), 0.91-0.83 (m, 2H).

**Example 16: Preparation of Compound 16**

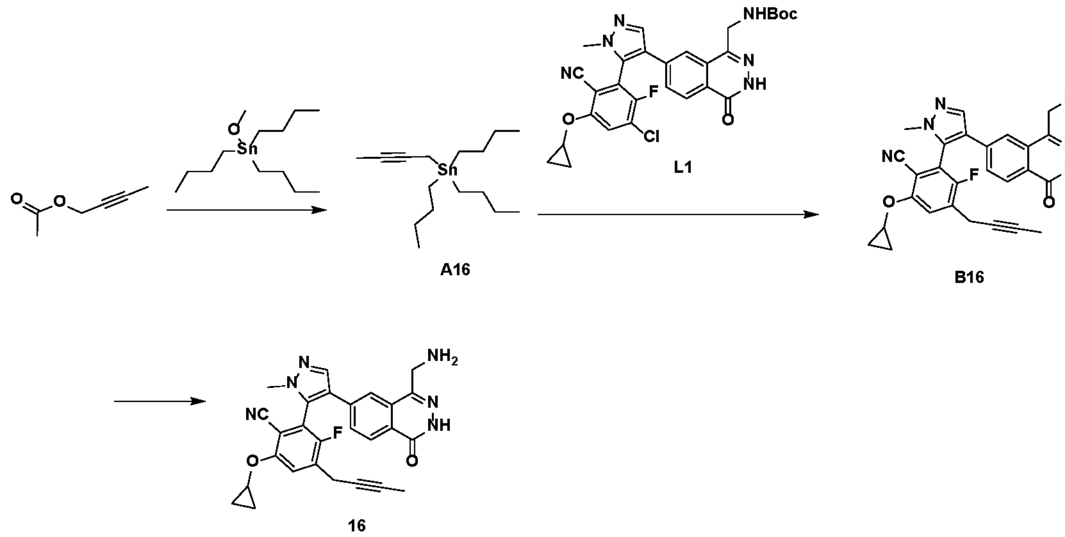

L1 A16 B16 16

**(1) Preparation method for compound A16:**

A manganese powder (550 mg), nickel bromide (110 mg), di-*tert*-butylpyridine 2.2.2 2,6-di-*tert*-butylpyridine (269 mg), and *N,N*-dimethylformamide (15 mL) were mixed. The reaction solution was purged with nitrogen, and then trimethylchlorosilane (109 mg) was added. The resulting mixture was stirred at 20-30 °C for 20 min. The reaction solution was heated to 80 °C and stirred for 3 min. The reaction solution was cooled to 20 °C, and then tributyltin chloride (1920 mg) was added. After the addition, the resulting mixture was reacted at 20-30 °C for 5 h. After the reaction was completed, the reaction solution was diluted with ethyl acetate (25 mL), and the reaction was quenched with water (25 mL). The aqueous phase was extracted with ethyl acetate (30 mL). The organic phase was isolated, washed with brine (30 mL), dried, and filtered under vacuum. The filtrate was concentrated to dryness at 45-55 °C to give intermediate **A16** (1720 mg).

**(2) Preparation method for compound B16:**

Intermediate **L1** (226 mg), intermediate **A16** (1600 mg), tetrakis(triphenylphosphine)palladium (144 mg), and sodium carbonate (255 mg) were dissolved in a mixed solvent (17 mL) of 1,4-dioxane and water (v:v = 5:1). The reaction solution was heated to 85-90 °C and reacted for 5 h under nitrogen atmosphere. After the reaction was completed, the reaction solution was filtered under vacuum, and concentrated at 45-55 °C. The concentrate was completely dissolved with ethyl acetate (20 mL), and then washed with water and saturated brine. The organic phase was isolated, dried, and filtered under vacuum. The filtrate was purified by silica gel column chromatography to give intermediate **B16** (256 mg). ESI-MS: m/z = 583.32$[M+H]^+$.

**(3) Preparation method for compound 16:**

Intermediate **B16** (256 mg) and trifluoroacetic acid (2.5 mL) were dissolved in dichloromethane (15 mL). The reaction solution was reacted at 10-30 °C for 2 h. After the reaction was completed, the filtrate was concentrated at 35-50 °C. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: YMC*GEL ODS-AQ-HG, specification: 50 × 250, 10 μm, mobile phase: A: 0.1% formic acid/water, B: acetonitrile, gradient: 18% B-68% B-40 min-90% B-60 min; second run: YMC-Triart Prep C18-S, specification: 30 × 250, 10 μm, mobile phase: A: 0.1% ammonium hydroxide, B: acetonitrile, gradient: 10% B-70% B-70 min) to give compound **16** (15 mg). ESI-MS: m/z = 483.32$[M+H]^+$.

$^1$H NMR (500 MHz, DMSO-d6) δ 12.44 (s, 1H), 8.30-8.13 (m, 2H), 8.05-7.83 (m, 1H), 7.79-7.40 (m, 3H), 5.19 (dd, J = 7.4, 3.7 Hz, 1H), 4.33-4.08 (m, 2H), 3.87-3.70 (m, 5H), 2.27-2.09 (m, 3H), 0.93-0.79 (m, 4H).

**Example 17: Preparation of Compound 17**

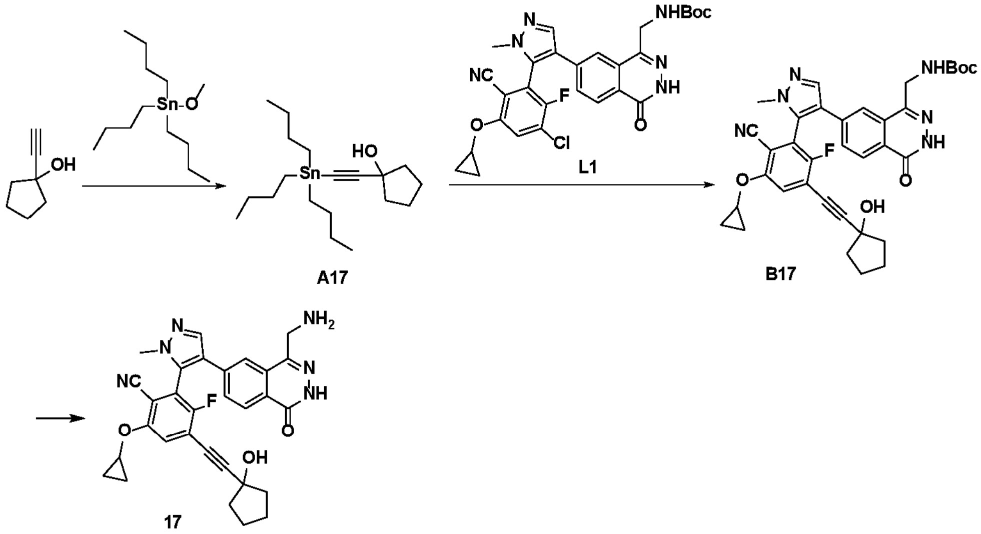
L1
A17
B17
17

### (1) Preparation method for compound A17:

1-Ethynylcyclopentan-1-ol (1.1 g) and tributyl(methoxy)stannane (12 mL) were reacted at 120 °C under nitrogen atmosphere. After the reaction was completed, the reaction solution was cooled to room temperature, and then purified by column chromatography (petroleum ether/ethyl acetate) to give compound **A17.**

### (2) Preparation method for compound B17:

Compound **L1** (226 mg), compound **A17** (479 mg), tetrakis(triphenylphosphine)palladium (138 mg), and sodium carbonate (512 mg) were dissolved in dioxane-water (5:1, v/v) (17 mL). The reaction solution was purged with nitrogen for 3 min, and reacted at 130 °C under microwave at 120 W for 1 h. After the reaction was completed, the reaction solution was cooled to room temperature and filtered. The filtrate was concentrated under reduced pressure. Water was added to the resulting concentrate, and the resulting mixture was extracted with EA. The organic phase was isolated, washed with water and saturated brine, dried, and concentrated to dryness at 40-50 °C to give compound B17. ESI-MS: m/z = 639.38 $[M+H]^+$.

### (5) Preparation method for compound 17:

Compound **B17** (300 mg), trifluoroacetic acid (2 mL), and dichloromethane (7 mL) were reacted at room temperature. After the reaction was completed, the reaction solution was concentrated under reduced pressure, and separated and purified by preparative liquid phase chromatography (liquid phase chromatography conditions: column: YMC*GEL ODS-AQ-HG 50 × 250 mm, 10 μm; mobile phase: A: 0.1% formic acid/water, B: acetonitrile; gradient: 10% B-70% B (0-60 min); flow rate: 60 mL/min; wavelength: 254 nm; retention time: 31.28 min) to give compound **17.** ESI-MS: m/z = 539.29 $[M+H]^+$.

$^1$H NMR (500MHz DMSO-$d_6$) δH 12.89 (s, 1H), 8.32 (s, 1H), 8.29 (s, 1H), 8.16 (d, J = 8.4 Hz, 1H), 7.84 (d, J = 1.2 Hz, 1H), 7.75 (d, J = 5.6 Hz, 1H), 7.51-7.47 (m, 1H), 4.28-4.20 (m, 2H), 4.20-4.15 (1H, m), 3.77 (s, 3H), 1.99-1.85 (m, 4H), 1.80-1.64 (m, 4H), 0.95-0.89 (m, 2H), 0.85-0.77 (m, 2H).

### Example 18: Preparation of Compound 18

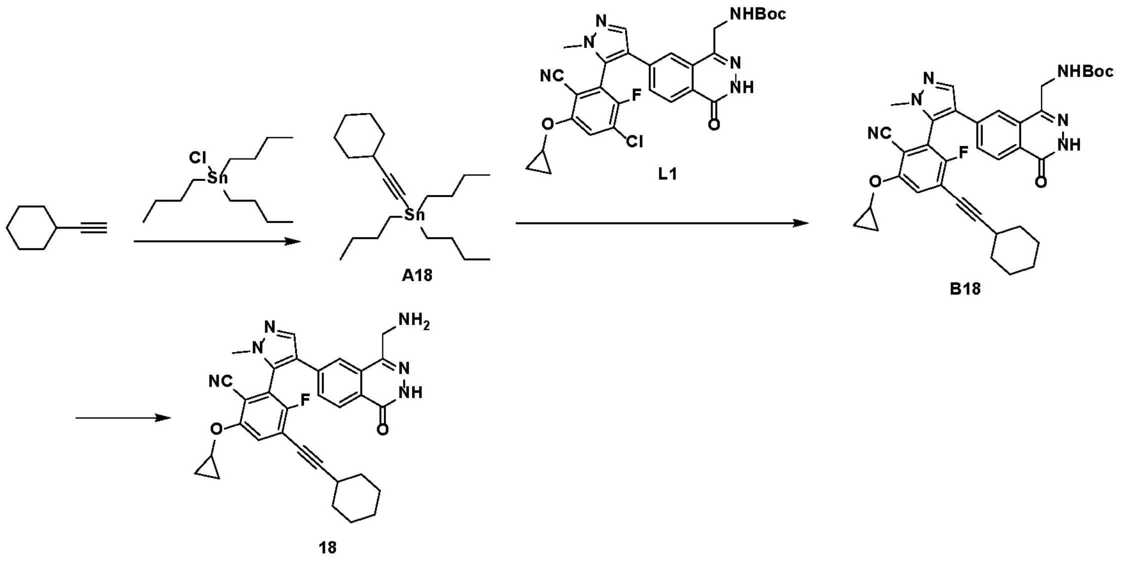

L1 A18 B18 18

### (1) Preparation method for compound A18:

Cyclohexyl ethynyl ether (1634 mg) was dissolved in tetrahydrofuran (46 mL). The reaction solution was purged with nitrogen, and then cooled to -78 °C. A solution of 2.5 N *n*-butyllithium in hexane (6.6 mL) was added dropwise. After the dropwise addition, the resulting mixture was stirred for 0.5 h. Tributyltin chloride (5136 mg) was added dropwise. After the dropwise addition, the resulting mixture was reacted for 4 h.

After the reaction was completed, the reaction was quenched with a saturated aqueous ammonium chloride solution (50 mL), and the reaction solution was extracted with ethyl acetate. The organic phase was isolated, washed with brine, dried, and filtered under vacuum. The filtrate was concentrated to dryness at 45-55 °C to give intermediate **A18** (5.35 g).

### (2) Preparation method for compound B18:

Intermediate **L1** (226 mg), intermediate **A18** (956 mg), tetrakis(triphenylphosphine)palladium (138 mg), and sodium carbonate (255 mg) were dissolved in a mixed solvent (17 mL) of 1,4-dioxane and water (v:v = 5:1). The reaction solution was reacted in a microwave reactor (130 °C, 100 W) for 1 h under nitrogen atmosphere. After the reaction was completed, the reaction solution was filtered under vacuum, and concentrated at 45-55 °C. The concentrate was completely dissolved with ethyl acetate (20 mL), and then washed with water and saturated brine. The organic phase was isolated, dried, and concentrated at 45-55 °C to give intermediate **B18** (267 mg). ESI-MS: m/z = 637.35$[M+H]^+$.

### (3) Preparation method for compound 18:

Intermediate **B18** (267 mg) and trifluoroacetic acid (1.6 mL) were dissolved in dichloromethane (7.2 mL). The reaction solution was reacted at 10-30 °C for 2 h. After the reaction was completed, the filtrate was concentrated at 35-50 °C. Dichloromethane (15 mL) was added to the concentrate, and the resulting mixture was concentrated to dryness by rotary evaporation. This process was repeated for 4 times in total (with rotary evaporation under high vacuum for above 30 min each time). The residue was purified by preparative liquid phase chromatography (chromatographic column: YMC-AQ-C18, 10 μm, 30 × 250 mm, mobile phase: A: acetonitrile, B: 10 mM ammonium acetate (prepared by adding 7.7 g of ammonium acetate to 10 L of water); elution gradient: 40-60B-90 min; λ = 254 nm, V = 60 mL/min) to give compound **18** (59 mg). ESI-MS: m/z = 537.34$[M+H]^+$.

$^1$H NMR (500 MHz, DMSO-$d_6$) δ 12.45 (s, 1H), 8.27 (s, 1H), 8.16 (d, J = 8.3 Hz, 1H), 7.77-7.48 (m, 4H), 7.43-7.35 (m, 1H), 4.19 (tt, J = 6.1, 3.0 Hz, 1H), 3.78 (d, J = 13.1 Hz, 5H), 2.78 (tt, J = 8.8, 3.9 Hz, 1H), 1.84-1.18 (m, 10H), 0.94-0.76 (m, 4H).

**Example 19:**

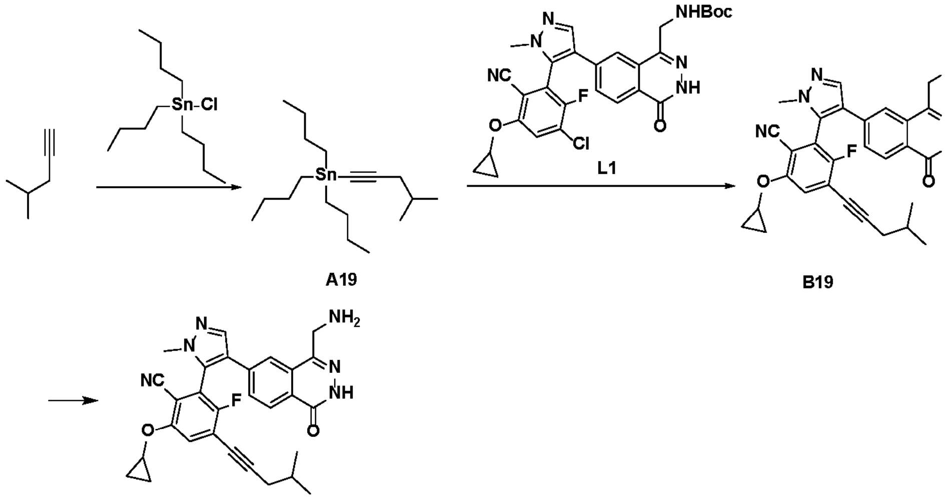


A19

B19

19

**(1) Preparation method for compound A19:**

*n*-Butyllithium (2.5 M, 4 mL) was slowly added dropwise to 4-methyl-1-pentyne (820 mg) and tetrahydrofuran (100 mL) at -78 °C under nitrogen atmosphere. After the dropwise addition, the resulting mixture was reacted for 1 h. After the reaction was completed, tributyltin chloride (3.25 g) was slowly added dropwise. The reaction solution was heated to room temperature and reacted overnight. After the reaction was completed, the reaction was quenched with a saturated ammonium chloride solution in an ice bath. Water was added, and the resulting mixture was extracted with n-hexane. The organic phase was isolated, washed with water and saturated brine, dried, and concentrated to dryness at 40-50 °C to give compound **A19.**

**(2) Preparation method for compound B19:**

Compound **L1** (226 mg), compound **A19** (479 mg), tetrakis(triphenylphosphine)palladium (138 mg), and sodium carbonate (512 mg) were dissolved in dioxane-water (5:1, v/v) (17 mL). The reaction solution was purged with nitrogen for 3 min, and reacted at 130 °C under microwave at 120 W for 1 h. After the reaction was completed, the reaction solution was cooled to room temperature and filtered. The filtrate was concentrated under reduced pressure. Water was added to the resulting concentrate, and the resulting mixture was extracted with EA. The organic phase was isolated, washed with water and saturated brine, dried, and concentrated to dryness at 40-50 °C to give compound **B19.** ESI-MS: m/z = 611.34 $[M+H]^+$.

**(5) Preparation method for compound 19:**

Compound **B19** (300 mg), trifluoroacetic acid (2 mL), and dichloromethane (7 mL) were reacted at room temperature. After the reaction was completed, the reaction solution was concentrated under reduced pressure, and separated and purified by preparative liquid phase chromatography (liquid phase chromatography conditions: column: TMC TA C18 30 × 250 mm, 5 μm; mobile phase: A: acetonitrile, B: 0.1% aqueous acetic acid solution; gradient: 80% B-15% B (0-40 min); flow rate: 40 mL/min; wavelength: 254 nm; retention time: 15.724 min) to give compound **19.** ESI-MS: m/z = 511.25 $[M+H]^+$.

$^1H$ NMR (500MHz DMSO-$d_6$) δH 12.89 (s, 1H), 8.40-8.35 (br, 3H), 8.33 (s, 1H), 8.15 (d, J = 8.3 Hz, 1H), 7.88 (d, J = 1.3 Hz, 1H), 7.76 (d, J = 5.7 Hz, 1H), 4.49-4.82 (m, 2H), 4.20-4.15 (m, 1H), 3.76 (s, 3H), 2.46 (d, J =6.5 Hz, 2H), 1.93-1.82 (m, 1H), 1.00 (d, J =6.5 Hz, 6H), 0.95-0.85 (m, 2H), 0.85-0.075 (m, 2H).

**Example 20: Preparation of Compound 20**

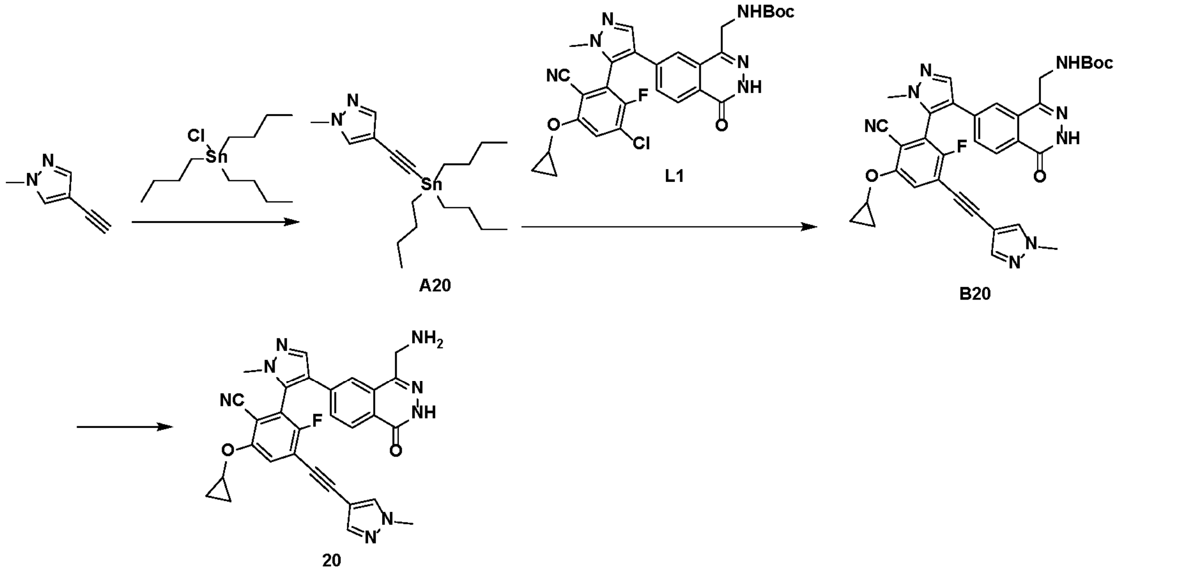

A20 L1 B20 20

**(1) Preparation method for compound A20:**

1-Methyl-1*H*-pyrazolylacetylene (1485 mg) was dissolved in tetrahydrofuran (40 mL). The reaction solution was purged with nitrogen, and then cooled to -78 °C. A solution of 2.5 N *n*-butyllithium in hexane (6.2 mL) was added dropwise. After the dropwise addition, the resulting mixture was stirred for 0.5 h. Tributyltin chloride (4792 mg) was added dropwise. After the dropwise addition, the resulting mixture was reacted for 4 h, then heated to 20-30 °C and reacted for 20 h.

After the reaction was completed, the reaction was quenched with a saturated aqueous ammonium chloride solution (50 mL), and the reaction solution was extracted with n-hexane. The organic phase was isolated, washed with brine (150 mL), dried, and filtered under vacuum. The filtrate was concentrated to dryness at 45-55 °C to give intermediate **A20** (5.55 g).

**(2) Preparation method for compound B20:**

Intermediate **L1** (226 mg), intermediate **A20** (1036 mg), tetrakis(triphenylphosphine)palladium (138 mg), and sodium carbonate (255 mg) were dissolved in a mixed solvent (17 mL) of 1,4-dioxane and water (v:v = 5:1). The reaction solution was reacted in a microwave reactor (130 °C, 100 W) for 1 h under nitrogen atmosphere. After the reaction was completed, the reaction solution was filtered under vacuum, and concentrated at 45-55 °C. The concentrate was completely dissolved with ethyl acetate (20 mL), and then washed with water and saturated brine. The organic phase was isolated, dried, and filtered under vacuum. The filtrate was purified by silica gel column chromatography to give intermediate **B20** (278 mg). ESI-MS: m/z = 635.37[M+H]$^+$.

**(3) Preparation method for compound 20:**

Intermediate **B20** (278 mg) and trifluoroacetic acid (3.2 mL) were dissolved in dichloromethane (7.5 mL). The reaction solution was reacted at 10-30 °C for 5 h. After the reaction was completed, the filtrate was concentrated at 35-50 °C. Dichloromethane (15 mL) was added, and the resulting mixture was concentrated to dryness by rotary evaporation. This process was repeated for 4 times in total (with rotary evaporation under high vacuum for above 30 min each time). The residue was purified by preparative liquid phase chromatography (liquid phase chromatography conditions: column: YMC*GEL ODS-AQ-HG, specification: 50 × 250, 10 μm; mobile phase: A: 0.1% formic acid/water, B: acetonitrile; gradient: 5% B-65% B (0-60 min); wavelength 254 nm, v = 60 mL/min) to give compound **20** (47 mg). ESI-MS: m/z = 535.26 [M+H]$^+$.

$^1$H NMR (500 MHz, DMSO-d6) δ 12.64 (s, 1H), 8.30 (s, 1H), 8.25 (s, 2H), 8.23-8.13 (m, 2H), 7.85 (d, J = 5.6 Hz, 1H), 7.79 (d, J = 1.8 Hz, 2H), 7.59 (dd, J = 8.4, 1.6 Hz, 1H), 4.05 (d, J = 2.4 Hz, 2H), 3.83 (d, J = 44.2 Hz, 7H), 0.97-0.77 (m, 4H).

**Example 21: Preparation of Compound 21**

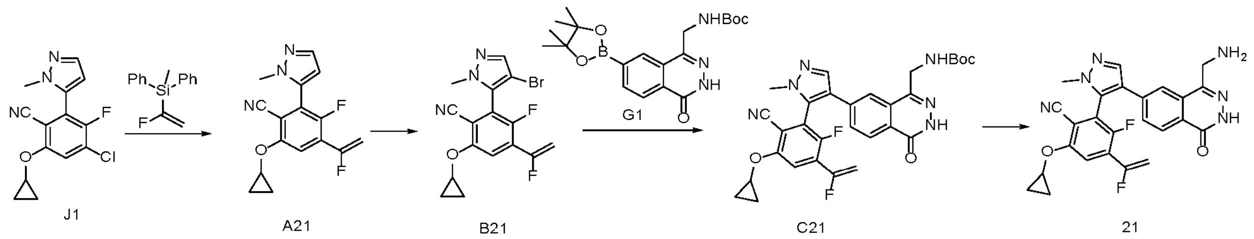


**(1) Preparation method for compound A21:**

Compound **J1** (650 mg), (1-fluorovinyl)methyldiphenylsilane (1 g), bis(triphenylphosphine)palladium(II) dichloride (154 mg), copper(I) iodide (42 mg), cesium fluoride (1 g), and 1,3-dimethyl-2-imidazolidinone (80 mL) were reacted at 130 °C under nitrogen atmosphere. After the reaction was completed, the reaction solution was cooled to room temperature. Water was added, and the resulting mixture was extracted with EA. The organic phase was isolated, washed with water and saturated brine, and concentrated to dryness at 40-50 °C to give compound **A21.** ESI-MS: m/z = 302.09 $[M+H]^+$.

**(2) Preparation method for compound B21:**

Compound **A21** (451 mg), N-bromosuccinimide (479 mg), and acetonitrile (40 mL) were reacted at room temperature. After the reaction was completed, water was added, and the resulting mixture was extracted with EA. The organic phase was isolated, washed with water and saturated brine, and concentrated to dryness at 40-50 °C to give compound **B21.** ESI-MS: m/z = 380.09 $[M+H]^+$.

**(3) Preparation method for compound C21:**

Compound **B21** (570 mg), compound **G1** (374 mg), mesylate[(di(1-adamantyl)-*n*-butylphosphine)-2-(2'-amino-1,1'-biphenyl)]palladium(II) (327 mg), and cesium fluoride (1.37 g) were dissolved in dioxane-water (5:1, v/v) (40 mL). The reaction solution was heated to 85 °C under nitrogen atmosphere and reacted. After the reaction was completed, the reaction solution was cooled to room temperature. Water was added, and the resulting mixture was extracted with EA. The organic phase was isolated, washed with water and saturated brine, and concentrated to dryness at 40-50 °C to give compound **C21.** ESI-MS: m/z = 575.29 $[M+H]^+$.

**(4) Preparation method for compound 21:**

Compound **C21** (1 g), trifluoroacetic acid (2 mL), and dichloromethane (10 mL) were reacted at room temperature. After the reaction was completed, the reaction solution was concentrated under reduced pressure, and separated and purified by preparative liquid phase chromatography (liquid phase chromatography conditions: column: TMC AQ C18 50 × 250 mm, 10 μm; mobile phase: A: 0.1% formic acidin water, B: acetonitrile; gradient: 10% B-80% B (0-60 min)) to give compound **21.** ESI-MS: m/z = 475.22 $[M+H]^+$.

$^1$H NMR (500MHz DMSO-$d_6$) δH 12.89 (s, 1H), 8.37 (s, 3H), 8.35 (s, 1H,), 8.15 (d, J = 8.3 Hz, 1H), 7.84 (d, J = 1.2 Hz, 1H), 7.81 (d, J = 6.0 Hz, 1H), 7.48 (dd, J = 8.3, 1.2 Hz, 1H), 5.69-5.60 (m, 1H), 4.50-4.28 (m, 2H), 4.30-4.20 (m, 1H), 3.78 (s, 3H), 0.99-0.85 (2H, m), 0.85-0.61 (m, 2H).

**Example 22: Preparation of Compound 22**

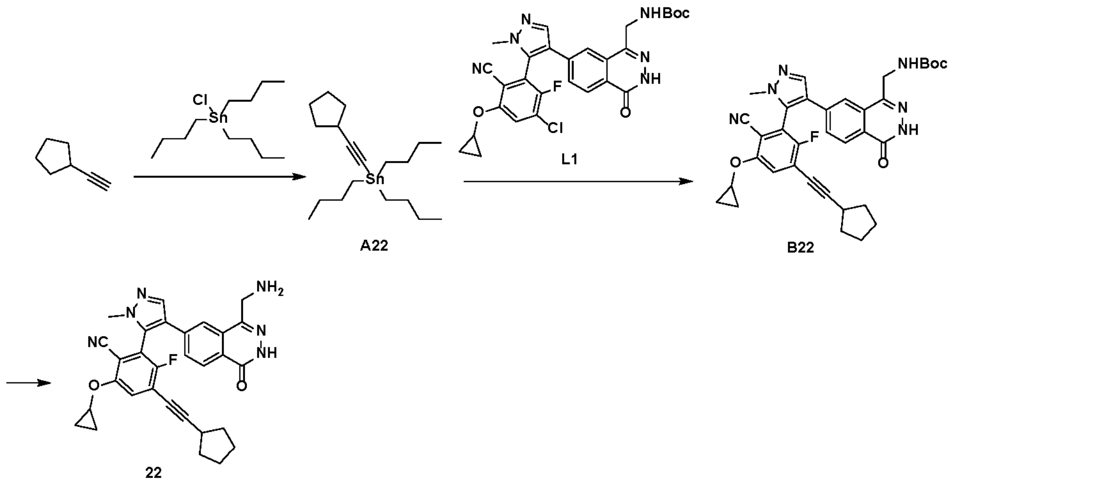

A22, L1, B22, 22

### (1) Preparation method for compound A22:

Referring to the reaction in step 1 of Example 8, 3,3-dimethyl-1-butyne was replaced with cyclopentylacetylene to give intermediate **A22** (2885 mg).

### (2) Preparation method for compound B22:

Referring to step 1 of Example 3, tributylpropynyltin was replaced with intermediate **A22** to give intermediate **B22** (250 mg). ESI-MS: m/z = 623.33 $[M+H]^+$.

### (3) Preparation method for compound 22:

Referring to step 2 of Example 3, compound **A3** was replaced with **B22** to give compound **22.** Preparative liquid phase chromatography purification conditions: column: Novasep C18 50 × 250 mm, 10 μm; A: formic acid, B: acetonitrile; gradient: 90% A-10%-20% B to 50% A-50% B (0-60 min); λ: 254 nm, V: 50 mL/min. ESI-MS: m/z = 523.28$[M+H]^+$.

$^1$H NMR (500 MHz, DMSO-$d_6$) δ 12.72 (s, 1H), 8.31 (s, 1H), 8.23 (s, 1H), 8.14 (d, *J* = 8.3 Hz, 1H), 7.82 (d, *J* = 1.7 Hz, 1H), 7.74 (d, *J* = 5.6 Hz, 1H), 7.50 (dd, *J* = 8.3, 1.7 Hz, 1H), 4.22-4.13 (m, 3H), 3.75 (s, 3H), 3.01-2.94 (m, 1H), 2.05-1.96 (m, 2H), 1.73-1.55 (m, 6H), 0.92-0.77 (m, 4H).

## Example 23: Preparation of Compound 23

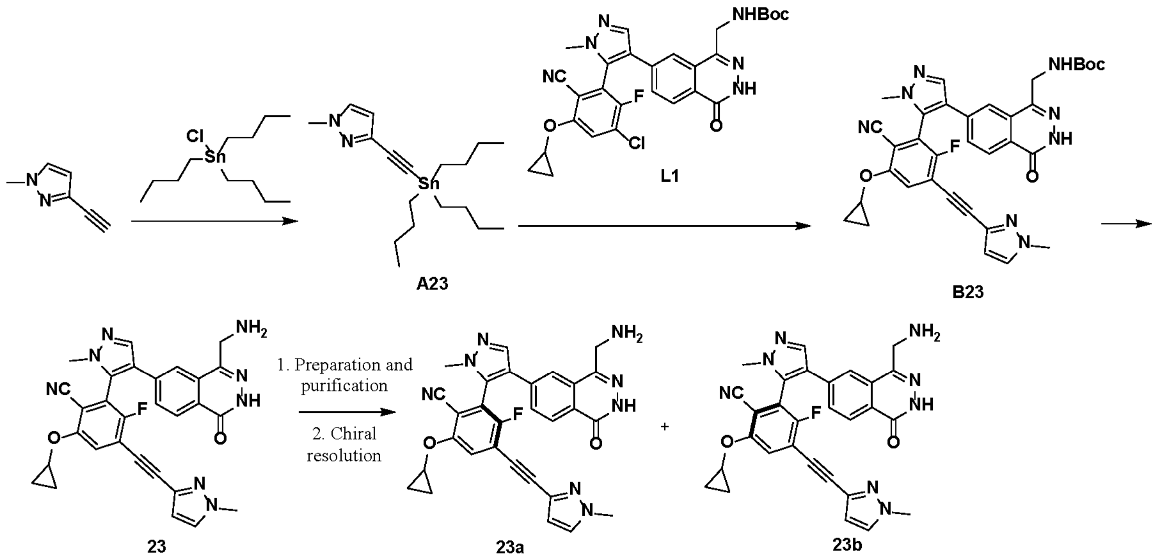


A23, L1, B23, 23, 23a + 23b

**(1) Preparation method for compound A23:**

1-Methyl-3-acetylene-1*H*-pyrazole (2493 mg) was dissolved in tetrahydrofuran (50 mL). The reaction solution was purged with nitrogen, and then cooled to -78 °C. Lithium bis(trimethylsilyl)amide (1 mol/L, 6.2 mL) was added dropwise. After the dropwise addition, the resulting mixture was stirred at the same temperature for 0.5 h. Tributyltin chloride (9349 mg) was added dropwise. After the dropwise addition, the resulting mixture was reacted at the same temperature for 4 h.

After the reaction was completed, the reaction was quenched with a saturated aqueous ammonium chloride solution (50 mL), and the reaction solution was extracted with n-hexane. The organic phase was isolated, washed with brine, dried, filtered under vacuum, rinsed, and concentrated to dryness at 45-55 °C to give intermediate **A23** (9320 mg).

**(2) Preparation method for compound B23:**

Referring to step 1 of Example 3, tributylpropynyltin was replaced with intermediate **A23** to give intermediate **B23** (780 mg). ESI-MS: m/z = 635.37[M+H]$^+$.

**(3) Preparation method for compound 23:**

Referring to step 2 of Example 3, compound **A3** was replaced with compound **B23** to give compound **23.** Preparative liquid phase chromatography purification conditions: column: YMC AQ C18, specification: 50 × 250 mm, 10 μm, mobile phase: A: 30 mM ammonium acetate, B: acetonitrile; gradient: 20%-80% B-60 min, wavelength: 254 nm, v = 60 mL/min. ESI-MS: m/z = 535.26[M+H]$^+$.

$^1$H NMR (500 MHz, DMSO-d6) δ 12.87 (s, 1H), 8.34 (s, 1H), 8.17 (d, J = 8.3 Hz, 1H), 7.93 (d, J = 5.5 Hz, 1H), 7.86 (t, J = 2.2 Hz, 2H), 7.55-7.47 (m, 1H), 6.64 (d, J = 2.4 Hz, 1H), 4.37 (q, J = 16.1 Hz, 2H), 3.90 (s, 3H), 3.81 (s, 3H), 0.94 (td, J = 5.9, 2.8 Hz, 2H), 0.88-0.79 (m, 2H).

**(4) Preparation method for compounds 23a and 23b:**

Compound **23** was subjected to chiral resolution (liquid phase chromatography conditions (resolution): column: CHIRALPAK IG, specification: 20 × 250, 5 μm, mobile phase: A: *n*-hexane, B: ethanol; gradient: 10% B-70% B (0-60 min), wavelength: 254 nm, v = 10 mL/min) to give compounds 23a and 23b. 23a and 23b: ESI-MS: m/z = 535.26[M+H]$^+$.

**Example 24: Preparation of Compound 24**

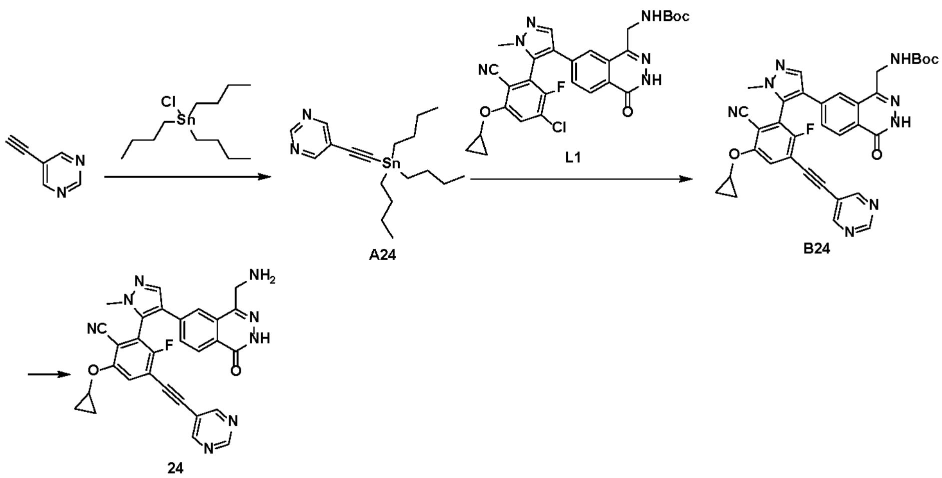


**(1) Preparation method for compound A24:**

Referring to step 1 of Example 22, cyclopentylacetylene was replaced with 5-alkynylpyrimidine to give intermediate **A24** (5910 mg).

**(2) Preparation method for compound B24:**

Referring to step 1 of Example 3, tributylpropynyltin was replaced with intermediate **A24** to give intermediate **B24** (300 mg). ESI-MS: m/z = 633.29 $[M+H]^+$.

**(3) Preparation method for compound 24:**

Referring to step 2 of Example 3, compound **A3** was replaced with compound **B24** to give compound **24.** Preparative liquid phase chromatography purification (liquid phase chromatography conditions: column: YMC-Triart Prep C18-S 50 × 250 mm, 10 μm; A: 0.1% ammonium hydroxide, B: acetonitrile; gradient: 90% A-10% B to 10%A-70% B (0-60 min); λ: 254 nm, V: 50 mL/min, rt 40 min). ESI-MS: m/z = 533.28$[M+H]^+$.

$^1H$ NMR (500 MHz, DMSO-d6) δ 12.45 (s, 1H), 9.28 (s, 1H), 9.11 (s, 2H), 8.28 (s, 1H), 8.17 (d, J = 8.4 Hz, 1H), 8.03 (d, J = 5.4 Hz, 1H), 7.81-7.67 (m, 2H), 4.21 (s, 1H), 3.80 (s, 4H), 0.95-0.83 (m, 4H).

**Example 25: Preparation of Compound 25**

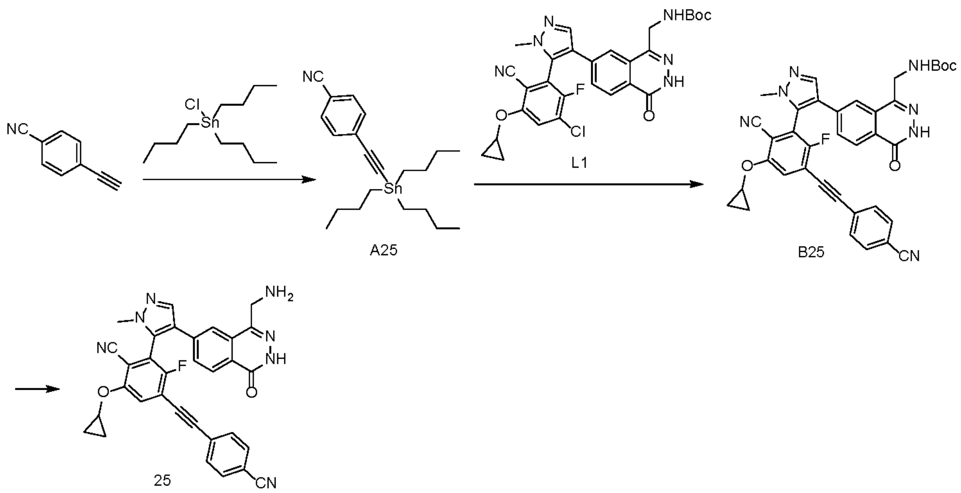

**(1) Preparation method for compound A25:**

Referring to step 1 of Example 22, cyclopentylacetylene was replaced with 4-ethynylbenzonitrile to give intermediate **A25** (5668 mg).

**(2) Preparation method for compound B25:**

Referring to step 1 of Example 3, tributylpropynyltin was replaced with intermediate **A25** to give intermediate **B25.** ESI-MS: m/z = 656.31 $[M+H]^+$.

**(3) Preparation method for compound 25:**

Referring to step 2 of Example 3, compound **A3** was replaced with compound **B25** to give compound **25.** Preparative liquid phase chromatography purification conditions: column: YMC-Triart Prep C18-S 30 × 250 mm, 10 μm; A: 0.1% formic acid, B: acetonitrile; gradient: 90% A-10% B to 20% A-80% B (0-60 min); λ: 254 nm, V: 30 mL/min. ESI-MS: m/z = 556.31 $[M+H]^+$.

$^1H$ NMR (500 MHz, DMSO-$d_6$) δ 12.89 (s, 1H), 8.40 (s, 3H), 8.35 (s, 1H), 8.15 (d, *J* = 8.3 Hz, 1H), 8.02 (d, *J* = 5.5 Hz, 1H), 7.98-7.94 (m, 2H), 7.87 (d, *J* = 1.7 Hz, 1H), 7.84-7.80 (m, 2H), 7.47 (dd, *J* = 8.3, 1.7 Hz, 1H), 4.45-4.35 (m, 2H), 4.20 (tt, *J* = 6.1, 2.9 Hz, 1H), 3.79 (s, 3H), 0.96-0.91 (m, 2H), 0.87-0.81 (m, 2H).

**Example 26: Preparation of Compound 26**

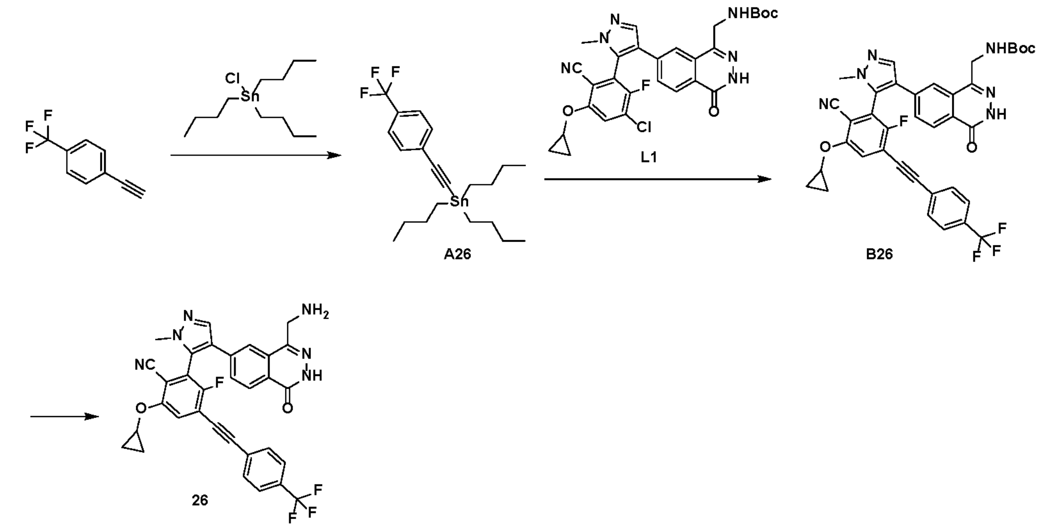
L1
A26
B26
26

**(1) Preparation method for compound A26:**

Referring to step 1 of Example 22, cyclopentylacetylene was replaced with *p*-trifluoromethylphenylacetylene to give intermediate **A26** (8.3 g).

**(2) Preparation method for compound B26:**

Referring to step 1 of Example 3, tributylpropynyl was replaced with intermediate **A26** and sodium carbonate was replaced with cesium fluoride to give intermediate **B26** (623 mg). ESI-MS: m/z = 699.34[M+H]$^+$.

**(3) Preparation method for compound 26:**

Compound **B26** (623 mg) was dissolved in a solution of hydrogen chloride in ethyl acetate (4 M, 10 mL). The reaction solution was reacted at room temperature for 2 h. After the reaction was completed, the filtrate was concentrated at 35-50 °C until no liquid flowed out. The residue was purified by preparative liquid phase chromatography (liquid phase chromatography conditions: column: YMC AQ C18 30 × 250 mm, 10 μm; A: 20 mM ammonium acetate, B: acetonitrile; gradient: 15% B-60% B (0-70 min); desalting: A: 0.1% acetic acid, B: acetonitrile; gradient: 15% B-60% B (0-70 min); λ: 254 nm, V: 40 mL/min) to give compound **26** (54 mg). ESI-MS: m/z = 599.32[M+H]$^+$.

$^1$H NMR (500 MHz, DMSO-d6) δ 12.47 (s, 1H), 8.29 (s, 1H), 8.17 (d, *J* = 8.2 Hz, 1H), 8.03 (d, *J* = 5.5 Hz, 1H), 7.87 (t, *J* = 6.0 Hz, 4H), 7.77 (d, *J* = 1.7 Hz, 1H), 7.73-7.68 (m, 1H), 4.26-4.16 (m, 1H), 3.82 (s, 3H), 3.80 (s, 2H), 0.94 (dt, *J* = 6.3, 3.6 Hz, 2H), 0.84 (dt, *J* = 5.8, 3.2 Hz, 2H).

**Example 27: Preparation of Compound 27**

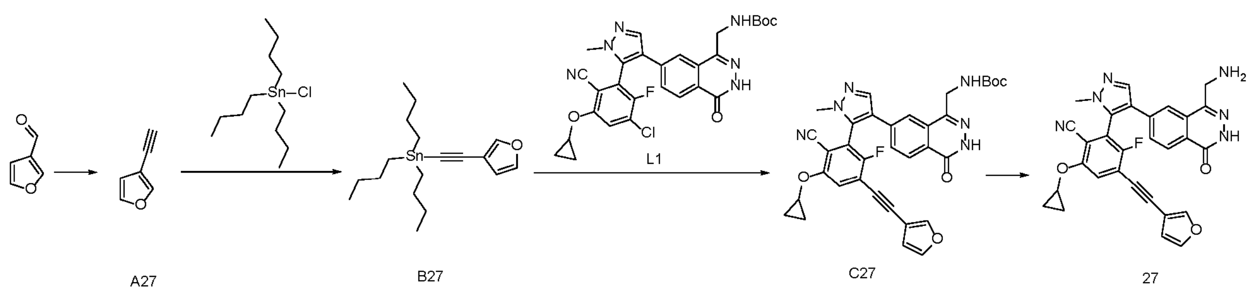
L1
A27
B27
C27
27

**(1) Preparation method for compound A27:**

Potassium bis(trimethylsilyl)amide (1 M, 11 mL) was slowly added dropwise to trimethylsilyldiazomethane (2 M, 6 mL) and tetrahydrofuran (80 mL) at -78 °C under nitrogen atmosphere. After the dropwise addition, the resulting mixture

was reacted at the same temperature for 30 min. After the reaction was completed, a solution of 3-furfural (1 g) in tetrahydrofuran (20 mL) was slowly added dropwise. The resulting mixture was heated and stirred in an ice bath. After the reaction was completed, the reaction was quenched with a saturated ammonium chloride solution in an ice bath. Water was added, and the resulting mixture was extracted with ethyl acetate. The organic phase was isolated, washed with saturated brine, dried, and concentrated to dryness at 40-50 °C to give compound **A27.**

**(2) Preparation method for compound B27:**

Referring to step 1 of Example 23, 1-methyl-3-ethynyl-1*H*-pyrazole was replaced with compound **A27** to give intermediate **B27.**

**(2) Preparation method for compound C27:**

Referring to step 1 of Example 3, tributylpropynyltin was replaced with compound **B27** to give intermediate **C27.** ESI-MS: m/z = 621.33 $[M+H]^+$.

**(3) Preparation method for compound 27:**

Referring to step 2 of Example 3, compound **A3** was replaced with compound **C27** to give compound **27.** The residue was separated by preparative liquid phase chromatography (liquid phase chromatography conditions: chromatographic column: YMC TA C18 (30 × 250 mm, 10 μm); mobile phase: A: 0.05% ammonium hydroxide, B: acetonitrile; elution gradient: 0-5 min 15% B, 5-10 min 15%-40% B, 10-85 min 40% -65% B) to give compound **27.** ESI-MS: m/z = 521.30 $[M+H]^+$.

**Example 28: Preparation of Compound 28**

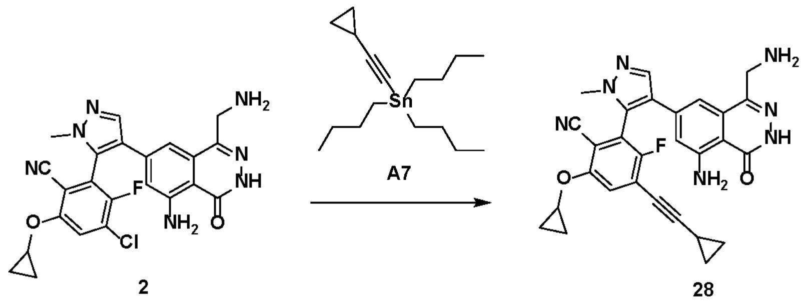


2 28

**(1) Preparation method for compound 28:**

Referring to step 1 of Example 3, compound **L1** was replaced with compound **2** and tributylpropynyltin was replaced with intermediate **A7** to give compound **28.** Preparative liquid phase chromatography purification conditions: Novasep C18 250 × 50 mm,10 μm chromatographic column; methanol-0.1% aqueous formic acid solution (gradient elution: 10%-50%-90%/0-60-100 min, 8 mg, Rt: 63 min). ESI-MS: m/z = 510.25 $[M+H]^+$.

$^1H$ NMR (500 MHz, DMSO-d6) δ 12.25 (s, 1H), 8.30 (s, 2H), 8.09 (s, 1H), 7.71 (d, J = 5.6 Hz, 1H), 6.63 (d, J = 41.5 Hz, 2H), 4.14 (tt, J = 6.2, 3.0 Hz, 2H), 3.79 (d, J = 85.2 Hz, 6H), 1.68 (tt, J = 8.4, 5.0 Hz, 1H), 0.98 (dq, J = 6.8, 3.9 Hz, 2H), 0.91-0.77 (m, 6H).

**Example 29: Preparation of Compound 29**

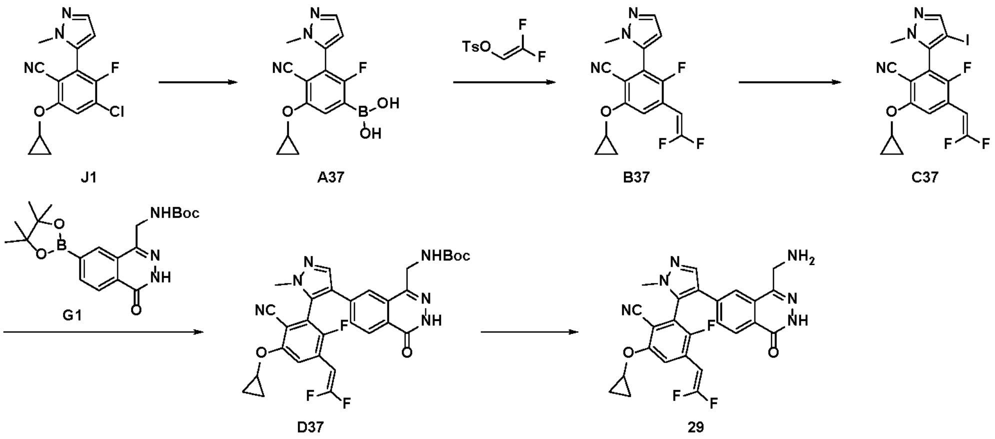

J1 A37 B37 C37 G1 D37 29

**(1) Preparation method for compound A37:**

Compound **J1** (2.9 g), bis(pinacolato)diboron (3.0 g), Pd(dppf)$Cl_2$ (2.1 g), and potassium acetate (2.9 g) were added to 1,4-dioxane (60 mL). The reaction solution was purged with nitrogen, and then reacted at 100 °C for 5 h. After the reaction was completed, the reaction solution was filtered under vacuum. The resulting filtrate was concentrated to dryness and subjected to column chromatography to give compound **A37** (3.1 g). ESI-MS: m/z = 302.08$[M+H]^+$.

**(2) Preparation method for compound B37:**

Intermediate **A37** (3.0 g), 2,2-difluorovinyl 4-methylbenzenesulfonate (3.5 g), Pd(dppf)$Cl_2$ (2.9 g), sodium iodide (3.0 g), and sodium carbonate (3.2 g) were added to a mixed solvent (120 mL) of tetrahydrofuran and water (5:1). The reaction solution was reacted at 70 °C for 2.5 h under nitrogen atmosphere.

After the reaction was completed, the reaction solution was filtered under vacuum, concentrated at 35-45 °C until no liquid flowed out, and purified by column chromatography to give compound **B37** (1.1 g). ESI-MS: m/z = 320.13$[M+H]^+$.

**(3) Preparation method for compound C37:**

Referring to step 2 of Example 4, compound **A4** was replaced with compound **B37** to give compound **C37** (830 mg). ESI-MS: m/z = 446.03$[M+H]^+$.

**(4) Preparation method for compound D37:**

Compound **C37** (670 mg), compound **G1** (780 mg), cataCXium A Pd G3 (109 mg), and cesium fluoride (1368 mg) were added to a mixed solvent (40 mL) of 1,4-dioxane and water (5:1). The reaction solution was reacted at 60 °C for 3 h under nitrogen atmosphere. After the reaction was completed, the reaction solution was filtered under vacuum, concentrated at 35-45 °C, and purified by column chromatography to give compound **D37** (1.1 g). ESI-MS: m/z = 593.32$[M+H]^+$.

**(5) Preparation method for Example 29:**

Compound **D37** (830 mg) was dissolved in a solution of hydrogen chloride in ethyl acetate (4 M, 15 mL). The reaction solution was reacted at room temperature for 2 h. After the reaction was completed, the filtrate was concentrated at 35-45 °C until no liquid flowed out. The residue was purified by preparative liquid phase chromatography (liquid phase chromatography conditions: column: YMC AQ C18, 50 × 250 mm, 10 μm; A: acetonitrile, B: 0.05% aqueous acetic acid solution; gradient: 15%A-45%A (0-60 min); λ: 254nm, V: 60 mL/min). Hydrochloric acid (2 M, 20 mL) was added to the resulting eluate, and the resulting mixture was concentrated to dryness to give compound **29.** ESI-MS: m/z = 493.24 $[M+H]^+$.

**Example 30: Preparation of Compound 30**

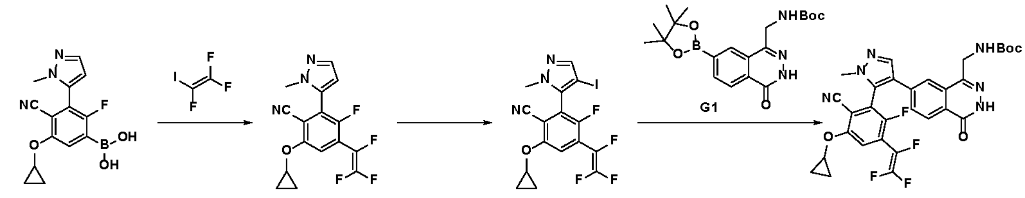


A37 A38 B38 C38

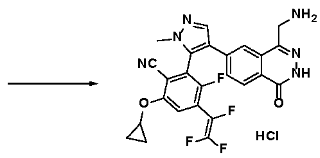


30

**(1) Preparation method for compound A38:**

Intermediate **A37** (3.0 g), iodotrifluoroethylene (3.2 g), Pd(dppf)$Cl_2$ (1.5 g), and sodium carbonate (2.6 g) were added to a mixed solvent (10 mL) of tetrahydrofuran and water (5:1). The reaction solution was reacted at 70 °C for 3.5 h under nitrogen atmosphere. After the reaction was completed, the reaction solution was filtered under vacuum, concentrated at 35-45 °C, and purified by column chromatography to give compound **A38** (1.6 g). ESI-MS: m/z = 338.14$[M+H]^+$.

**(2) Preparation method for compound B38:**

Referring to step 2 of Example 4, compound **A4** was replaced with compound **A38** to give compound **B38** (320 mg).

**(3) Preparation method for compound C38:**

Referring to step 4 of Example 29, compound **C37** was replaced with compound **B38** (463 mg) to give compound **C38** (50 mg). Preparative liquid phase chromatography purification conditions: column: YMC AQ C18, 50 × 250 mm, 10 μm; A: 0.05% aqueous acetic acid solution, B: acetonitrile; gradient: 35% B-70% B (0-60 min). λ: 254nm, V: 60 mL/min. ESI-MS: m/z = 611.40$[M+H]^+$.

**(5) Preparation method for Example 30:**

Compound **C38** (10 mg) was dissolved in a solution of hydrogen chloride in ethyl acetate (4 M, 2 mL). The reaction solution was reacted at room temperature for about 2 h. After the reaction was completed, the reaction solution was directly concentrated to dryness to give compound **30.** ESI-MS: m/z = 511.20$[M+H]^+$.

$^1$H NMR (500 MHz, DMSO-$d_6$) δ 12.88 (s, 1H), 8.60 (d, *J* = 5.9 Hz, 3H), 8.40 (s, 1H), 8.14 (d, *J* = 8.3 Hz, 1H), 7.93 (d, *J* = 5.4 Hz, 1H), 7.82 (d, *J* = 1.6 Hz, 1H), 7.50 (dd, *J* = 8.3, 1.6 Hz, 1H), 4.32 (dd, *J* = 5.9, 2.6 Hz, 2H), 4.27-4.20 (m, 1H), 3.79 (s, 3H), 0.95-0.90 (m, 2H), 0.88-0.78 (m, 2H).

**Test Example 1:** Thermal Stability of Protease *In Vitro*

1.1 Assay for thermal stability of hPRMT5 & hMEP50 (with MTA)

An hPRMT5 (2-637) & hMEP50 (2-342) protein solution (100 ng/μL) was added to assay wells at 16 μL/well. Different compounds dissolved in MTA and DMSO at a final concentration of 3 μM were added to assay wells using a nanoliter pipettor, such that the final concentrations of the compounds were 10 μM and 1 μM. The wells were set in duplicate, and a control well was set. The protein dye SYPRO Orange dye (manufacturer: sigma, 20×) was added to the assay wells at 4 μL/well. The assay was performed using the Roche LightCycler480 fluorescence quantitative PCR instrument, with a running system of 20 °C 15 s; 30-90 °C 0.02 °C/s; 20 °C 15 s. The melting temperature (Tm) was obtained by analysis using the LightCycler Thermal Shift Analysis software, and the melting temperature difference ΔTm was calculated.

1.2 Assay for thermal stability of hPRMT5 & hMEP50 (without MTA)

An hPRMT5 (2-637) & hMEP50 (2-342) protein solution (100 ng/μL) was added to assay wells at 16 μL/well. Different compounds dissolved in DMSO were added to assay wells using a nanoliter pipettor, such that the final concentrations of the compounds were 10 μM and 1 μM. The wells were set in duplicate, and a control well was set. The protein dye SYPRO Orange dye (manufacturer: sigma, 20×) was added to the assay wells at 4 μL/well. The assay was performed using the Roche LightCycler480 fluorescence quantitative PCR instrument, with a running system of 20 °C 15 s; 30-90 °C 0.02 °C/s; 20 °C 15 s. The melting temperature (Tm) was obtained by analysis using the LightCycler Thermal Shift Analysis software, and the melting temperature difference ΔTm was calculated.

$$\Delta Tm = Tm\ (\text{experimental group}) - Tm\ (\text{blank group})$$

The compounds of the present disclosure have good binding activity with proteins (hPRMT5 & hMEP50 proteins). For hPRMT5 & hMEP50 (with MTA), at a final concentration of 1 μM, the melting temperature (Tm) > 60 °C, and ΔTm > 5 °C, and preferably, ΔTm > 10 °C; at a final concentration of 10 μM, the melting temperature (Tm) > 60 °C, and ΔTm > 5 °C, and preferably, ΔTm > 10 °C.

**Test Example 2:** Inhibition of Cell Proliferation *In Vitro*

2.1 Assay for inhibitory activity for HCT116 WT cell proliferation

HCT116 WT cells in a good growth state were collected and added to a centrifuge tube, adjusted to a cell density of 1 × $10^4$ cells/mL, and seeded in a 96-well plate (100 μL/well). The cells were cultured overnight in a cell incubator. Compounds were added using a nanoliter pipettor, such that the final concentrations of the compounds were 2000 nM-0.91 nM. The wells were set in duplicate, and a control well was set. After another 7 days of culture in the cell incubator, the assay reagent CCK-8 (manufacturer: Dojindo Laboratories; 10 μL/well) was added. After 1.5 h of incubation in the cell incubator, absorbance values were detected at 450 nm using an Envision microplate reader. The four-parameter analysis was performed, the dose-response curves were fit, and $IC_{50}$ was calculated.

2.2 Assay for inhibitory activity for HCT116 MTAP-/- cell proliferation

HCT116 MTAP-/-cells in a good growth state were collected and added to a centrifuge tube, adjusted to a cell density of 0.25 × $10^4$ cells/mL, and seeded in a 96-well plate (100 μL/well). The cells were cultured overnight in a cell incubator. Compounds were added using a nanoliter pipettor, such that the final concentrations of the compounds were 2000 nM-0.91 nM. The wells were set in duplicate, and a control well was set. After another 7 days of culture in the cell incubator, the assay reagent CCK-8 (manufacturer: Dojindo Laboratories; 10 μL/well) was added. After 1.5 h of incubation in the cell incubator, absorbance values were detected at 450 nm using an Envision microplate reader. The four-parameter analysis was performed, the dose-response curves were fit, and $IC_{50}$ was calculated.

The test results show that the compounds of the present disclosure have proliferation inhibitory activity against HCT116 MTAP-/- cells, with an $IC_{50}$ of < 15 nM; compared with HCT116 WT, the compounds of the present disclosure have selective inhibitory activity against HCT116 MTAP-/- cells.

**Test Example 3:** Evaluation on *In Vitro* Stability in Liver Microsomes

Liver microsome incubation samples were prepared by mixing a PBS buffer (pH 7.4), a liver microsome solution (0.5 mg/mL; species: human, rat, and mouse), a test compound, and an NADPH+ $MgCl_2$ solution and incubated at 37 °C and 300 rpm for 1 h. Zero-hour samples were prepared by mixing a PBS buffer (pH 7.4), a liver microsome solution (0.5 mg/mL), and a test compound. An acetonitrile solution was added to the samples. Supernatants were prepared by protein precipitation, diluted, and then assayed by LC/MS/MS. The test results show that the compounds of the present application are stable in the metabolism of liver microsomes.

**Test Example 4:** Evaluation on *In Vivo* Drug Metabolism

4.1 Mouse pharmacokinetics

ICR mice weighing 18-22 g were randomly divided into groups of 9 mice each after 3-5 days of acclimatization, and then subjected to intragastric administration.

Plasma samples to be tested were prepared by taking blood from the orbit at time points of 15 min, 30 min, 1 h, 2 h, 4 h, 6 h, 8 h, 10 h, and 24 h.
20 μL of each of the plasma samples to be tested and a standard curve sample were pipetted, and an acetonitrile solution was added. Supernatants were obtained by protein precipitation, diluted, and then assayed by LC/MS/MS. Pharmacokinetic parameters were fitted using a non-compartmental model.

4.2 Rat pharmacokinetics

SD rats weighing 180-220 g were randomly divided into groups of 3 rats each after 3-5 days of acclimatization, and then subjected to intragastric administration.
Plasma samples to be tested were prepared by taking blood from the orbit at time points of 15 min, 30 min, 1 h, 2 h, 4 h, 6 h, 8 h, 10 h, and 24 h.
30 μL of each of the plasma samples to be tested and a standard curve sample were pipetted, and an acetonitrile solution was added. Supernatants were obtained by protein precipitation, diluted, and then assayed by LC/MS/MS. Pharmacokinetic parameters were fitted using a non-compartmental model.

4.3 Dog pharmacokinetics

Beagle dogs weighing 8-12 kg were divided into groups of 3 dogs each, and then subjected to intragastric administration.
Plasma samples to be tested were prepared by taking blood from the forelimb vein at time points of 15 min, 30 min, 1 h, 2 h, 4 h, 6 h, 8 h, 10 h, and 24 h.
50 μL of each of the plasma samples to be tested and a standard curve sample were pipetted, and an acetonitrile solution was added. Supernatants were obtained by protein precipitation, diluted, and then assayed by LC/MS/MS. Pharmacokinetic parameters were fitted using a non-compartmental model.
The test results show that the compounds of the present application have good *in vivo* pharmacokinetic properties ($T_{1/2}$ or AUC).

**Test Example 5:** Pharmacodynamic Evaluation in Subcutaneous Xenograft Tumor Nude Mouse Model of HCT116 MTAP-/- Colon Cancer

HCT116 MTAP-/- cells ($1 \times 10^7$ cells/mouse) were subcutaneously inoculated into the right axilla of SPF-grade female nude mice (source: Huachuang Sino). When the mean tumor volume reached 100-200 $mm^3$, the animals were grouped.
The day of grouping was defined as day 0, and intragastric administration was started on day 0 and performed once a day. The tumor volume was measured 2-3 times a week, and meanwhile, the mice were weighed and the data were recorded. The general behavior of the mice was observed and recorded every day. On the last day of the experiment, after intragastric administration, plasma samples to be tested were prepared by taking blood from the forelimb vein at time points of 0 h, 0.25 h, 1 h, 4 h, 8 h, and 24 h. 50 μL of each of the plasma samples to be tested and a standard curve sample were pipetted, and an acetonitrile solution was added. Supernatants were obtained by protein precipitation, diluted, and then assayed by LC/MS/MS. Pharmacokinetic parameters were fitted using a non-compartmental model. After the experiment was completed, the tumors were excised, weighed, and photographed.
The detection indexes and the calculation formulas are as follows:

Tumor volume TV ($mm^3$) = $1/2 \times (a \times b^2)$, where a represents the long diameter of the tumor, and b represents the short diameter of the tumor;
Relative tumor volume RTV = $TV_t/TV_0$, where $TV_0$ represents the tumor volume on day 0, and $TV_t$ represents the tumor volume at each measurement;
Relative tumor proliferation rate T/C (%) = $(T_{RTV}/C_{RTV}) \times 100\%$, where $T_{RTV}$ represents RTV of the treatment group, and $C_{RTV}$ represents RTV of the vehicle control group;
Tumor growth inhibition rate TGI (%) = $(1 - TW/TW_0) \times 100\%$; where TW represents the tumor weight of the treatment group, and $TW_0$ represents the tumor weight of the vehicle control group;
Weight change rate WCR (%) = $(Wtt - Wt_0)/Wt_0 \times 100\%$, where $Wt_0$ represents the body weight of the mouse on day 0, and Wtt represents the body weight of the mouse at each measurement.

$AUC_{(0-24\ h)}$ and free $AUC_{(0-24\ h)}$ were obtained by fitting with a non-compartmental model.
The results are shown in Tables 1 and 2.

**Table 1**

| Group | Day 21 of the experiment | | | | Day 28 of the experiment |
|---|---|---|---|---|---|
| | **TV($mm^3$)** (mean±SD) | **RTV** (mean ±SD) | **T/C(%)** | **Weight change rate** (%) (mean ± SD) | **Tumor weight** |
| Control group (vehicle control) | 1027±156 | 7.7±1.8 | ---- | 0.5±9.2 | 0.715±0.144 |
| Example 3b-30 mg/kg | 116±20 | 0.8±0.2 | 10.4% | 5.8±5.1 | 0.113±0.042 |
| Note: The drug was discontinued for observation on d13. The tumors were excised from the control group on d21, and from the administration group on d28. | | | | | |

**Table 2**

| Group | Day 21 of the experiment | | | | |
|---|---|---|---|---|---|
| | **TV($mm^3$)** (mean±SD) | **RTV** (mean ±SD) | **T/C(%)** | **Weight change rate** (%) (mean ± SD) | **TGI(%)** |
| Control group (vehicle control) | 1032±201 | 8.8±2.8 | ---- | 5.5±2.3 | ---- |
| Example 4b-30 mg/kg | 280±73 | 2.3±0.5 | 26.1% | -2.0±4.6 | 74.9%** |
| Note: comparison with the control group, where ** denotes $p < 0.01$. | | | | | |

The test result shows that the compounds of the present application have *in vivo* tumor inhibitory activity. The pharmacokinetic data ($AUC_{(0-24h)}$ and free $AUC_{(0-24h)}$) are favorable.

**Test Example 6:** *In Vitro* Plasma Protein Binding Rate

Plasma incubation samples were prepared by mixing heparin sodium anticoagulated plasma and the test compounds, and incubated at 37 °C and 300 rpm for 4 h (including the positive group). Zero-hour samples were prepared by mixing heparin sodium anticoagulated plasma and the test compounds. Buffer side samples were prepared using PBS. An acetonitrile solution was added to the samples. Supernatants were prepared by protein precipitation, diluted, and then assayed by LC/MS/MS. The test results show that the compounds of the present application have a high plasma protein binding rate (preferably > 1%).

## Claims

1. A compound of formula I, a stereoisomer thereof, or a pharmaceutically acceptable salt thereof,

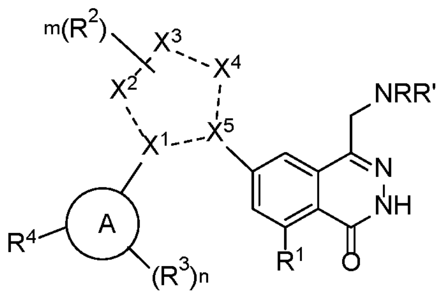

I

wherein

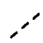

represents a single bond or a double bond;

R and R' are each independently selected from the group consisting of H, $C_{1-10}$ alkyl, and $C_{3-10}$ cycloalkyl;
$R^1$ is selected from the group consisting of hydrogen, halogen, OH, $C_{1-10}$ alkyl, halogenated $C_{1-10}$ alkyl, $C_{1-10}$ alkoxy, $C_{3-10}$ cycloalkyl, 3- to 10-membered heterocycloalkyl, 3- to 10-membered heterocycloalkenyl, $C_{2-10}$ alkenyl, $C_{2-10}$ alkynyl, $-NHR^a$, and $-NR^aR^b$;
$R^a$ and $R^b$ are each independently selected from the group consisting of H, $C_{1-10}$ alkyl, $C_{3-10}$ cycloalkyl, $-COC_{1-10}$ alkyl, and $-S(O)_2C_{1-10}$ alkyl;
$X^1$, $X^2$, $X^3$, $X^4$, and $X^5$ are each independently selected from the group consisting of S, C, CH, $CH_2$, N, and NH;
$R^2$ are each independently selected from the group consisting of =O, OH, $NH_2$, CN, halogen, $C_{1-10}$ alkyl, $C_{1-10}$ alkoxy, $C_{3-10}$ cycloalkyl, and 3- to 10-membered heterocycloalkyl, the $C_{1-10}$ alkyl, $C_{1-10}$ alkoxy, $C_{3-10}$ cycloalkyl, or 3- to 10-membered heterocycloalkyl being optionally substituted with one or more groups selected from the group consisting of OH, $NH_2$, CN, and halogen;
m is selected from the group consisting of 0, 1, 2, 3, 4, and 5;
ring A is selected from the group consisting of $C_{6-10}$ aryl, 5- to 12-membered heteroaryl, 5- to 15-membered heterocyclyl, and benzo $C_{5-7}$ cycloalkenyl;
$R^3$ are each independently selected from the group consisting of OH, $NH_2$, CN, halogen, and the following groups optionally substituted with one or more $R^5$: $C_{1-12}$ alkyl, $C_{1-12}$ alkoxy, $C_{1-12}$ alkyl S-, $C_{1-12}$ alkyl NH-, $(C_{1-12}$ alkyl$)_2$ N-, $C_{1-12}$ alkyl NHC(O)-, $(C_{1-12}$ alkyl$)_2$NC(O)-, $C_{1-12}$ alkyl C(O)NH-, $C_{1-12}$ alkyl OC(O)-, $C_{1-12}$ alkyl C(O)O-, $C_{2-12}$ alkenyl, $C_{2-12}$ alkynyl, $C_{3-12}$ cycloalkyl, 3- to 12-membered heterocycloalkyl, $C_{6-12}$ aryl, or 5- to 12-membered heteroaryl;
$R^5$ is selected from the group consisting of OH, $NH_2$, CN, halogen, and the following groups optionally substituted with one or more $R^d$: $C_{1-10}$ alkyl, $C_{1-10}$ alkoxy, $C_{1-10}$ alkyl S-, $C_{1-10}$ alkyl NH-, $(C_{1-10}$ alkyl$)_2$ N-, $C_{2-10}$ alkenyl, $C_{3-10}$ cycloalkyl, 3- to 10-membered heterocycloalkyl, $C_{6-10}$ aryl, or 5- to 10-membered heteroaryl;
$R^d$ is selected from the group consisting of OH, $NH_2$, CN, COOH, CHO, halogen, $C_{1-10}$ alkyl, $C_{1-10}$ alkoxy, $C_{1-10}$ alkyl S-, $C_{1-10}$ alkyl NH-, and $(C_{1-10}$ alkyl$)_2$ N-, the $C_{1-10}$ alkyl, $C_{1-10}$ alkoxy, $C_{1-10}$ alkyl S-, $C_{1-10}$ alkyl NH-, or $(C_{1-10}$ alkyl$)_2$ N- being optionally substituted with one or more groups selected from the group consisting of OH, $NH_2$, CN, and halogen;
n is selected from the group consisting of 0, 1, 2, 3, 4, and 5;
$R^4$ is selected from being absent, or is selected from -X-cycB;
X is selected from the group consisting of NH, $N(C_{1-10}$ alkyl), O, and S;
cycB is selected from the group consisting of $C_{3-10}$ cycloalkyl, 3- to 10-membered heterocycloalkyl, $C_{6-10}$ aryl, and 5- to 10-membered heteroaryl, the $C_{3-10}$ cycloalkyl, 3- to 10-membered heterocycloalkyl, $C_{6-10}$ aryl, or 5- to 10-membered heteroaryl being optionally substituted with one or more $R^c$;
$R^c$ are each independently selected from the group consisting of OH, $NH_2$, CN, halogen, $C_{1-10}$ alkyl, $C_{1-10}$ alkoxy, $C_{2-10}$ alkenyl, and $C_{2-10}$ alkynyl, the $C_{1-10}$ alkyl, $C_{1-10}$ alkoxy, $C_{2-10}$ alkenyl, or $C_{2-10}$ alkynyl being optionally substituted with one or more groups selected from the group consisting of OH, $NH_2$, CN, and halogen;
and the following compounds are excluded:

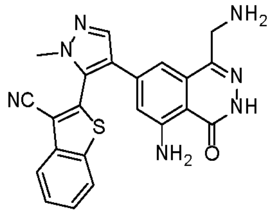
and
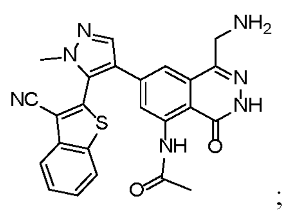
;

optionally, the proviso is that:
when $R^1$ is selected from the group consisting of hydrogen, halogen, OH, $C_{1-10}$ alkyl, halogenated $C_{1-10}$ alkyl, $C_{1-10}$ alkoxy, $C_{3-10}$ cycloalkyl, 3- to 10-membered heterocycloalkyl, and 3- to 10-membered heterocycloalkenyl, n is selected from the group consisting of 1, 2, 3, 4, and 5, one of $R^3$ is selected from the group consisting of $C_{2-12}$ alkenyl and $C_{2-12}$ alkynyl, the $C_{2-12}$ alkenyl or $C_{2-12}$ alkynyl being optionally substituted with one or more $R^5$, and the others of $R^3$ (if present, i.e., when n is selected from the group consisting of 2, 3, 4, and 5) are each independently selected from the group consisting of OH, $NH_2$, CN, halogen, and the following groups optionally substituted with one or more $R^5$: $C_{1-12}$ alkyl, $C_{1-12}$ alkoxy, $C_{1-12}$ alkyl S-, $C_{1-12}$ alkyl NH-, $(C_{1-12}$ alkyl$)_2$ N-, $C_{1-12}$ alkyl NHC(O)-, $(C_{1-12}$ alkyl$)_2$ NC(O)-, $C_{1-12}$ alkyl C(O)NH-, $C_{1-12}$ alkyl OC(O)-, $C_{1-12}$ alkyl C(O)O-, $C_{2-12}$ alkenyl, $C_{2-12}$ alkynyl, $C_{3-12}$ cycloalkyl, 3- to 12-membered heterocycloalkyl, $C_{6-12}$ aryl, or 5- to 12-membered heteroaryl.

**2.** The compound, the stereoisomer thereof, or the pharmaceutically acceptable salt thereof according to claim 1, wherein R and R' are each independently selected from the group consisting of H, $C_{1-6}$ alkyl, and $C_{3-8}$ cycloalkyl;

or, R and R' are each independently selected from the group consisting of H, $C_{1-3}$ alkyl, and $C_{3-6}$ cycloalkyl;
or, R and R' are each independently selected from the group consisting of H and $C_{1-3}$ alkyl;
or, R and R' are selected from H.

**3.** The compound, the stereoisomer thereof, or the pharmaceutically acceptable salt thereof according to claim 1 or 2, wherein $R^1$ is selected from the group consisting of hydrogen, halogen, OH, $C_{1-6}$ alkyl, one or more halogenated $C_{1-6}$ alkyl, $C_{1-6}$ alkoxy, $C_{3-6}$ cycloalkyl, 3- to 6-membered heterocycloalkyl, 3- to 6-membered heterocycloalkenyl, $C_{2-6}$ alkenyl, $C_{2-6}$ alkynyl, $-NHR^a$, and $-NR^aR^b$;

or, $R^1$ is selected from the group consisting of hydrogen, halogen, OH, $C_{1-6}$ alkyl, $C_{2-6}$ alkenyl, $C_{2-6}$ alkynyl, $-NHR^a$, and $-NR^aR^b$;
or, $R^1$ is selected from the group consisting of hydrogen, halogen, $NH_2$, $C_{2-6}$ alkenyl, and $C_{2-6}$ alkynyl;
or, $R^1$ is selected from the group consisting of hydrogen, F, Cl, Br, $NH_2$, $C_{2-4}$ alkenyl, and $C_{2-4}$ alkynyl;
or, $R^1$ is selected from the group consisting of hydrogen, Cl, $NH_2$, ethenyl, ethynyl, and propynyl;
or, $R^1$ is selected from the group consisting of hydrogen, $NH_2$, $C_{2-6}$ alkenyl, and $C_{2-6}$ alkynyl;
or, $R^1$ is selected from the group consisting of hydrogen, $NH_2$, $C_{2-4}$ alkenyl, and $C_{2-4}$ alkynyl; or, $R^1$ is selected from the group consisting of hydrogen, $NH_2$, $C_{2-3}$ alkenyl, and $C_{2-3}$ alkynyl;
or, $R^1$ is selected from the group consisting of hydrogen, $NH_2$, ethenyl, and ethynyl;
or, $R^1$ is selected from the group consisting of $NH_2$, $C_{2-6}$ alkenyl, and $C_{2-6}$ alkynyl;
or, $R^1$ is selected from the group consisting of hydrogen, Cl, $NH_2$, ethenyl, ethynyl, and 1-propynyl;
or, $R^1$ is hydrogen.

**4.** The compound, the stereoisomer thereof, or the pharmaceutically acceptable salt thereof according to any one of claims 1-3, wherein $R^a$ and $R^b$ are each independently selected from the group consisting of H, $C_{1-6}$ alkyl, $C_{3-6}$ cycloalkyl, $-COC_{1-6}$ alkyl, and $-S(O)_2C_{1-6}$ alkyl;

or, $R^a$ and $R^b$ are each independently selected from the group consisting of H, $C_{1-4}$ alkyl, $C_{3-6}$ cycloalkyl, $-COC_{1-4}$ alkyl, and $-S(O)_2C_{1-4}$ alkyl;
or, $R^a$ and $R^b$ are each independently selected from the group consisting of H, $C_{1-3}$ alkyl, $C_{3-5}$ cycloalkyl, $-COC_{1-3}$ alkyl, and $-S(O)_2C_{1-3}$ alkyl;
or, $R^a$ and $R^b$ are each independently selected from the group consisting of H and $C_{1-3}$ alkyl;
or, $R^a$ and $R^b$ are selected from H.

**5.** The compound, the stereoisomer thereof, or the pharmaceutically acceptable salt thereof according to any one of claims 1-4, wherein $X^1$ is selected from the group consisting of C and N;

optionally, $X^2$ is selected from the group consisting of CH and NH;
optionally, $X^3$ is selected from the group consisting of CH, $CH_2$, and N;
optionally, $X^4$ is selected from the group consisting of CH, N, and NH;
optionally, $X^5$ is selected from the group consisting of C and N;
optionally, $X^1$ and $X^5$ are each independently selected from the group consisting of C and N, and $X^2$, $X^3$, and $X^4$ are each independently selected from the group consisting of CH, $CH_2$, N, and NH.

**6.** The compound, the stereoisomer thereof, or the pharmaceutically acceptable salt thereof according to any one of claims 1-5, wherein the structural moiety

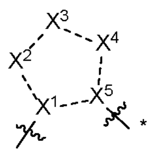

is selected from the group consisting of pyrrolyl, imidazolyl, dihydroimidazolyl, and pyrazolyl;
or, the structural moiety

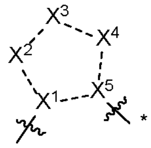

is selected from the group consisting of

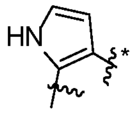 , 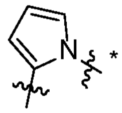 ,

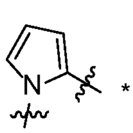 , 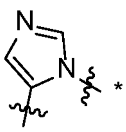 , 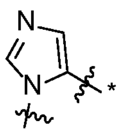 , 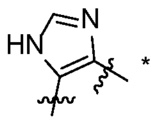 , 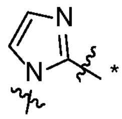 , 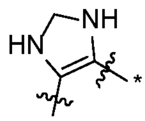 , and 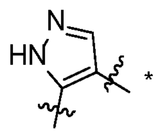 ,

wherein * indicates a corresponding site connected to

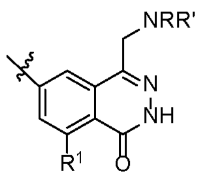 .

7. The compound, the stereoisomer thereof, or the pharmaceutically acceptable salt thereof according to any one of claims 1-6, wherein $R^2$ are each independently selected from the group consisting of =O, OH, $NH_2$, CN, halogen, $C_{1-6}$ alkyl, $C_{1-6}$ alkoxy, $C_{3-6}$ cycloalkyl, and 3- to 6-membered heterocycloalkyl, the $C_{1-6}$ alkyl, $C_{1-6}$ alkoxy, $C_{3-6}$ cycloalkyl, or 3- to 6-membered heterocycloalkyl being optionally substituted with one or more groups selected from the group consisting of OH, $NH_2$, CN, and halogen;

or, $R^2$ are each independently selected from the group consisting of =O, OH, $NH_2$, CN, halogen, $C_{1-6}$ alkyl, and $C_{1-6}$ alkoxy, the $C_{1-6}$ alkyl or $C_{1-6}$ alkoxy being optionally substituted with one or more groups selected from the group consisting of OH, $NH_2$, CN, and halogen;
or, $R^2$ are each independently selected from the group consisting of =O, OH, $NH_2$, CN, halogen, and $C_{1-6}$ alkyl, the $C_{1-6}$ alkyl being optionally substituted with one or more groups selected from the group consisting of OH, $NH_2$, CN, and halogen;
or, $R^2$ are each independently selected from the group consisting of =O and $C_{1-6}$ alkyl;
or, $R^2$ are each independently selected from the group consisting of =O and $C_{1-3}$ alkyl;
or, $R^2$ are each independently selected from the group consisting of =O and methyl;
or, $R^2$ is methyl.

8. The compound, the stereoisomer thereof, or the pharmaceutically acceptable salt thereof according to any one of claims 1-7, wherein m is selected from the group consisting of 1, 2, 3, and 4; or, m is selected from the group consisting of 1, 2, and 3; or, m is selected from the group consisting of 1 and 2; or, m is 1, and $R^2$ is connected to $X^2$; or, m is 2, and two $R^2$ are connected to $X^2$ and $X^3$, respectively; or, m is 2, and two $R^2$ are connected to $X^2$ and $X^4$, respectively.

9. The compound, the stereoisomer thereof, or the pharmaceutically acceptable salt thereof according to any one of claims 1-8, wherein ring A is selected from the group consisting of $C_{6-10}$ aryl and 5- to 15-membered heterocyclyl;

ring A is selected from the group consisting of $C_{6-10}$ aryl and 5- to 12-membered heterocyclyl;
or, ring A is selected from the group consisting of $C_{6-10}$ aryl, 5- to 10-membered heteroaryl, benzo 5- to 7-membered heterocycloalkenyl, and benzo $C_{5-7}$ cycloalkenyl;
or, ring A is selected from the group consisting of $C_{6-10}$ aryl, 5- to 6-membered heteroaryl, benzo 5- to 6-membered heterocycloalkenyl, and benzo $C_{5-6}$ cycloalkenyl;

or, ring A is selected from the group consisting of $C_{6-10}$ aryl and 5- to 10-membered heteroaryl;
or, ring A is selected from the group consisting of $C_{6-10}$ aryl and 5- to 6-membered heteroaryl;
or, ring A is selected from $C_{6-10}$ aryl; or, ring A is selected from the group consisting of phenyl and $C_{10}$ aryl;
or, ring A is selected from the group consisting of phenyl and naphthyl.

**10.** The compound, the stereoisomer thereof, or the pharmaceutically acceptable salt thereof according to any one of claims 1-9, wherein $R^3$ are each independently selected from the group consisting of OH, $NH_2$, CN, halogen, and the following groups optionally substituted with one or more $R^5$: $C_{1-10}$ alkyl, $C_{1-10}$ alkoxy, $C_{1-10}$ alkyl S-, $C_{1-10}$ alkyl NH-, $(C_{1-10}$ alkyl$)_2$ N-, $C_{1-10}$ alkyl NHC(O)-, $(C_{1-10}$ alkyl$)_2$ NC(O)-, $C_{1-10}$ alkyl C(O)NH-, $C_{1-10}$ alkyl OC(O)-, $C_{1-10}$ alkyl C(O) O-, $C_{2-10}$ alkenyl, $C_{2-1O}$ alkynyl, $C_{3-10}$ cycloalkyl, 3- to 10-membered heterocycloalkyl, $C_{6-10}$ aryl, or 5- to 10-membered heteroaryl;

or, $R^3$ are each independently selected from the group consisting of OH, $NH_2$, CN, halogen, and the following groups optionally substituted with one or more $R^5$: $C_{1-6}$ alkyl, $C_{1-6}$ alkoxy, $C_{2-8}$ alkenyl, $C_{2-10}$ alkynyl, $C_{3-6}$ cycloalkyl, 3- to 6-membered heterocycloalkyl, $C_{6-10}$ aryl, and 5- to 6-membered heteroaryl;
or, $R^3$ are each independently selected from the group consisting of OH, $NH_2$, CN, halogen, $C_{2-3}$ alkenyl, and $C_{2-6}$ alkynyl, the $C_{2-3}$ alkenyl or $C_{2-6}$ alkynyl being optionally substituted with one or more $R^5$;
or, $R^3$ are each independently selected from the group consisting of OH, $NH_2$, CN, F, Cl, Br, I, and the following groups optionally substituted with one or more $R^5$: ethenyl, ethynyl, propynyl, butynyl, dimethylbutynyl, or methylpenynyl;
or, $R^3$ are each independently selected from the group consisting of OH, $NH_2$, CN, F, Cl, Br, I, ethenyl, ethynyl, propynyl, butynyl, dimethylbutynyl, and methylpentynyl, the ethenyl, ethynyl, propynyl, butynyl, dimethylbutynyl, or methylpentynyl being optionally substituted with one or more substituents selected from the group consisting of OH, $NH_2$, CN, halogen, methoxy, ethoxy, cyclopropyl, cyclopentyl, cyclohexyl, phenyl, thienyl, pyrazolyl, pyrimidinyl, pyrazinyl, furanyl, and pyrrolyl, further optionally, the methoxy, ethoxy, cyclopropyl, cyclopentyl, cyclohexyl, phenyl, thienyl, pyrazolyl, pyrimidinyl, pyrazinyl, furanyl, or pyrrolyl being substituted with one or more $R^d$;
or, $R^3$ are each independently selected from the group consisting of $C_{2-6}$ alkenyl and $C_{2-8}$ alkynyl, the $C_{2-6}$ alkenyl or $C_{2-8}$ alkynyl being optionally substituted with one or more $R^5$;in some embodiments, $R^3$ are each independently selected from the group consisting of $C_{2-3}$ alkenyl and $C_{2-6}$ alkynyl, the $C_{2-3}$ alkenyl or $C_{2-6}$ alkynyl being optionally substituted with one or more $R^5$;in some embodiments, $R^3$ are each independently selected from the group consisting of $C_{2-3}$ alkenyl and $C_{2-4}$ alkynyl, the $C_{2-3}$ alkenyl or $C_{2-4}$ alkynyl being optionally substituted with one or more $R^5$;
or, $R^3$ are each independently selected from the group consisting of OH, CN, F, Cl, propynyl, butynyl,

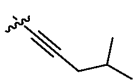, 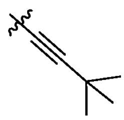, 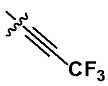, 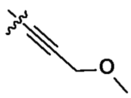, 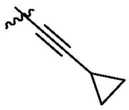, 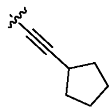, 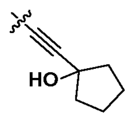, 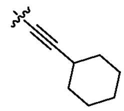,

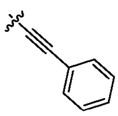, 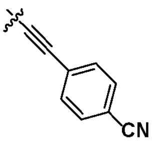, 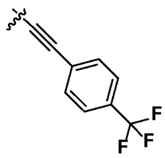, 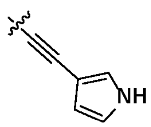, 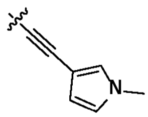, 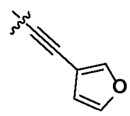, 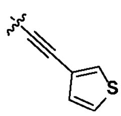,

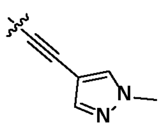, 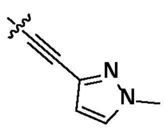, 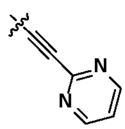, 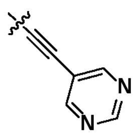, 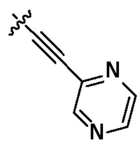,

ethenyl,

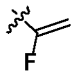, 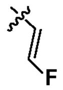, 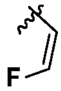, 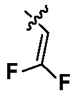,

and

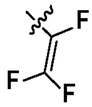;

or, $R^3$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl;
or, $R^3$ are each independently selected from the group consisting of OH, $NH_2$, CN, halogen, $C_{2-6}$ alkenyl, and $C_{2-6}$ alkynyl;
or, $R^3$ are each independently selected from the group consisting of CN, halogen, $C_{2-6}$ alkenyl, and $C_{2-6}$ alkynyl;
or, $R^3$ are each independently selected from the group consisting of CN, halogen, $C_{2-4}$ alkenyl, and $C_{2-4}$ alkynyl;
or, $R^3$ are each independently selected from the group consisting of CN, halogen, $C_{2-3}$ alkenyl, and $C_{2-3}$ alkynyl;
or, $R^3$ are each independently selected from the group consisting of CN, fluorine, chlorine, ethenyl, and propynyl.

**11.** The compound, the stereoisomer thereof, or the pharmaceutically acceptable salt thereof according to any one of claims 1-10, wherein $R^5$ is selected from the group consisting of OH, $NH_2$, CN, halogen, and the following groups optionally substituted with one or more $R^d$: $C_{1-8}$ alkyl, $C_{1-8}$ alkoxy, $C_{1-8}$ alkyl S-, $C_{1-8}$ alkyl NH-, $(C_{1-8}$ alkyl$)_2$ N-, $C_{2-8}$ alkenyl, $C_{3-8}$ cycloalkyl, 3- to 8-membered heterocycloalkyl, $C_{6-8}$ aryl, or 5- to 8-membered heteroaryl;

or, $R^5$ is selected from the group consisting of OH, $NH_2$, CN, halogen, and the following groups optionally substituted with one or more $R^d$: $C_{1-4}$ alkyl, $C_{1-4}$ alkoxy, $C_{1-4}$ alkyl S-, $C_{1-4}$ alkyl NH-, $(C_{l-4}$ alkyl$)_2$ N-, $C_{2-4}$ alkenyl, $C_{3-6}$ cycloalkyl, 3- to 6-membered heterocycloalkyl, phenyl, or 5- to 6-membered heteroaryl;
or, $R^5$ is selected from the group consisting of OH, $NH_2$, CN, fluorine, chlorine, bromine, and the following groups optionally substituted with one or more $R^d$: $C_{1-3}$ alkoxy, $C_{3-6}$ cycloalkyl, 4- to 6-membered heterocycloalkyl, phenyl, or 5- to 6-membered heteroaryl;
or, $R^5$ is selected from the group consisting of OH, $NH_2$, CN, fluorine, chlorine, bromine, and the following groups optionally substituted with one or more $R^d$: methoxy, ethoxy, cyclopropyl, cyclobutyl, cyclopentyl, azetidinyl, piperidinyl, dioxanyl, piperazinyl, morpholinyl, tetrahydropyranyl, cyclohexyl, phenyl, pyrrolyl, furanyl, thienyl, pyrazolyl, imidazolyl, pyridazinyl, pyrimidinyl, or pyrazinyl;
or, $R^5$ is selected from the group consisting of OH, fluorine, and the following groups optionally substituted with one or more $R^d$: methoxy, cyclopropyl, cyclopentyl, cyclohexyl, phenyl, pyrrolyl, furanyl, thienyl, pyrazolyl, pyrimidinyl, or pyrazinyl;
or, $R^5$ is selected from the group consisting of the following groups optionally substituted with one or more $R^d$: $C_{3-6}$ cycloalkyl, phenyl, or 5- to 6-membered heteroaryl;
or, $R^5$ is selected from the group consisting of the following groups optionally substituted with one or more $R^d$: cyclopropyl, cyclopentyl, cyclohexyl, phenyl, pyrrolyl, furanyl, thienyl, pyrazolyl, pyrimidinyl, or pyrazinyl.

**12.** The compound, the stereoisomer thereof, or the pharmaceutically acceptable salt thereof according to any one of claims 1-11, wherein $R^d$ is selected from the group consisting of OH, $NH_2$, CN, halogen, $C_{1-6}$ alkyl, $C_{1-6}$ alkoxy, $C_{1-6}$ alkyl S-, $C_{1-6}$ alkyl NH-, and $(C_{1-6}$ alkyl$)_2$ N-, the $C_{1-6}$ alkyl, $C_{1-6}$ alkoxy, $C_{1-6}$ alkyl S-, $C_{1-6}$ alkyl NH-, or $(C_{1-6}$ alkyl$)_2$ N- being optionally substituted with one or more groups selected from the group consisting of OH, $NH_2$, CN, and halogen;

or, $R^d$ is selected from the group consisting of OH, $NH_2$, CN, halogen, $C_{1-4}$ alkyl, and $C_{1-4}$ haloalkyl;
or, $R^d$ is selected from the group consisting of OH, $NH_2$, CN, F, Cl, Br, $C_{1-3}$ alkyl, and $C_{1-3}$ haloalkyl; or, $R^d$ is selected from the group consisting of CN, $C_{1-3}$ alkyl, and $C_{1-3}$ haloalkyl;
or, $R^d$ is selected from the group consisting of OH, CN, F, Cl, Br, I, methyl, ethyl, and trifluoromethyl;
or, $R^d$ is selected from the group consisting of CN, methyl, and trifluoromethyl.

**13.** The compound, the stereoisomer thereof, or the pharmaceutically acceptable salt thereof according to any one of claims 1-12, wherein n is selected from the group consisting of 1, 2, and 3; or, n is selected from the group consisting of 2 and 3; or, n is 3.

**14.** The compound, the stereoisomer thereof, or the pharmaceutically acceptable salt thereof according to any one of claims 1-13, wherein X is selected from the group consisting of NH, N($C_{1-6}$ alkyl), O, and S;

or, X is selected from the group consisting of NH, N($C_{1-3}$ alkyl), O, and S;
or, X is selected from the group consisting of NH, O, and S;
or, X is selected from the group consisting of NH and O; or, X is selected from O.

**15.** The compound, the stereoisomer thereof, or the pharmaceutically acceptable salt thereof according to any one of claims 1-14, wherein cycB is selected from the group consisting of $C_{3-6}$ cycloalkyl, 3- to 6-membered heterocycloalkyl, $C_{6-10}$ aryl, and 5- to 6-membered heteroaryl, the $C_{3-6}$ cycloalkyl, 3- to 6-membered heterocycloalkyl, $C_{6-10}$ aryl, and 5- to 6-membered heteroaryl being optionally substituted with one or more $R^c$;

or, cycB is selected from the group consisting of $C_{3-10}$ cycloalkyl and 3- to 10-membered heterocycloalkyl, the $C_{3-10}$ cycloalkyl or 3- to 10-membered heterocycloalkyl being optionally substituted with one or more $R^c$;
or, cycB is selected from the group consisting of $C_{3-6}$ cycloalkyl and 3- to 6-membered heterocycloalkyl, the $C_{3-6}$ cycloalkyl or 3- to 6-membered heterocycloalkyl being optionally substituted with one or more $R^c$;
or, cycB is selected from the group consisting of $C_{3-4}$ cycloalkyl and 3- to 4-membered heterocycloalkyl, the $C_{3-4}$ cycloalkyl or 3- to 4-membered heterocycloalkyl being optionally substituted with one or more $R^c$;
or, cycB is selected from $C_{3-10}$ cycloalkyl, the $C_{3-10}$ cycloalkyl being optionally substituted with one or more $R^c$; or, cycB is selected from $C_{3-6}$ cycloalkyl, the $C_{3-6}$ cycloalkyl being optionally substituted with one or more $R^c$;
or, cycB is selected from $C_{3-4}$ cycloalkyl, the $C_{3-4}$ cycloalkyl being optionally substituted with one or more $R^c$;
or, cycB is selected from cyclopropyl, the cyclopropyl being optionally substituted with one or more $R^c$;
or, cycB is selected from cyclopropyl.

**16.** The compound, the stereoisomer thereof, or the pharmaceutically acceptable salt thereof according to any one of claims 1-15, wherein $R^c$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl, the $C_{1-6}$ alkyl, $C_{1-6}$ alkoxy, $C_{2-6}$ alkenyl, or $C_{2-6}$ alkynyl being optionally substituted with one or more groups selected from the group consisting of OH, $NH_2$, CN, and halogen;

or, $R^c$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl;
or, $R^c$ are each independently selected from the group consisting of OH, $NH_2$, CN, halogen, and $C_{1-6}$ alkyl;
or, $R^c$ are each independently selected from the group consisting of halogen and $C_{1-6}$ alkyl; or, $R^c$ are each independently selected from the group consisting of halogen and $C_{1-3}$ alkyl.

**17.** The compound, the stereoisomer thereof, or the pharmaceutically acceptable salt thereof according to any one of claims 1-16, wherein the compound of formula I, the stereoisomer thereof, or the pharmaceutically acceptable salt thereof is selected from the group consisting of compounds of formulas II, III, IV, II-1, II-1A, II-1B, III-1, III-1A, III-1B, IV-1, IV-1A, IV-1B, VI-1C, VI-1D, VI-1E, VII, VII-1, VII-1A, and VII-1B, stereoisomers thereof, or pharmaceutically acceptable salts thereof,

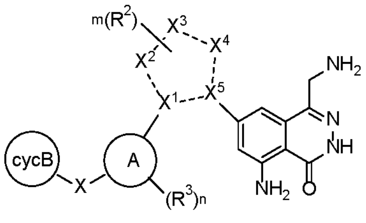

II

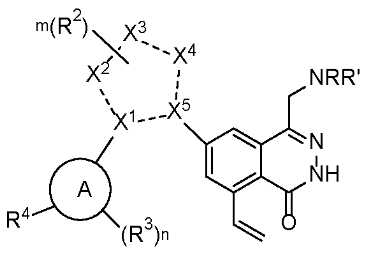

III

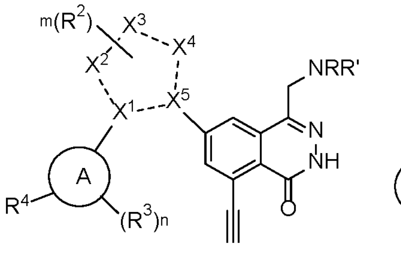

IV

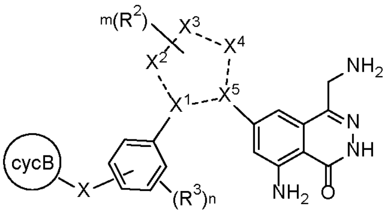

II-1

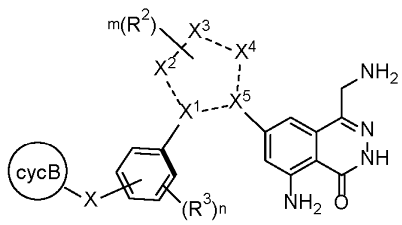

II-1A

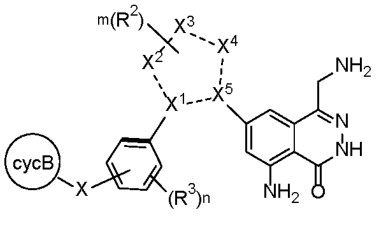

II-1B

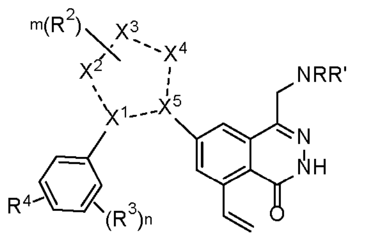

III-1

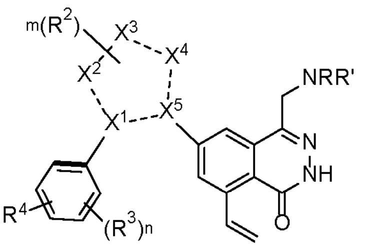

III-1A

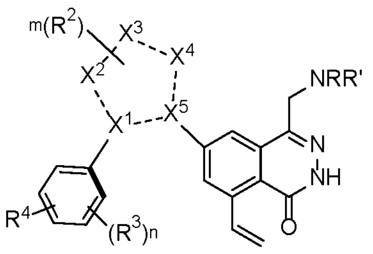

III-1B

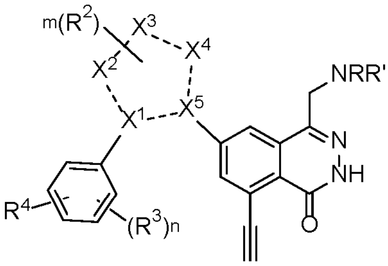

IV-1

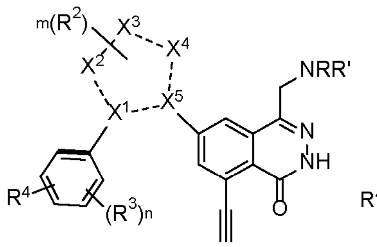

IV-1A

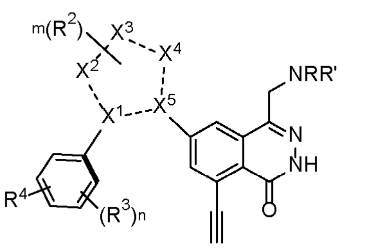

IV-1B

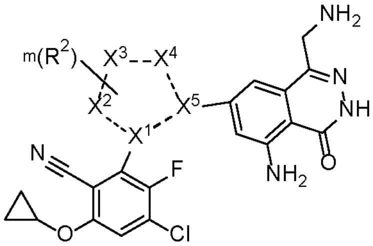

VI-1C

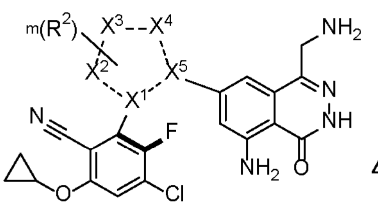

VI-1D

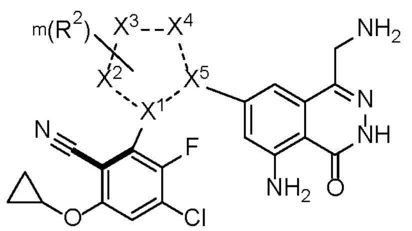

VI-1E

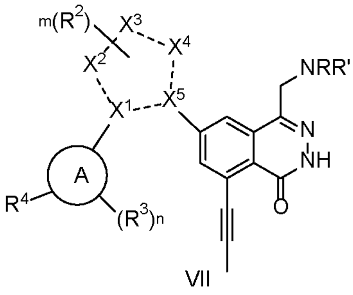

VII

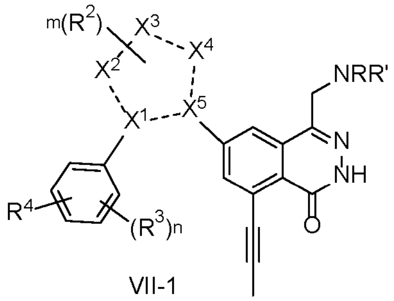

VII-1

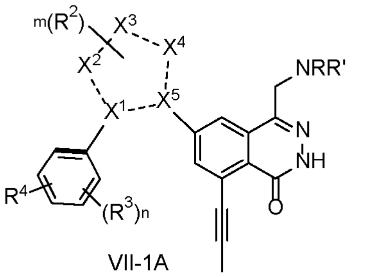

VII-1A

and

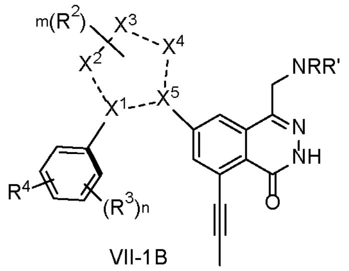

VII-1B

.

**18.** The compound, the stereoisomer thereof, or the pharmaceutically acceptable salt thereof according to any one of claims 1-17, wherein the compound of formula I, the stereoisomer thereof, or the pharmaceutically acceptable salt thereof is selected from a compound of formula V, a stereoisomer thereof, or a pharmaceutically acceptable salt thereof,

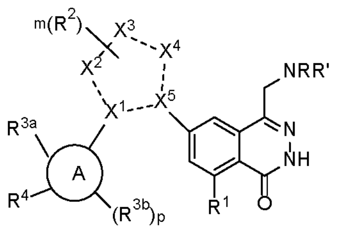

V

$R^1$ is selected from the group consisting of hydrogen, halogen, OH, $C_{1-10}$ alkyl, halogenated $C_{1-10}$ alkyl, $C_{1-10}$ alkoxy, $C_{3-10}$ cycloalkyl, 3- to 10-membered heterocycloalkyl, 3- to 10-membered heterocycloalkenyl, $C_{2-10}$ alkenyl, $C_{2-10}$ alkynyl, $-NHR^a$, and $-NR^aR^b$;
p is selected from the group consisting of 0, 1, 2, 3, and 4;
$R^{3a}$ is selected from the group consisting of $C_{2-10}$ alkenyl and $C_{2-10}$ alkynyl, the $C_{2-10}$ alkenyl or $C_{2-10}$ alkynyl being optionally substituted with one or more $R^5$;
$R^5$ is selected from the group consisting of OH, $NH_2$, CN, halogen, and the following groups optionally substituted with one or more $R^d$: $C_{1-10}$ alkyl, $C_{1-10}$ alkoxy, $C_{1-10}$ alkyl S-, $C_{1-10}$ alkyl NH-, $(C_{1-10}$ alkyl$)_2$ N-, $C_{2-10}$ alkenyl, $C_{3-10}$ cycloalkyl, 3- to 10-membered heterocycloalkyl, $C_{6-10}$ aryl, or 5- to 10-membered heteroaryl;
$R^d$ is selected from the group consisting of OH, $NH_2$, CN, COOH, CHO, halogen, $C_{1-10}$ alkyl, $C_{1-10}$ alkoxy, $C_{1-10}$ alkyl S-, $C_{1-10}$ alkyl NH-, and $(C_{1-10}$ alkyl$)_2$ N-, the $C_{1-10}$ alkyl, $C_{1-10}$ alkoxy, $C_{1-10}$ alkyl S-, $C_{1-10}$ alkyl NH-, or $(C_{1-10}$ alkyl$)_2$ N- being optionally substituted with one or more groups selected from the group consisting of OH, $NH_2$, CN, and halogen;
$R^{3b}$ are each independently selected from the group consisting of OH, $NH_2$, CN, halogen, $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, and 5- to 10-membered heteroaryl, the $C_{1-10}$ alkyl, $C_{1-10}$ alkoxy, $C_{2-10}$ alkenyl, $C_{2-10}$ alkynyl, $C_{3-10}$ cycloalkyl, 3- to 10-membered heterocycloalkyl, $C_{6-10}$ aryl, or 5- to 10-membered heteroaryl being optionally substituted with one or more groups selected from the group consisting of OH, $NH_2$, CN, and halogen.

**19.** The compound, the stereoisomer thereof, or the pharmaceutically acceptable salt thereof according to claim 18, wherein $R^1$ is selected from the group consisting of hydrogen, halogen, OH, $C_{1-6}$ alkyl, halogenated $C_{1-6}$ alkyl, $C_{1-6}$

alkoxy, $C_{3-6}$ cycloalkyl, 3- to 6-membered heterocycloalkyl, 3- to 6-membered heterocycloalkenyl, $C_{2-6}$ alkenyl, $C_{2-6}$ alkynyl, $-NHR^a$, and $-NR^aR^b$;

or, $R^1$ is selected from the group consisting of hydrogen, halogen, OH, $C_{1-6}$ alkyl, $C_{2-6}$ alkenyl, $C_{2-6}$ alkynyl, $-NHR^a$, and $-NR^aR^b$;
or, $R^1$ is selected from the group consisting of hydrogen, halogen, $NH_2$, $C_{2-6}$ alkenyl, and $C_{2-6}$ alkynyl;
or, $R^1$ is selected from the group consisting of hydrogen, F, Cl, Br, I, $NH_2$, $C_{2-4}$ alkenyl, and $C_{2-4}$ alkynyl;
or, $R^1$ is selected from the group consisting of hydrogen, F, Cl, $NH_2$, $C_{2-3}$ alkenyl, and $C_{2-3}$ alkynyl;
or, $R^1$ is selected from the group consisting of hydrogen, Cl, $NH_2$, ethenyl, ethynyl, and propynyl;
or, $R^1$ is selected from the group consisting of hydrogen, $NH_2$, $C_{2-6}$ alkenyl, and $C_{2-6}$ alkynyl;
or, $R^1$ is selected from the group consisting of hydrogen, $NH_2$, $C_{2-4}$ alkenyl, and $C_{2-4}$ alkynyl;
or, $R^1$ is selected from the group consisting of hydrogen, $NH_2$, $C_{2-3}$ alkenyl, and $C_{2-3}$ alkynyl;
or, $R^1$ is selected from the group consisting of hydrogen, $NH_2$, ethenyl, and ethynyl;
or, $R^1$ is selected from the group consisting of hydrogen, halogen, OH, $C_{1-6}$ alkyl, halogenated $C_{1-6}$ alkyl, $C_{1-6}$ alkoxy, $C_{3-6}$ cycloalkyl, 3- to 6-membered heterocycloalkyl, and 3- to 6-membered heterocycloalkenyl; or, $R^1$ is selected from the group consisting of hydrogen, halogen, OH, and $C_{1-6}$ alkyl; or, $R^1$ is selected from hydrogen; or, $R^1$ is selected from the group consisting of $C_{2-6}$ alkenyl, $C_{2-6}$ alkynyl, $-NHR^a$, and $-NR^aR^b$; or, $R^1$ is selected from the group consisting of $NH_2$, $C_{2-6}$ alkenyl, and $C_{2-6}$ alkynyl; or, $R^1$ is selected from the group consisting of $NH_2$, $C_{2-4}$ alkenyl, and $C_{2-4}$ alkynyl; or, $R^1$ is selected from the group consisting of $NH_2$, $C_{2-3}$ alkenyl, and $C_{2-3}$ alkynyl; or, $R^1$ is selected from the group consisting of $NH_2$, ethenyl, and ethynyl;
or, $R^1$ is selected from the group consisting of $C_{2-6}$ alkenyl and $C_{2-6}$ alkynyl; or, $R^1$ is selected from the group consisting of $C_{2-4}$ alkenyl and $C_{2-4}$ alkynyl; or, $R^1$ is selected from the group consisting of $C_{2-3}$ alkenyl and $C_{2-3}$ alkynyl; or, $R^1$ is selected from the group consisting of ethenyl and ethynyl; or, $R^1$ is selected from the group consisting of $-NHR^a$ and $-NR^aR^b$; or, $R^1$ is selected from $NH_2$;
optionally, p is selected from the group consisting of 0, 1, 2, and 3; or, p is selected from the group consisting of 0, 1, and 2;
optionally, $R^{3a}$ is selected from the group consisting of $C_{2-6}$ alkenyl and $C_{2-6}$ alkynyl, the $C_{2-6}$ alkenyl or $C_{2-6}$ alkynyl being optionally substituted with one or more $R^5$;
or, $R^{3a}$ is selected from the group consisting of $C_{2-4}$ alkenyl and $C_{2-4}$ alkynyl, the $C_{2-4}$ alkenyl or $C_{2-4}$ alkynyl being optionally substituted with one or more $R^5$;
or, $R^{3a}$ is selected from the group consisting of propynyl, butynyl,

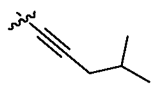, 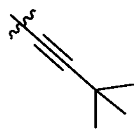, 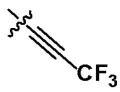,

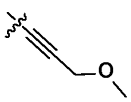, 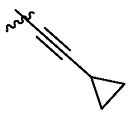, 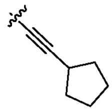, 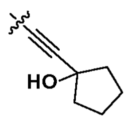, 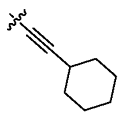, 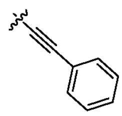, 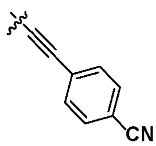,

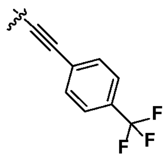, 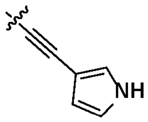, 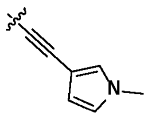, 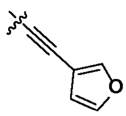, 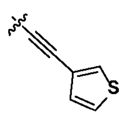, 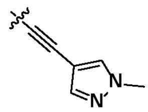, 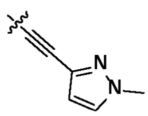,

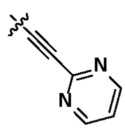, 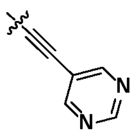, 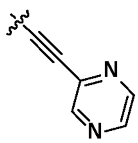,

ethenyl,

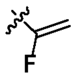, 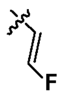, 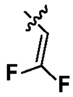, and 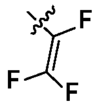;

or, $R^{3a}$ is selected from the group consisting of $C_{2-10}$ alkenyl and $C_{2-10}$ alkynyl, the $C_{2-10}$ alkenyl or $C_{2-10}$ alkynyl being optionally substituted with one or more groups selected from the group consisting of OH, $NH_2$, CN, and halogen;
or, $R^{3a}$ is selected from the group consisting of $C_{2-6}$ alkenyl and $C_{2-6}$ alkynyl, the $C_{2-6}$ alkenyl or $C_{2-6}$ alkynyl being optionally substituted with one or more groups selected from the group consisting of OH, $NH_2$, CN, and halogen;
or, $R^{3a}$ is selected from the group consisting of $C_{2-4}$ alkenyl and $C_{2-4}$ alkynyl, the $C_{2-4}$ alkenyl or $C_{2-4}$ alkynyl being optionally substituted with one or more groups selected from the group consisting of OH, $NH_2$, CN, and halogen;
or, $R^{3a}$ is selected from the group consisting of $C_{2-6}$ alkenyl and $C_{2-6}$ alkynyl;
or, $R^{3a}$ is selected from the group consisting of $C_{2-4}$ alkenyl and $C_{2-4}$ alkynyl;
or, $R^{3a}$ is selected from the group consisting of $C_{2-3}$ alkenyl and $C_{2-3}$ alkynyl;
or, $R^{3a}$ is selected from the group consisting of ethenyl and propynyl;
or, $R^{3b}$ are each independently selected from the group consisting of OH, $NH_2$, CN, halogen, $C_{1-6}$ alkyl, $C_{1-6}$ alkoxy, $C_{2-6}$ alkenyl, $C_{2-6}$ alkynyl, $C_{3-6}$ cycloalkyl, 3- to 6-membered heterocycloalkyl, $C_{6-10}$ aryl, and 5- to 6-membered heteroaryl, the $C_{1-6}$ alkyl, $C_{1-6}$ alkoxy, $C_{2-6}$ alkenyl, $C_{2-6}$ alkynyl, $C_{3-6}$ cycloalkyl, 3- to 6-membered heterocycloalkyl, $C_{6-10}$ aryl, or 5- to 6-membered heteroaryl being optionally substituted with one or more groups selected from the group consisting of OH, $NH_2$, CN, and halogen;
or, $R^{3b}$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl, the $C_{1-6}$ alkyl, $C_{1-6}$ alkoxy, $C_{2-6}$ alkenyl, or $C_{2-6}$ alkynyl being optionally substituted with one or more groups selected from the group consisting of OH, $NH_2$, CN, and halogen;
or, $R^{3b}$ are each independently selected from the group consisting of OH, $NH_2$, CN, halogen, $C_{1-6}$ alkyl, $C_{1-6}$ alkoxy, $C_{2-6}$ alkenyl, and $C_{2-6}$ alkynyl;
or, $R^{3b}$ are each independently selected from the group consisting of OH, $NH_2$, CN, halogen, $C_{2-6}$ alkenyl, and $C_{2-6}$ alkynyl;
or, $R^{3b}$ are each independently selected from the group consisting of CN, halogen, $C_{2-6}$ alkenyl, and $C_{2-6}$ alkynyl;
or, $R^{3b}$ are each independently selected from the group consisting of CN, halogen, $C_{2-4}$ alkenyl, and $C_{2-4}$ alkynyl;
or, $R^{3b}$ are each independently selected from the group consisting of CN, halogen, $C_{2-3}$ alkenyl, and $C_{2-3}$ alkynyl;
or, $R^{3b}$ are each independently selected from the group consisting of OH, $NH_2$, CN, halogen, $C_{1-6}$ alkyl, and $C_{1-6}$ alkoxy, the $C_{1-6}$ alkyl or $C_{1-6}$ alkoxy being optionally substituted with one or more groups selected from the group consisting of OH, $NH_2$, CN, and halogen;
or, $R^{3b}$ are each independently selected from the group consisting of OH, $NH_2$, CN, halogen, $C_{1-6}$ alkyl, and $C_{1-6}$ alkoxy;
or, $R^{3b}$ are each independently selected from the group consisting of OH, $NH_2$, CN, halogen, $C_{1-4}$ alkyl, and $C_{1-4}$ alkoxy;
or, $R^{3b}$ are each independently selected from the group consisting of OH, $NH_2$, CN, halogen, and $C_{1-4}$ alkyl;
or, $R^{3b}$ are each independently selected from the group consisting of OH, $NH_2$, CN, and halogen;
or, $R^{3b}$ are each independently selected from the group consisting of CN and halogen;
or, $R^{3b}$ are each independently selected from the group consisting of CN, fluorine, and chlorine.

**20.** The compound, the stereoisomer thereof, or the pharmaceutically acceptable salt thereof according to any one of claims 1-19, wherein the compound of formula I, the stereoisomer thereof, or the pharmaceutically acceptable salt thereof is selected from the group consisting of compounds of formula V-1, formula V-2, and formula V-3, stereoisomers thereof, or pharmaceutically acceptable salts thereof,

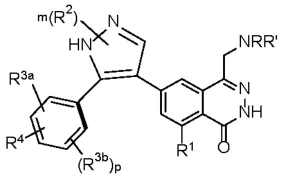
V-1

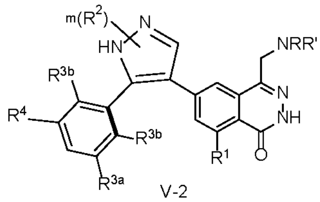
V-2

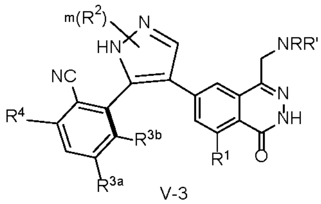
V-3

.

**21.** A compound of the following formula, a stereoisomer thereof, or a pharmaceutically acceptable salt thereof:

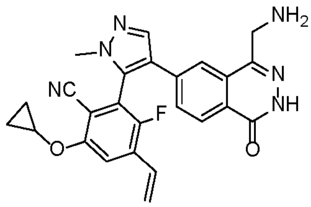
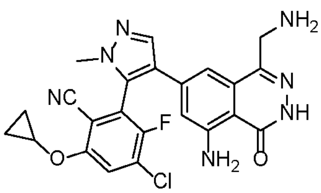
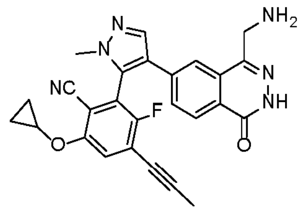
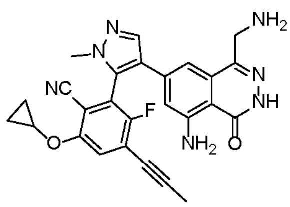

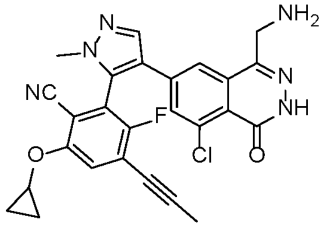
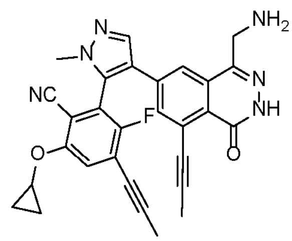
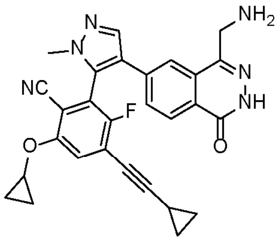
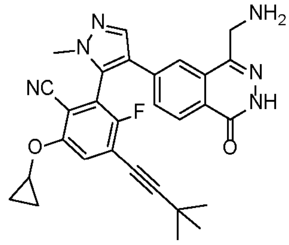

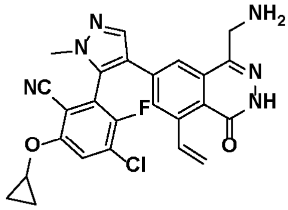
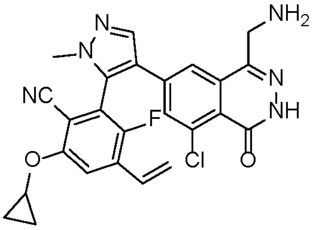
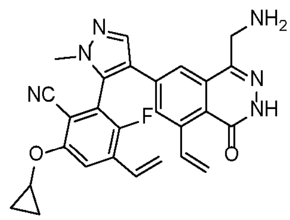
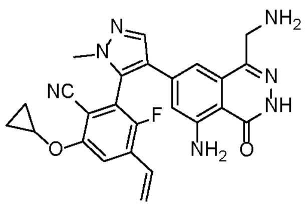

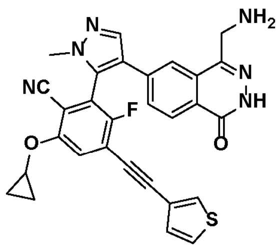
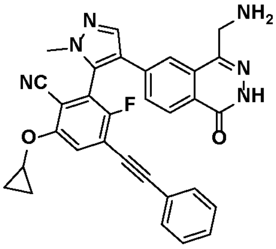
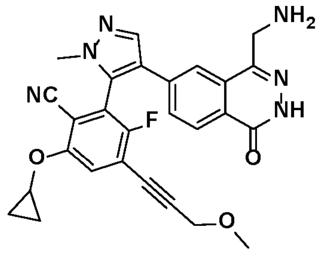
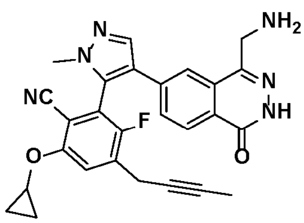

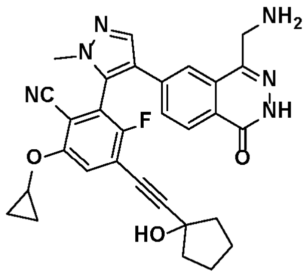
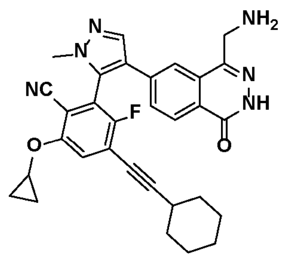
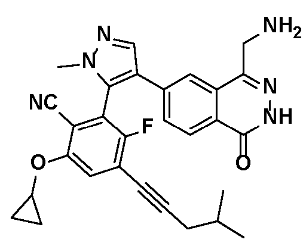
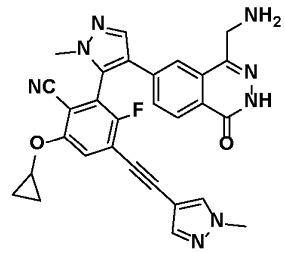

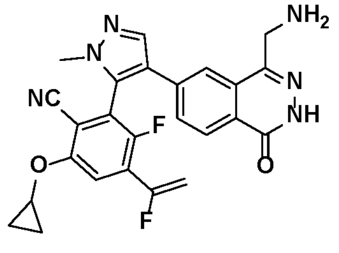

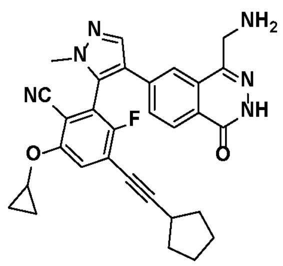
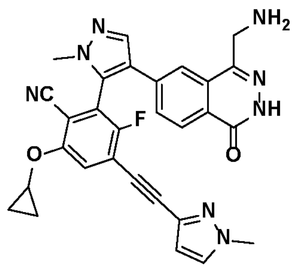
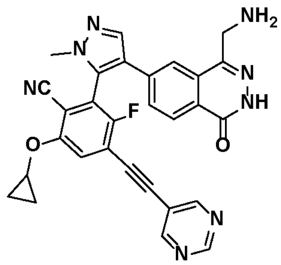
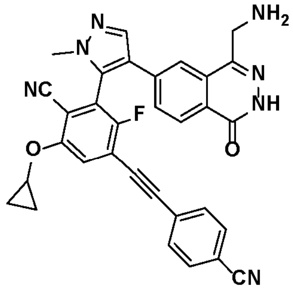

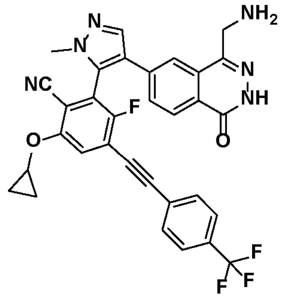
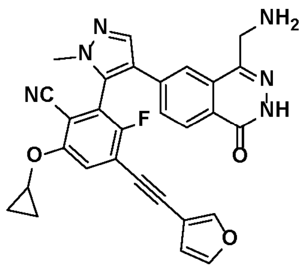
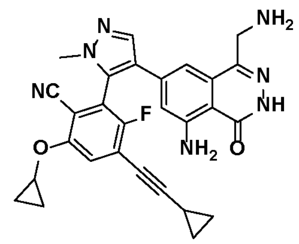
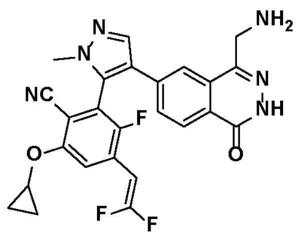

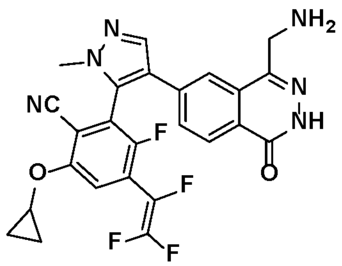

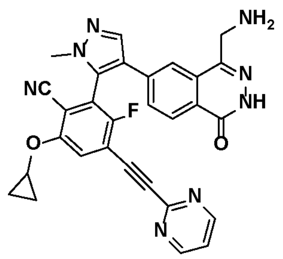
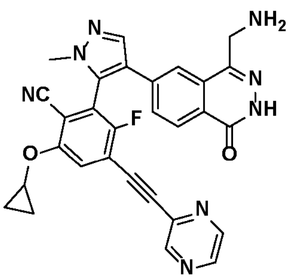
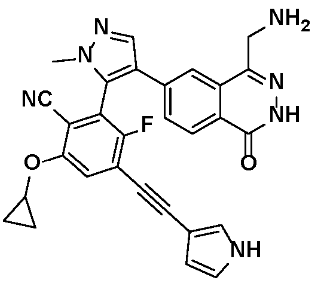
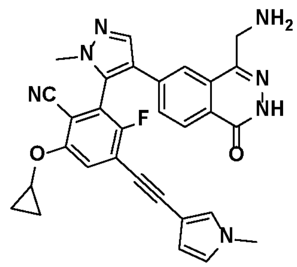

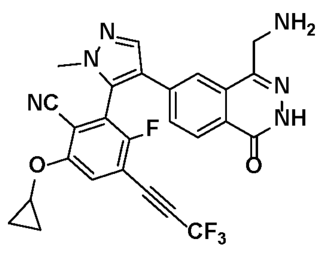
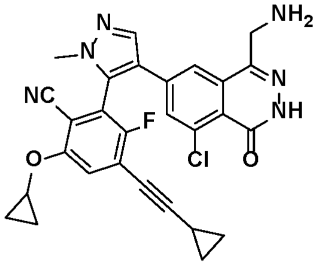
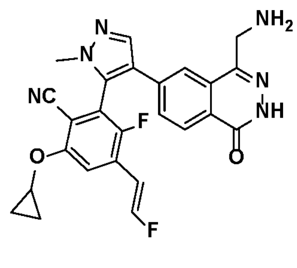

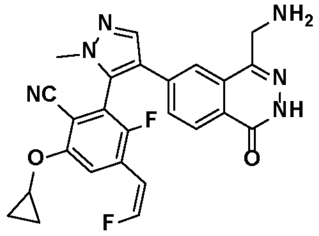 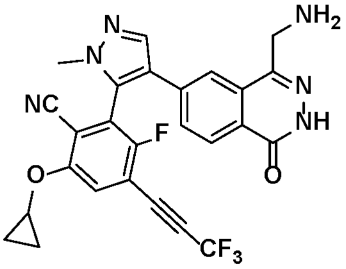

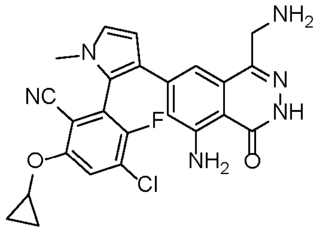 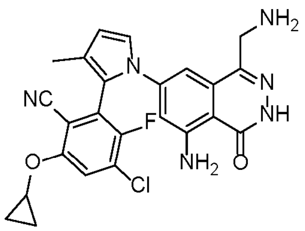 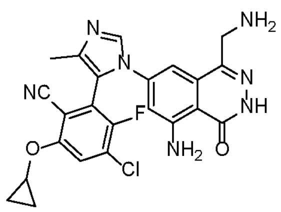 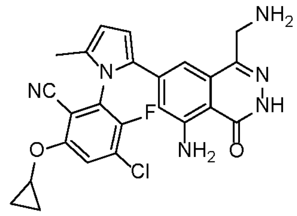

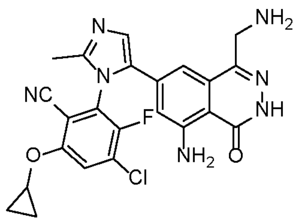 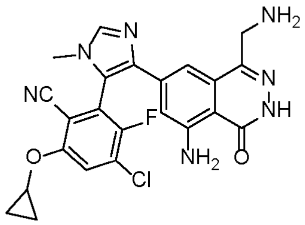 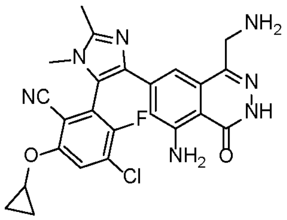 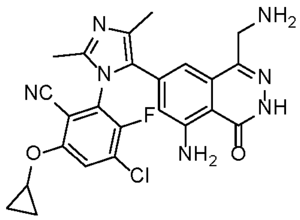

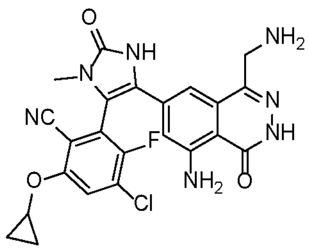 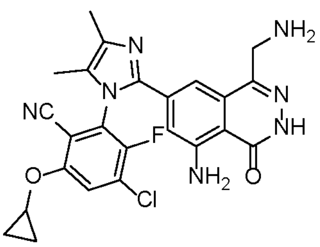 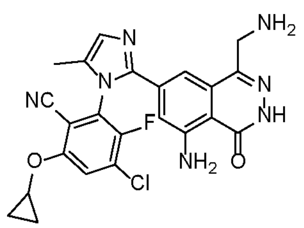

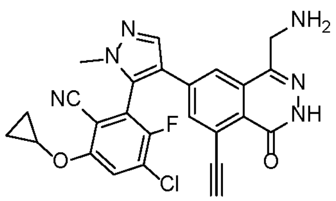 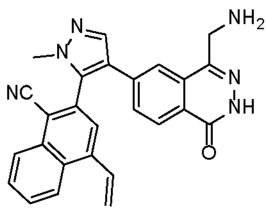 or 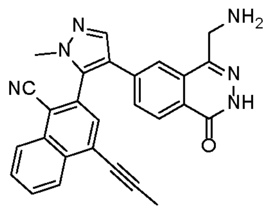 ;

or compounds of the following formulas or pharmaceutically acceptable salts thereof:

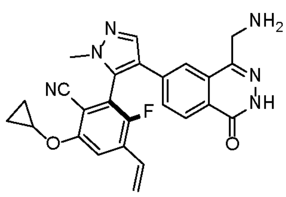 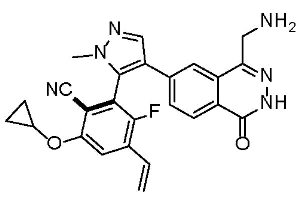 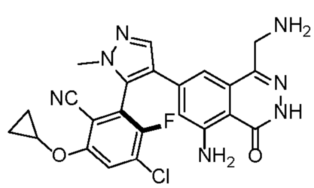 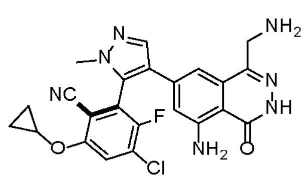

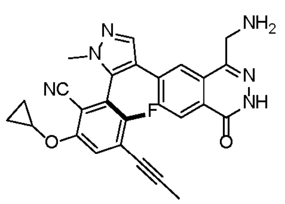
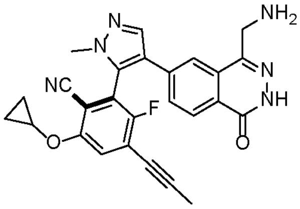
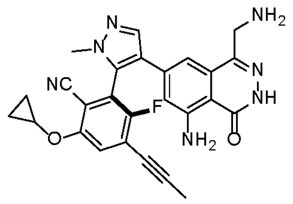
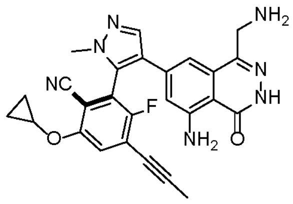

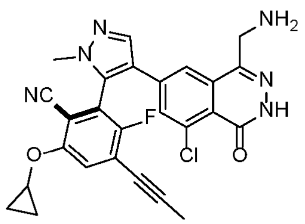
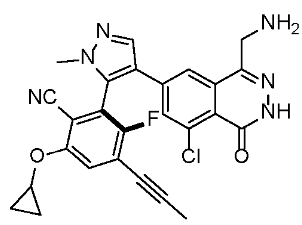
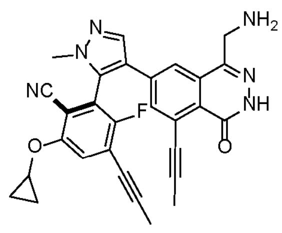
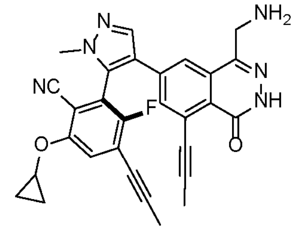

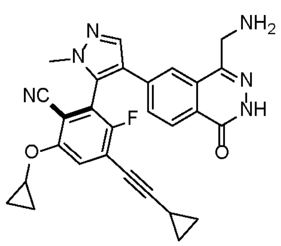
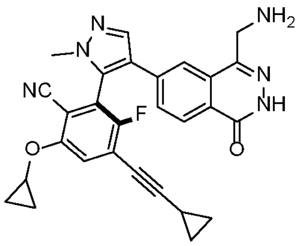
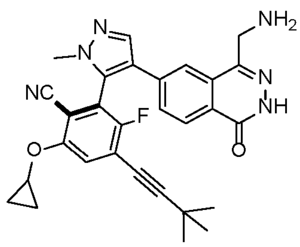
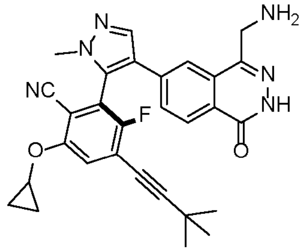

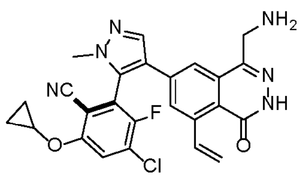
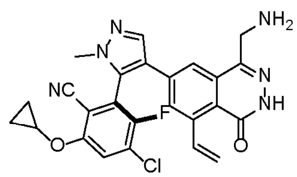
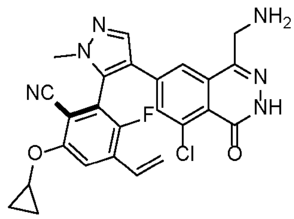
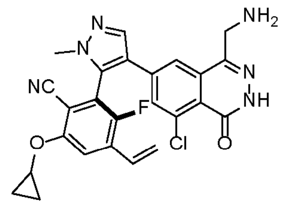

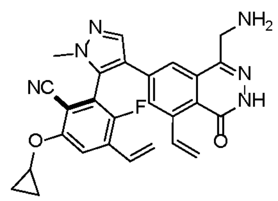
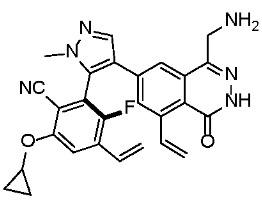
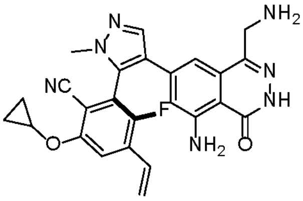
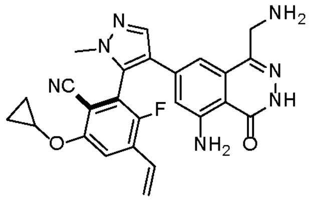

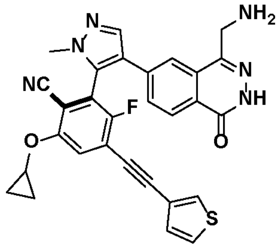
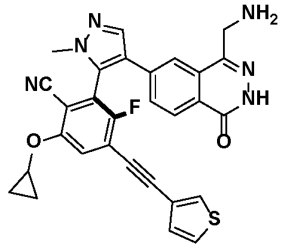
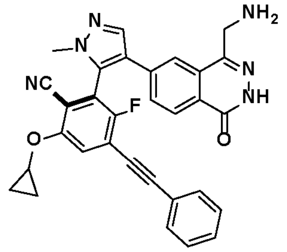
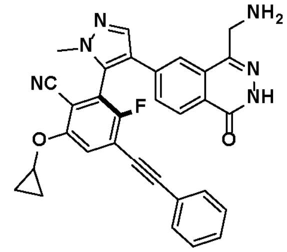

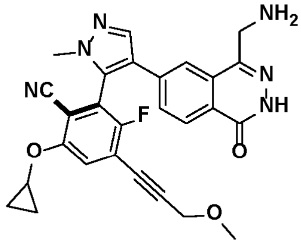 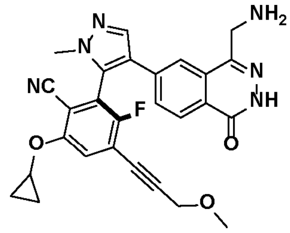 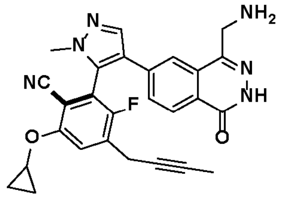 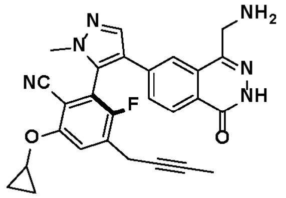

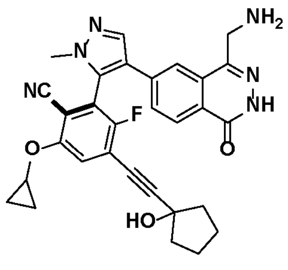 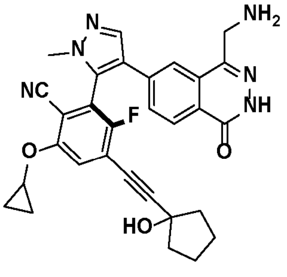 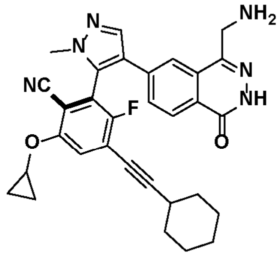 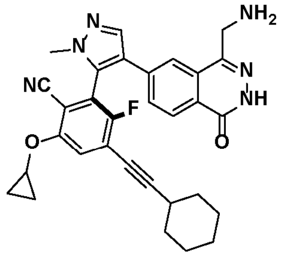

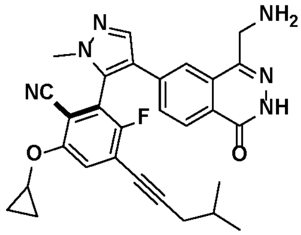 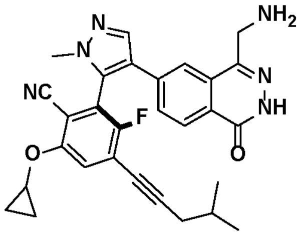 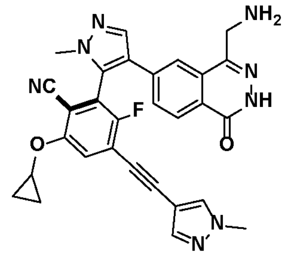 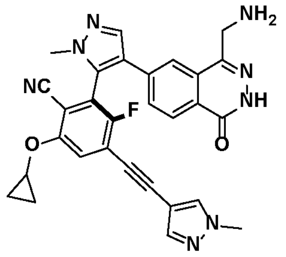

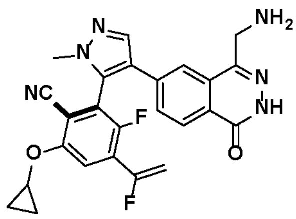 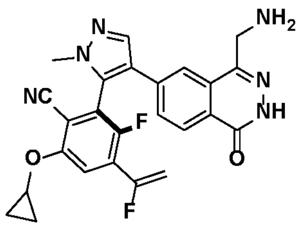

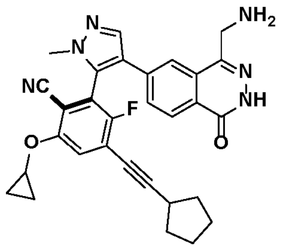 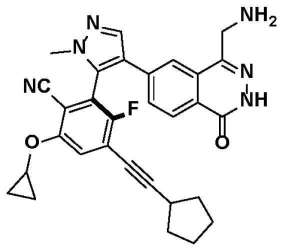 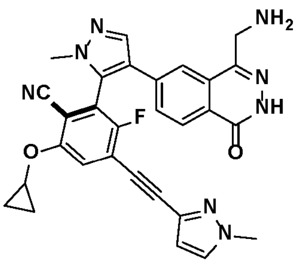 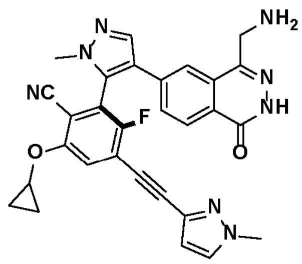

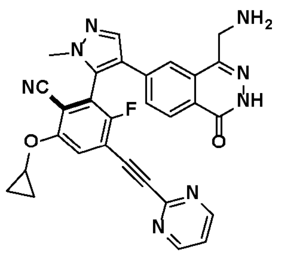 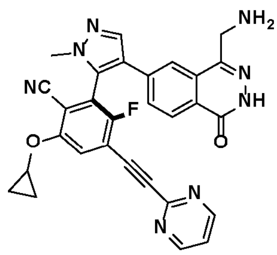 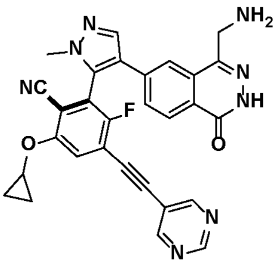 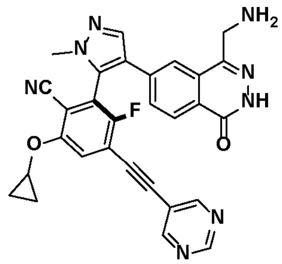

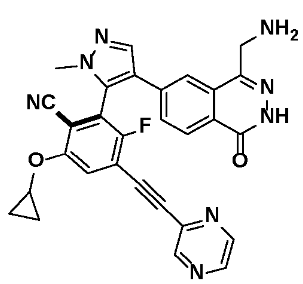
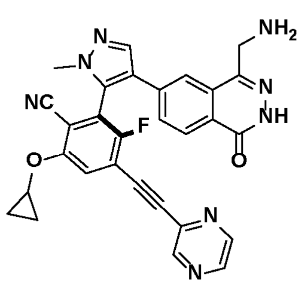
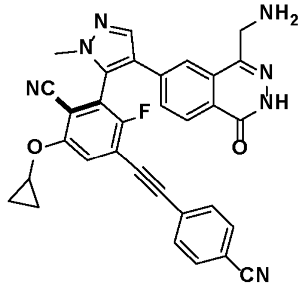
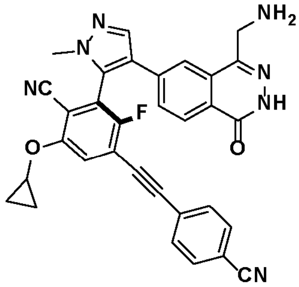

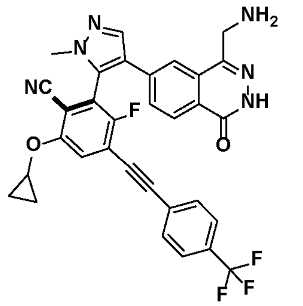
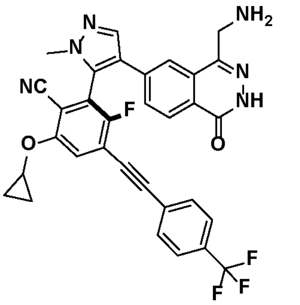
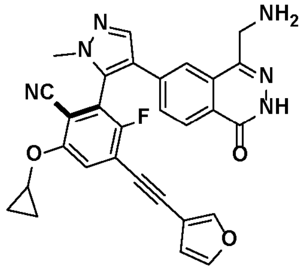
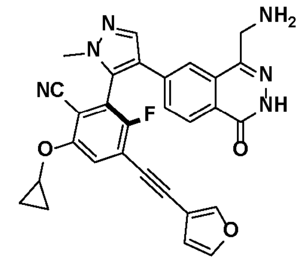

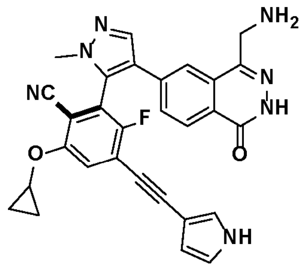
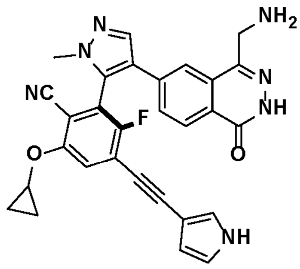
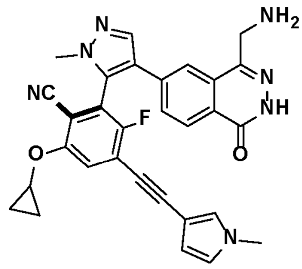
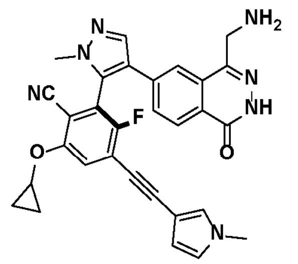

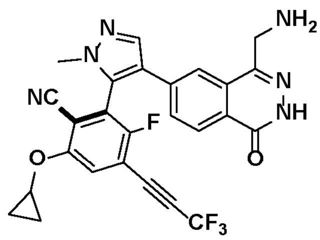
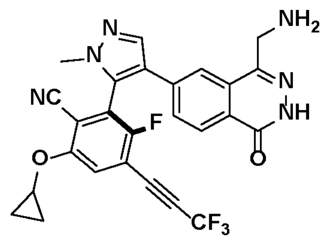
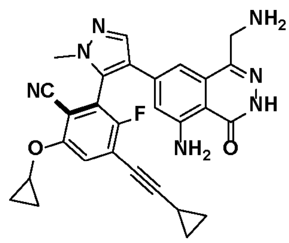

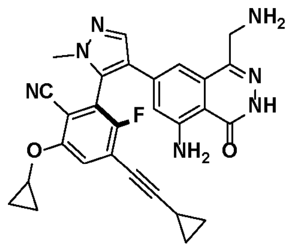
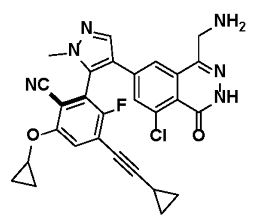
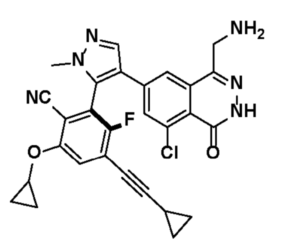
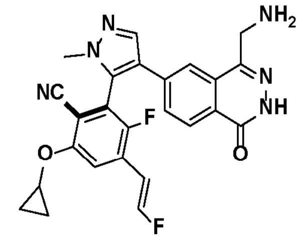

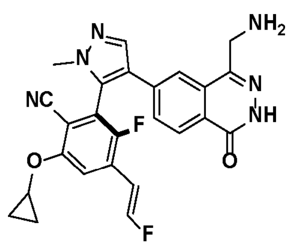
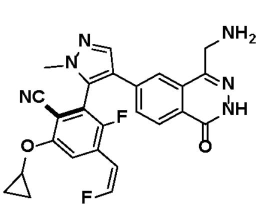
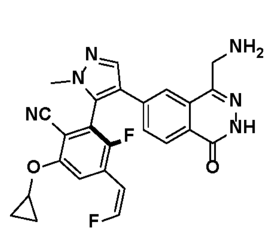
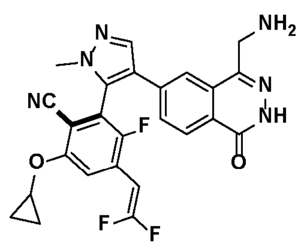

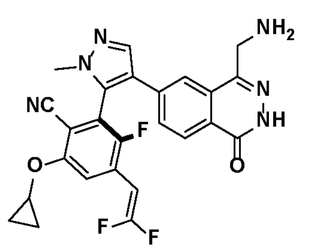
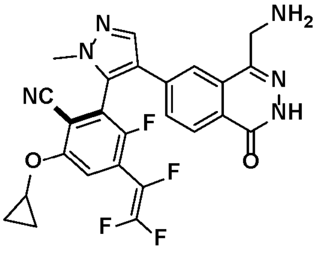
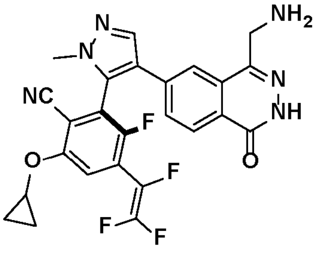

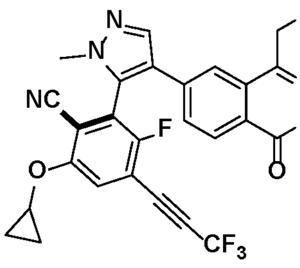
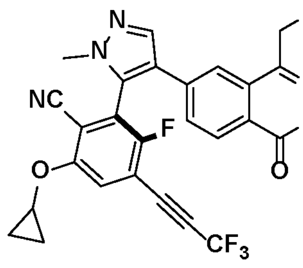

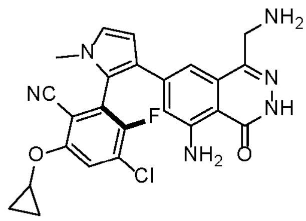
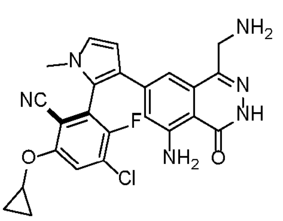
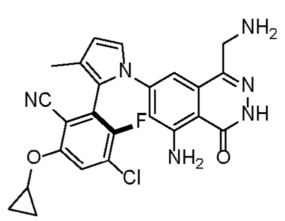
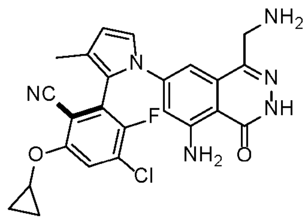

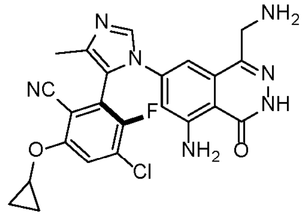
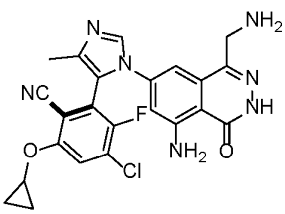
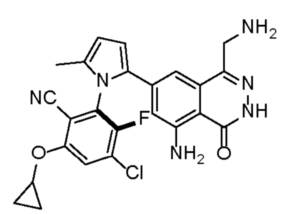
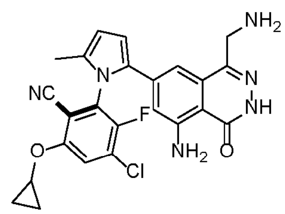

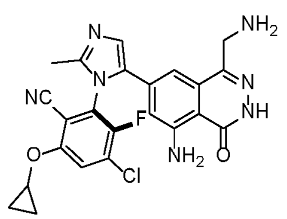
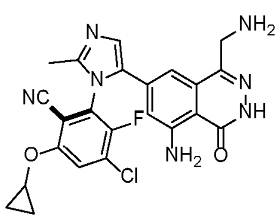
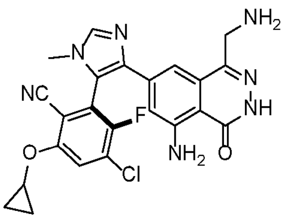
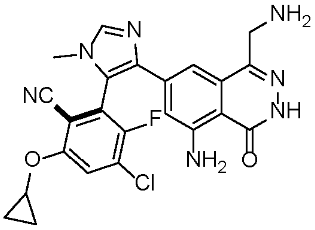

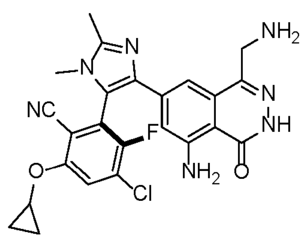
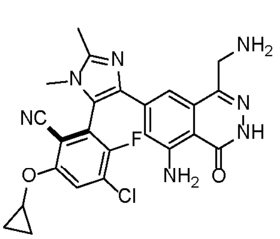
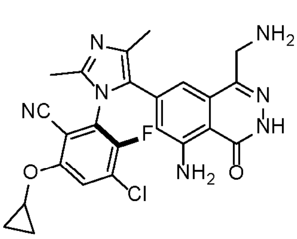
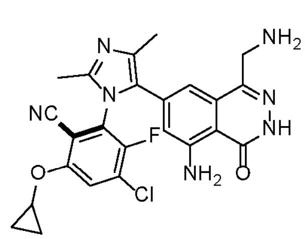

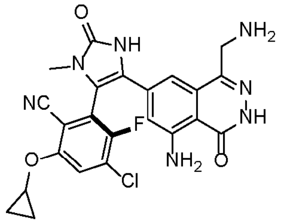 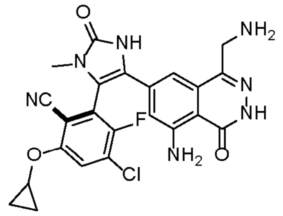 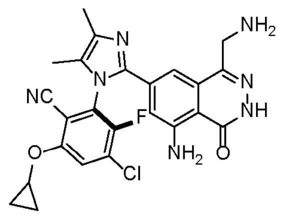 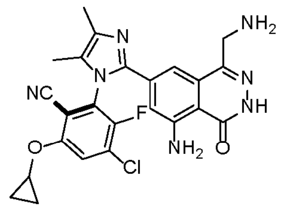

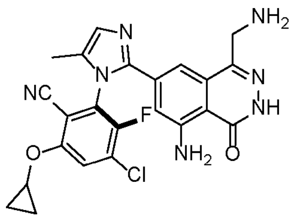 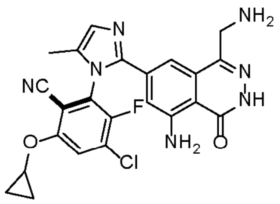 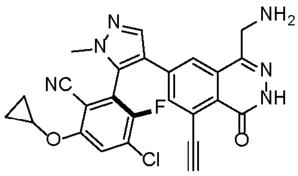 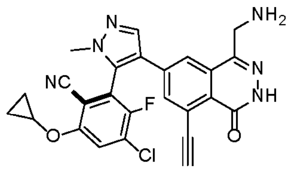

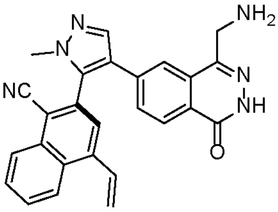 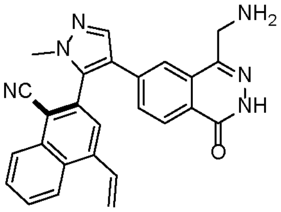 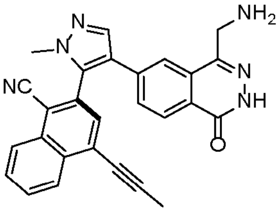 or 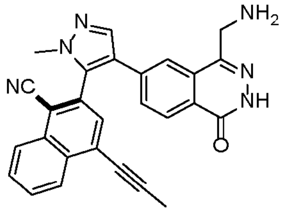 .

**22.** A pharmaceutical composition, comprising the compound, the stereoisomer thereof, or the pharmaceutically acceptable salt thereof according to any one of claims 1-21, and optionally further comprising a pharmaceutically acceptable excipient.

**23.** Use of the compound, the stereoisomer thereof, or the pharmaceutically acceptable salt thereof according to any one of claims 1-21, or the pharmaceutical composition according to claim 22 for preparing a medicament for preventing or treating cancer.

## INTERNATIONAL SEARCH REPORT

International application No.
**PCT/CN2023/134733**

### A. CLASSIFICATION OF SUBJECT MATTER

C07D 237/32(2006.01)i；C07D 403/04(2006.01)i；C07D 403/14(2006.01)i；A61K 31/502(2006.01)i；A61P 35/00(2006.01)i

According to International Patent Classification (IPC) or to both national classification and IPC

### B. FIELDS SEARCHED

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNTXT, WPABSC, ENTXTC, STN: 正大天晴, 吡唑, 酞嗪酮, 抑制剂, 癌, dihydrophthalazin, pyrazol, inhibitor, cancer, PRMT, MTA, 结构检索, Structural search

### C. DOCUMENTS CONSIDERED TO BE RELEVANT

| Category* | Citation of document, with indication, where appropriate, of the relevant passages | Relevant to claim No. |
|---|---|---|
| E | WO 2024008176 A1 (TIBET HAISCO PHARMACEUTICAL CO., LTD.) 11 January 2024 (2024-01-11)<br>description, table 1, and embodiments | 1-23 |
| PX | WO 2023125540 A1 (MEDSHINE DISCOVERY INC.) 06 July 2023 (2023-07-06)<br>claims 1-15 | 1-23 |
| PX | WO 2023098439 A1 (ABBISKO THERAPEUTICS CO., LTD.) 08 June 2023 (2023-06-08)<br>claims 1-16, and embodiments 1-37 | 1-23 |
| PX | CN 116178347 A (SUZHOU PUHE PHARMACEUTICAL TECHNOLOGY CO., LTD.) 30 May 2023 (2023-05-30)<br>claims 1-15 | 1-23 |
| PX | WO 2022256806 A1 (IDEAYA BIOSCIENCES, INC.) 08 December 2022 (2022-12-08)<br>description, pages 153-175 | 1-23 |
| X | WO 2022192745 A1 (MIRATI THERAPEUTICS, INC.) 15 September 2022 (2022-09-15)<br>claims 1-35, and embodiments 15-7 | 1-23 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 February 2024** | **21 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2023/134733**

**C. DOCUMENTS CONSIDERED TO BE RELEVANT**

| Category* | Citation of document, with indication, where appropriate, of the relevant passages | Relevant to claim No. |
|---|---|---|
| X | CN 114728912 A (MIRATI THERAPEUTICS INC.) 08 July 2022 (2022-07-08) claims 1-97, and table 4 | 1-23 |
| X | WO 2022216645 A1 (MIRATI THERAPEUTICS, INC.) 13 October 2022 (2022-10-13) claims 11-20 | 1-23 |
| X | WO 2022216648 A1 (MIRATI THERAPEUTICS, INC.) 13 October 2022 (2022-10-13) claims 12-20 | 1-23 |
| X | SMITH, C. R. et al. "Design and Evaluation of Achiral, Non-atropisomeric 4-(aminomethyl) Phthalazin-1(2H)-one Derivatives as Novel PRMT5/MTA Inhibitors" *Bioorganic & Medicinal Chemistry,* Vol. 71, 26 July 2022 (2022-07-26), 116947 figures 1-2, and tables 1-2 | 1-23 |

Form PCT/ISA/210 (second sheet) (July 2022)

**INTERNATIONAL SEARCH REPORT**
**Information on patent family members**

International application No.
**PCT/CN2023/134733**

| Patent document cited in search report | Publication date (day/month/year) | Patent family member(s) | Publication date (day/month/year) |
|---|---|---|---|
| WO 2024008176 A1 | 11 January 2024 | None | |
| WO 2023125540 A1 | 06 July 2023 | None | |
| WO 2023098439 A1 | 08 June 2023 | None | |
| CN 116178347 A | 30 May 2023 | None | |
| WO 2022256806 A1 | 08 December 2022 | CA 3220160 A1 | 08 December 2022 |
| | | AU 2022287057 A1 | 14 December 2023 |
| | | IL 308853 A | 01 January 2024 |
| WO 2022192745 A1 | 15 September 2022 | EP 4304720 A1 | 17 January 2024 |
| CN 114728912 A | 08 July 2022 | IL 290341 A | 01 April 2022 |
| | | US 2023054883 A1 | 23 February 2023 |
| | | AU 2020345899 A1 | 03 March 2022 |
| | | US 2021079003 A1 | 18 March 2021 |
| | | US 11492351 B2 | 08 November 2022 |
| | | JP 2022548255 A | 17 November 2022 |
| | | NO 20220309 A1 | 11 March 2022 |
| | | TW 202122387 A | 16 June 2021 |
| | | WO 2021050915 A1 | 18 March 2021 |
| | | CL 2022000603 A1 | 27 January 2023 |
| | | CA 3150515 A1 | 18 March 2021 |
| | | CO 2022004513 A2 | 21 June 2022 |
| | | US 2021078994 A1 | 18 March 2021 |
| | | US 11479551 B2 | 25 October 2022 |
| | | BR 112022004248 A2 | 31 May 2022 |
| | | MX 2022002938 A | 26 May 2022 |
| | | KR 20220083691 A | 20 June 2022 |
| | | EP 4028388 A1 | 20 July 2022 |
| WO 2022216645 A1 | 13 October 2022 | KR 20230167050 A | 07 December 2023 |
| | | US 2022331323 A1 | 20 October 2022 |
| | | CA 3214535 A1 | 13 October 2022 |
| | | IL 307393 A | 01 December 2023 |
| | | AU 2022254651 A1 | 02 November 2023 |
| WO 2022216648 A1 | 13 October 2022 | CA 3214530 A1 | 13 October 2022 |
| | | IL 307392 A | 01 December 2023 |
| | | AU 2022253455 A1 | 02 November 2023 |
| | | KR 20230167053 A | 07 December 2023 |
| | | US 2022331324 A1 | 20 October 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202211514520
- CN 202310612126
- CN 202311533426
- CN 202311545467X

### Non-patent literature cited in the description

- Greene's Protective Groups in Organic Synthesis. John Wiley & Sons, Inc